# EUROPEAN PATENT APPLICATION

(11) **EP 4 837 938 A2**
(43) Date of publication of application: **11.11.2026**
(21) Application number: 26198768.9
(22) Date of filing: 21.10.2022
(51) Int. Cl.: C09D 5/00

(54) **POLYURETHANE-ACETOACETATE COMPOUNDS**

(30) Priority: 26.10.2021 US 202163271753 P
(62) Document number(s) of the earlier application(s) in accordance with Art. 76 EPC: 22877684.5 / 4 423 059
(71) Applicant: PPG Industries Ohio, Inc., Cleveland, OH 44111 (US)
(72) Inventor: Zhou, Hongying, Allison Park, Pennsylvania, 15101 (US); Breon, Jonathan P., Memphis, Tennessee, 38119 (US); Morris, Trevor Brenton, Allison Park, Pennsylvania, 15101 (US); Moravek, Scott J., Mars, Pennsylvania, 16046 (US); Mayo, Michael A., Pittsburgh, Pennsylvania, 15239 (US); Nava Ortiz, Cesar Alejandro Bernabe, 52978 Mexico City (MX)
(74) Representative: f & e patent

(57) **Abstract**

A compound comprising polyacetoacetate functionality and at least one urethane linkage is disclosed. A coating composition comprising the (i) compound comprising polyacetoacetate functionality and at least one urethane linkage, (ii) a blocked polyamine, and (iii) a polyepoxide reactive with the condensation product of (i) and (ii) is also disclosed. A method of preparing a compound comprising polyacetoacetate functionality and at least one urethane linkage is also disclosed.

## Description

### FIELD OF THE INVENTION

The present invention relates to a compound having polyacetoacetate functionality and at least one urethane linkage and coating compositions including the compound.

### BACKGROUND OF THE INVENTION

With some applications, such a vehicular coatings, multi-layer coatings are applied and formed over a substrate, such as, a metal substrate. In some instances, such as, with original equipment automotive coatings (OEM), the multi-layer coatings include, in sequence from the metal substrate, an electrodeposited corrosion resistance layer, a primer layer, a pigmented basecoat layer, and a clearcoat layer. During use, such as, during driving, the multi-layer coating can become damaged, such as, by debris striking the multi-layer coating or through vehicular accidents.

In the automotive refinish process, repairing a defect in the original coating can involve sanding or grinding out the defect by mechanical means down to or into the electrodeposited corrosion resistance layer, followed by cleaning the sanded area with solvent. Typically, a refinish primer is applied over the sanded and cleaned area, allowed to dry, and then sanded. A refinish basecoat is applied over the sanded refinish primer and a refinish clearcoat is applied over the refinish basecoat using a wet-on-wet method. The applied refinish layers are cured at ambient conditions for a sufficient amount of time. Such a refinish undercoat application process can be time consuming and expensive, due to volatile organic abatement engineering requirements and the time required for drying the applied coating layers.

It would be desirable to develop a single refinish primer that provides both impact resistance and corrosion resistance. It would be further desirable that such a newly developed primer provides other properties such as, but not limited to, hardness, rapid cure rates, and a low amount of volatile organic compounds.

### SUMMARY OF THE INVENTION

The present invention is directed to a compound including polyacetoacetate functionality and at least one urethane linkage, including at least one of the following Structures (I) to (III): 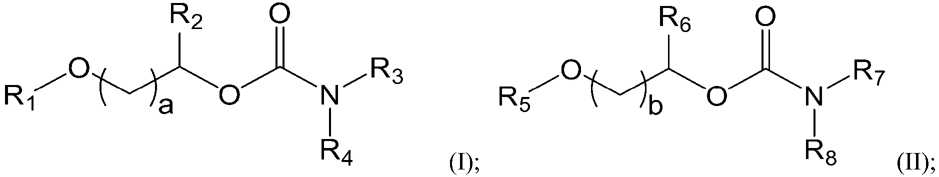 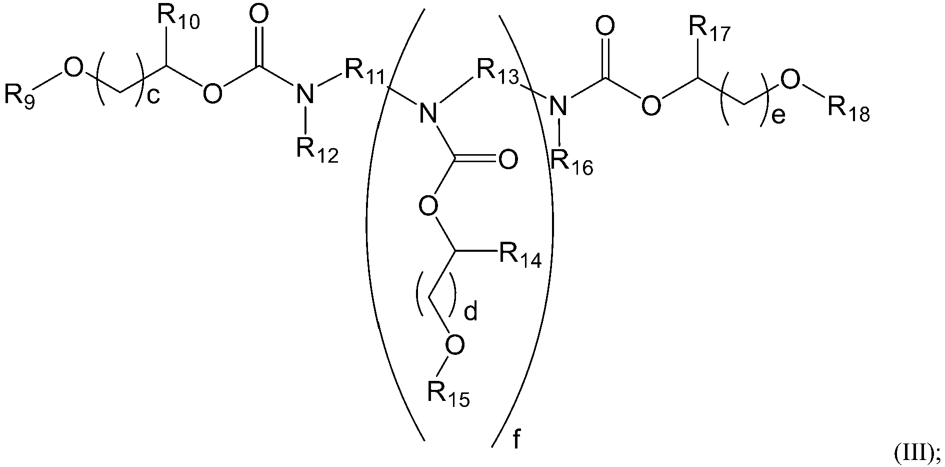 where R₁ includes an acetoacetate containing group; R₂ includes a hydrogen, methyl, or an acetoacetate containing group; R₃ is bound to the nitrogen of the urethane linkage and includes at least two acetoacetate containing groups; R₄ is bound to the nitrogen of the urethane linkage and includes a hydrogen, alkyl, or an acetoacetate containing group; a is 1 or 2; R₅ includes an acetoacetate containing group; R₆ includes a hydrogen, methyl, or an acetoacetate containing group; R₇ is bound to the nitrogen of the urethane linkage and includes at least one acetoacetate containing group; R₈ is bound to the nitrogen of the urethane linkage and includes at least one acetoacetate containing group; b is 1 or 2; R₉, R₁₅, and R₁₈ each independently includes an acetoacetate containing group; R₁₀, R₁₄, and R₁₇ each independently includes a hydrogen, methyl, or acetoacetate containing group; R₁₁ is bound to the nitrogen of the urethane linkage and includes an alkyl, a cycloalkyl, an aryl, 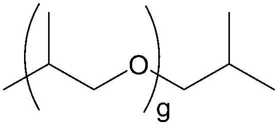 where g is 2 to 70, or 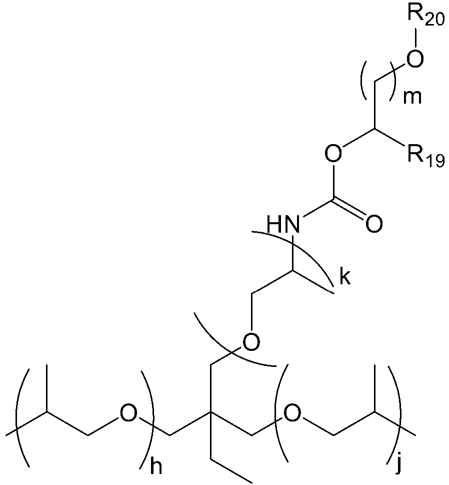 where the sum of h, j, and k is 5 to 6, R₁₉ includes a hydrogen, methyl, or an acetoacetate containing group, R₂₀ includes an acetoacetate containing group, and m is 1 or 2, or 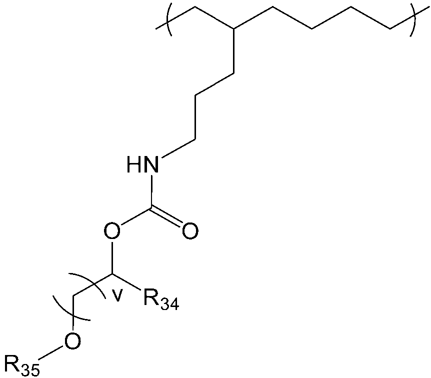 where R₃₄ includes a hydrogen, methyl, or an acetoacetate containing group, R₃₅ includes an acetoacetate containing group, and v is 1 or 2; R₁₂ and R₁₆ each independently includes a hydrogen, an alkyl, a cycloalkyl, or an aryl; R₁₃ is bound to the nitrogen of the urethane linkage and includes an alkyl; c, d, and e are each independently 1 or 2; and f is 0 to 3.

The present invention also relates to a method of preparing a compound that includes polyacetoacetate functionality and at least one urethane linkage, the method including: reacting (a) at least one cyclic carbonate compound, (b) at least one polyol having one primary amine and at least two hydroxyl groups, at least one polyol having one or more secondary amines, and/or at least one compound including two or more amine groups, and (c) optionally at least one lactone or at least one lactone cyclic di-ester to form a polyol-functional urethane compound; and reacting a compound including an acetoacetate functionality with a hydroxyl group of the polyol-functional urethane compound to introduce acetoacetate functionality into the polyol-functional urethane compound to form the compound including polyacetoacetate functionality and at least one urethane linkage.

### BRIEF DESCRIPTION OF THE DRAWINGS

Figure 1A is a photograph of a P99X substrate coated with the coating composition of Example A which contained the compound having polyacetoacetate and polyurethane functionality of Example 1 after the 500 hour salt spray.
Figure 1B is a photograph of a P99X substrate coated with the coating composition of Example A which contained the compound having polyacetoacetate and polyurethane functionality of Example 1 after the 700 hour salt spray test.
Figure 2A is a photograph of a P99X substrate coated with the coating composition of Example B which contained the compound having polyacetoacetate and polyurethane functionality of Example 2 after a 500 hour salt spray.
Figure 2B is a photograph of a P99X substrate coated with the coating composition of Example B which contained the compound having polyacetoacetate and polyurethane functionality of Example 2 after a 700 hour salt spray.
Figure 3 is a photograph of a P99X substrate coated with the coating composition of Comparative Example 1 that contained a compound having polyacetoacetate functionality after the 500 hour salt spray test.

### DESCRIPTION OF THE INVENTION

For the purposes of the following detailed description, it is to be understood that the invention may assume various alternative variations and step sequences, except where expressly specified to the contrary. Moreover, other than in any operating examples, or where otherwise indicated, all numbers expressing, for example, quantities of ingredients used in the specification and claims are to be understood as being modified in all instances by the term "about". Accordingly, unless indicated to the contrary, the numerical parameters set forth in the following specification and attached claims are approximations that may vary depending upon the desired properties to be obtained by the present invention. At the very least, and not as an attempt to limit the application of the doctrine of equivalents to the scope of the claims, each numerical parameter should at least be construed in light of the number of reported significant digits and by applying ordinary rounding techniques.

Notwithstanding that the numerical ranges and parameters setting forth the broad scope of the invention are approximations, the numerical values set forth in the specific examples are reported as precisely as possible. Any numerical value, however, inherently contains certain errors necessarily resulting from the standard variation found in their respective testing measurements.

Also, it should be understood that any numerical range recited herein is intended to include all sub-ranges subsumed therein. For example, a range of "1 to 10" is intended to include all sub-ranges between (and including) the recited minimum value of 1 and the recited maximum value of 10, that is, having a minimum value equal to or greater than 1 and a maximum value of equal to or less than 10.

In this application, the use of the singular includes the plural and plural encompasses the singular, unless specifically stated otherwise. In addition, in this application, the use of "or" means "and/or" unless specifically stated otherwise, even though "and/or" may be explicitly used in certain instances. Further, in this application, the use of "a" or "an" means "at least one" unless specifically stated otherwise. For example, "a" polyol, "an" oligomer, and the like refer to one or more of any of these items.

As used herein, "compound" refers to a macromolecule, a polymer, an oligomer, and/or a covalently bonded collection of atoms. As used herein, "macromolecule" refers to a material comprising a plurality of atoms bonded together to form a molecule having a molecular weight of at least 150 grams per mole (g/mol). As used herein, the term "polymer" refers to prepolymers, oligomers, and both homopolymers and copolymers. As used herein, "oligomer" refers to a polymer having only a small number (2-10) of monomer units, for example, a dimer, trimer or tetramer.

Any "R" group (e.g., R, R¹-Rⁿ, and the like) from this disclosure refers to any suitable atom, molecule, or polymer chain unless specifically indicated otherwise, and wherein the R groups may be the same or different from one another. The suitable atom may include a hydrogen atom or any other suitable atom.

As used herein, the transitional term "comprising" (and other comparable terms, e.g., "containing" and "including") is "open-ended" and open to the inclusion of unspecified matter. Although described in terms of "comprising", the terms "consisting essentially of' and "consisting of" are also within the scope of the invention.

As used herein, the terms "on", "applied on/over", "formed on/over", "deposited on/over", "overlay", "provided on/over", and the like mean applied, formed, overlaid, deposited, or provided on but not necessarily in direct contact with the surface. For example, a coating layer "applied over" a substrate does not preclude the presence of one or more other coating layers of the same or different composition located between the formed coating layer and the substrate.

As used herein, the term "urethane linkage" refers to a functional group having the following structure:

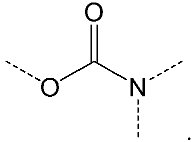

As used herein, the term "acetoacetate containing group" refers to a group bound to an -O- and terminated by an acetoacetyl functional group having the structure: 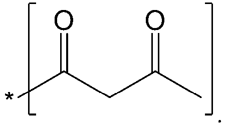 Non-limiting examples of acetoacetate containing groups include: 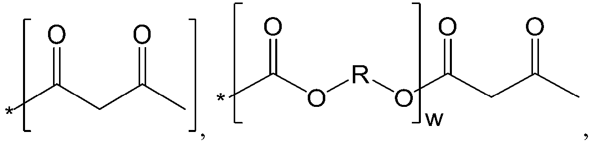 and 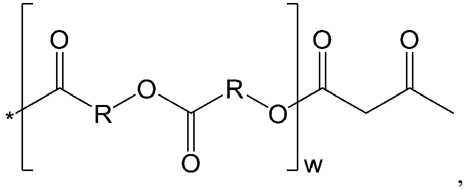 or some combination thereof, where R can be a linear or branched alkyl, and where w in each case can be less than 10, such as less than 5, such as less than 2. As used herein, the acetoacetate containing group is designated by "AcAc".

As used herein, "*" indicates where the depicted group may bind to a portion of the larger molecule.

As used herein, "monovalent" refers to an R group that, as a group, forms only one covalent bond with another atom or compound. As used herein "divalent" refers to an R group that, as a group, forms two covalent bonds with at least one other atom or compound. As used herein "trivalent" refers to an R group that, as a group, forms three covalent bonds with at least one other atom or compound. As used herein "tetravalent" refers to an R group that, as a group, forms four covalent bonds with at least one other atom or compound.

As used herein, "alkyl" refers to straight, branched chain, or cyclic hydrocarbon groups, or combinations thereof, including from 1 to 20 carbon atoms, for example and without limitation C₁₋₃, C₁₋₆, C₁₋₁₀, C₁-C₁₂, C₁-C₁₅, C₁-₁₈ groups, for example and without limitation, straight, branched chain alkyl groups such as methyl, ethyl, propyl, butyl, pentyl, hexyl, heptyl, octyl, nonyl, decyl, undecyl, dodecyl, and the like.

As used herein, "cycloalkyl" refers a non-aromatic mono- or multicyclic ring system comprising, for example, from 3 to 10 carbon atoms, or from 5 to 10 carbon atoms. In some non-limiting embodiments, the cycloalkyl ring contains from 5 to 7 ring atoms. The cycloalkyl can be optionally substituted with one or more "ring system substituents" which may be the same or different. Non-limiting examples of suitable monocyclic cycloalkyls include cyclopropyl, cyclopentyl, cyclohexyl, cycloheptyl and the like. Non-limiting examples of suitable multicyclic cycloalkyls include 1-decalinyl, norbornyl, adamantyl and the like.

As used herein, "aryl" refers to an aromatic monocyclic or multicyclic ring system comprising, for example, from 6 to 14 carbon atoms, or from 6 to 10 carbon atoms. The aryl group can be optionally substituted with one or more "ring system substituents" which may be the same or different. Non-limiting examples of suitable aryl groups include phenyl and naphthyl.

The present invention is directed to a compound comprising polyacetoacetate functionality and at least one urethane linkage, including at least one of the following Structures (I) to (III):

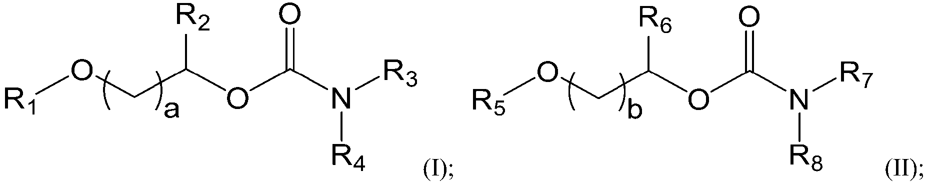

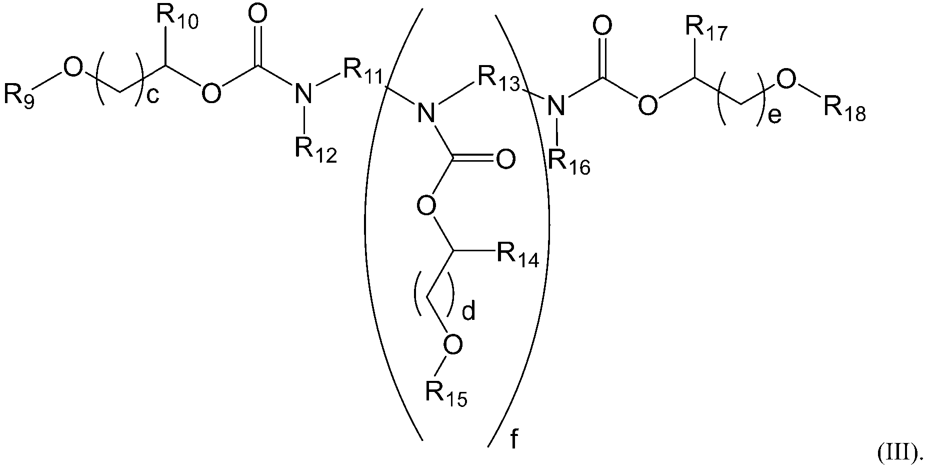

For example, the compound comprising polyacetoacetate functionality and at least one urethane linkage may comprise the following structure (I): 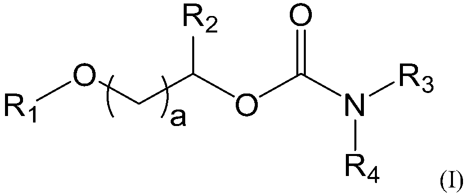 where R₁ can be monovalent and comprises an acetoacetate containing group; where R₂ can be monovalent and comprises a hydrogen, methyl, or an acetoacetate containing group; where R₃ can be monovalent, is bound to the nitrogen of the urethane linkage, and comprises at least two acetoacetate containing groups; where R₄ can be monovalent and is bound to the nitrogen of the urethane linkage and comprises a hydrogen, alkyl, or an acetoacetate containing group; and where a can be 1 or 2.

When R₁ comprises an acetoacetate containing group, R₃ comprises at least two acetoacetate containing groups, and R₄ is a hydrogen, the compound of Structure (I) may have one of the following structures: 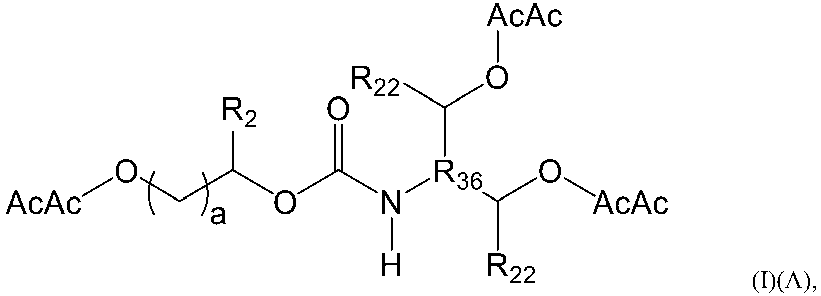 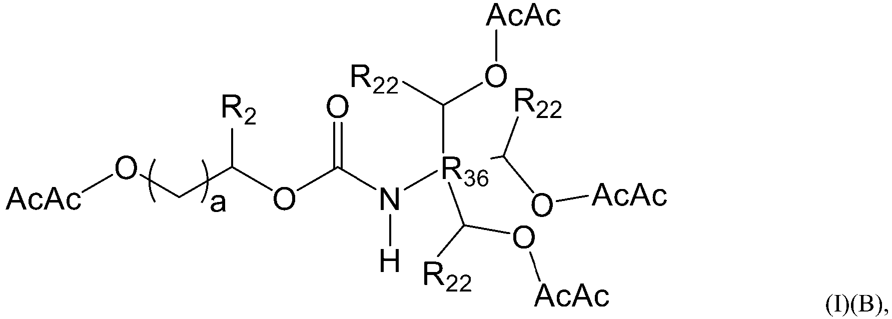 where R₂ can be monovalent and may be a hydrogen, a methyl, or an acetoacetate containing group, such as 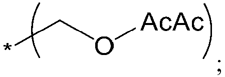 each R₂₂ can be monovalent and can independently be a hydrogen or an alkyl, such as a C₁ to C₆ alkyl; R₃₆ can be trivalent or tetravalent and can be a linear or branched alkyl, such as a C₁ to C₆ alkyl, a substituted or unsubstituted cycloalkyl, or a substituted or unsubstituted aryl, and a can be 1 or 2. Each acetoacetate containing group on Structures (I)(A) and (I)(B) may be the same or different and may be: 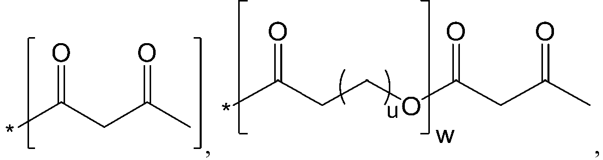 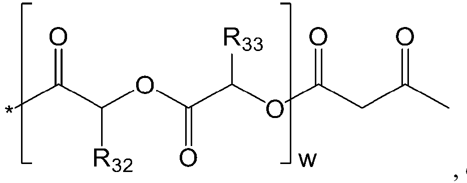 or some combination thereof, where u can be 1 or 2, w in each case can be less than 10, such as less than 5, such as less than 2, and R₃₂ and R₃₃ can each independently be monovalent and can each independently be a hydrogen or a methyl.

When R₁ comprises an acetoacetate containing group, R₃ comprises at least two acetoacetate containing groups, and R₄ comprises an acetoacetate containing group, the compound of Structure (I) may have one of the following exemplary structures: 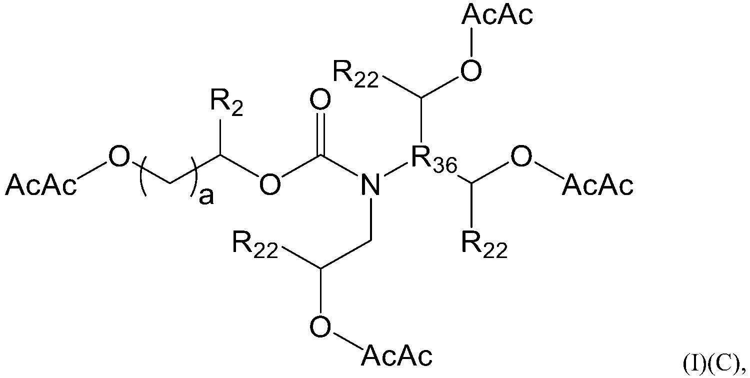 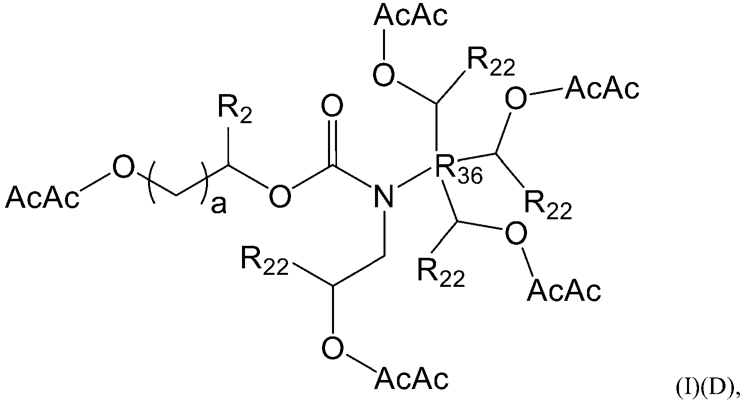 where R₂ may be monovalent and may be a hydrogen, a methyl, or an acetoacetate containing group, such as 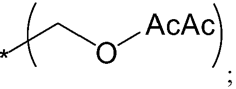 each R₂₂ can independently be monovalent and can independently be a hydrogen or an alkyl, such as a C₁ to C₆ alkyl; R₃₆ can be trivalent or tetravalent and can be a linear or branched alkyl, such as a C₁ to C₆ alkyl, a substituted or unsubstituted cycloalkyl, or a substituted or unsubstituted aryl, and a can be 1 or 2. Each acetoacetate containing group on Structure (I)(C)-(D) may be the same or different and may be: 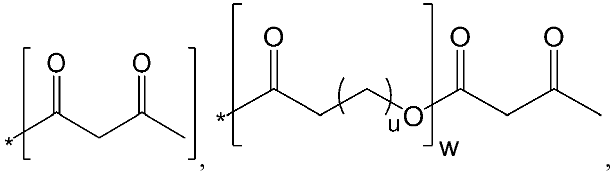 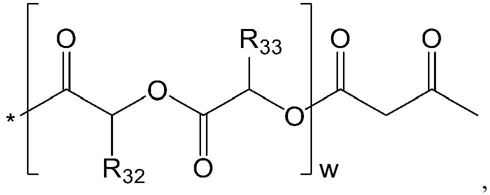 or some combination thereof, where u can be 1 or 2, where w in each case can be less than 10, such as less than 5, such as less than 2, and R₃₂ and R₃₃ can each independently be monovalent and can each independently be a hydrogen or a methyl.

The compound comprising polyacetoacetate functionality and at least one urethane linkage of Structure (I) can be prepared by reacting (a) at least one cyclic carbonate compound, (b) at least one polyol having one primary amine and at least two hydroxyl groups, and (c) optionally at least one lactone or at least one lactone cyclic di-ester to form a polyol-functional urethane compound. A compound comprising an acetoacetate functionality can be reacted with one or more hydroxyl groups of the polyol-functional urethane compound to introduce acetoacetate functionality into the polyol-functional urethane compound to form the compound comprising polyacetoacetate functionality and at least one urethane linkage.

The (a) at least one cyclic carbonate compound may comprise the following structure: 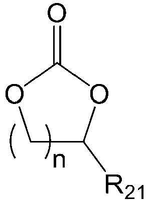 where R₂₁ can be monovalent and can be a hydrogen, methyl, or hydroxymethyl; and n can be 1 or 2. Non-limiting examples of the (a) at least one cyclic carbonate compound include ethylene carbonate, propylene carbonate, glycerol carbonate, trimethylene carbonate, 4-methyl-1,3-dioxan-2-one, and 4-(hydroxylmethyl)-1,3-dioxan-2-one, or some combination thereof.

The (b) at least one polyol having one primary amine and at least two hydroxyl groups may comprise the following structure: 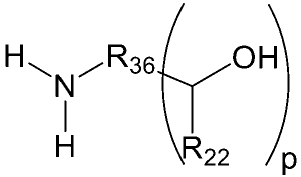 where R₂₂ can be monovalent and can be a hydrogen or an alkyl, such as a C₁ to C₆ alkyl; R₃₆ can be trivalent or tetravalent and can be a linear or branched alkyl, such as a C₁ to C₆ alkyl, a substituted or unsubstituted cycloalkyl, or a substituted or unsubstituted aryl; and p can be 2 or 3. Non-limiting examples of the (b) one polyol having one primary amine and at least two hydroxyl groups include 2-amino-1,3-propanediol, 3-aminopentane-2,4-diol, 2-amino-2-(hydroxymethyl)-1,3-propanediol, and 3-amino-3-(1-hydroxyethyl)pentane-2,4-diol, 3-amino-2,3-dideoxy-D-*myo*-inositol, or some combination thereof.

The (b) at least one polyol having one primary amine and at least two hydroxyl groups may comprise 3-amino-2,3-dideoxy-D-*myo*-inositol.

In a first reaction step, (a) at least one cyclic carbonate compound and (b) at least one polyol having one primary amine and at least two hydroxyl groups may be reacted to form a polyol-functional urethane compound. The reaction mixture used to prepare the polyol-functional urethane compound is substantially free of (c) at least one lactone or at least one lactone cyclic di-ester. As-used herein, the term "substantially free" means that if (c) at least one lactone or at least one lactone or at least one lactone cyclic di-ester being discussed is present, it is present as an incidental impurity, such as, in amount that is less than 0.1 wt.% based on total solids of the compound. The polyol-functional urethane compound may comprise one of the following exemplary structures: 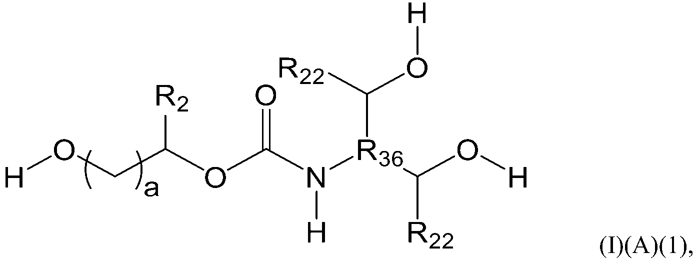 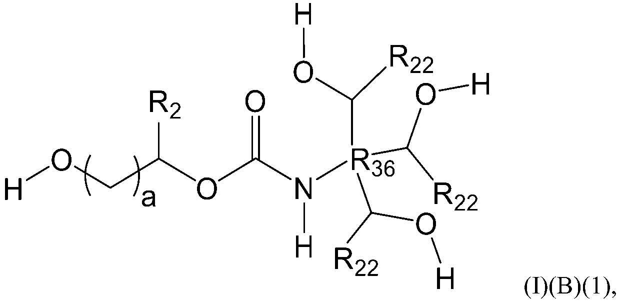 where R₂ can be monovalent and can be a hydrogen, methyl, or hydroxymethyl; R₂₂ can be monovalent and can be a hydrogen or an alkyl; R₃₆ can be trivalent or tetravalent and can be a linear or branched alkyl, a substituted or unsubstituted cycloalkyl, or a substituted or unsubstituted aryl; and a can be 1 or 2.

To form the compound comprising polyacetoacetate functionality and at least one urethane linkage of Structure (I), the polyol-functional urethane compound of Structure (I)(A)(1) or (I)(B)(1) may be reacted with a compound comprising an acetoacetate functionality to introduce acetoacetate functionality into the polyol-functional urethane compound. Non-limiting examples of compounds comprising an acetoacetate functionality include ethyl acetoacetate, methyl acetoacetate, isobutyl acetoacetate, isopropyl acetoacetate, and *tert*-butyl acetoacetate. The compound comprising polyacetoacetate functionality and at least one urethane linkage according to Structure (I) may comprise one of the following exemplary structures: 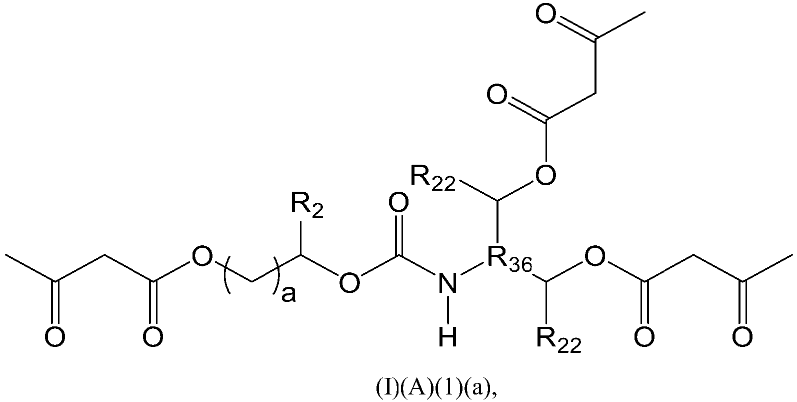 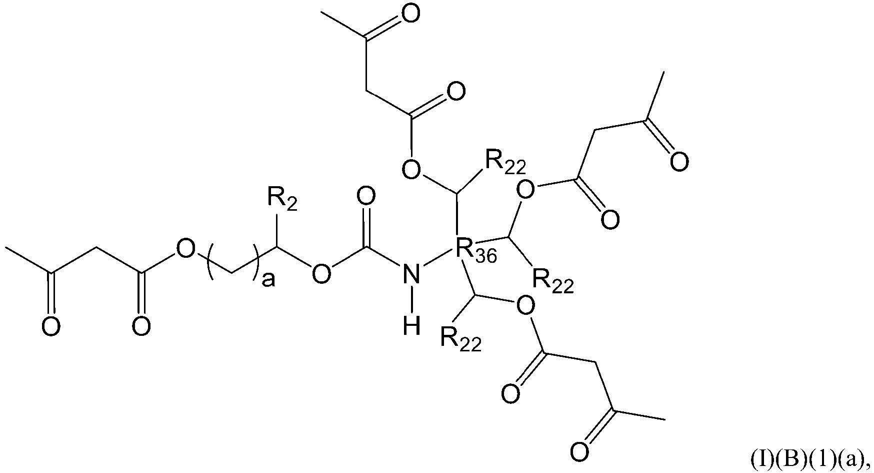 where R₂ can be monovalent and can be hydrogen, methyl, or 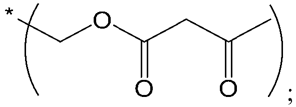 R₂₂ can be monovalent and can be hydrogen or an alkyl; R₃₆ can be trivalent or tetravalent and can be a linear or branched alkyl, a substituted or unsubstituted cycloalkyl, or a substituted or unsubstituted aryl; and a can be 1 or 2.

In an optional second reaction step, the reaction product of the first reaction may be further reacted with (c) at least one lactone to form a polyol-functional urethane compound. The optional (c) at least one lactone may comprise the following structure: 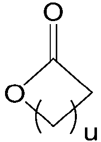 where u can be from 1 to 6. Non-limiting examples of the (c) lactone include β-propiolactone, γ-butyrolactone, δ-valerolactone, ε-caprolactone, or some combination thereof.

The optional (c) at least one lactone cyclic di-ester may comprise the following structure: 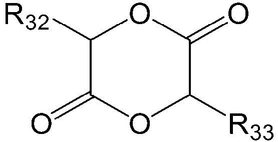 where R₃₂ and R₃₃ each independently be monovalent and can each independently comprise a hydrogen or a methyl. Non-limiting examples of the lactone cyclic di-ester includes D,D-lactide, L,L-lactide, D,L-lactide, glycolide, or some combination thereof.

The second reaction step may include a metal-based catalyst, such as, stannous octoate, zinc octoate, triazabicyclodecene, N-heterocyclic carbene, or some combination thereof. The polyol-functional urethane compound formed from a first and second reaction step, may comprise one of the following exemplary structures when each polyol functional group reacts with lactone: 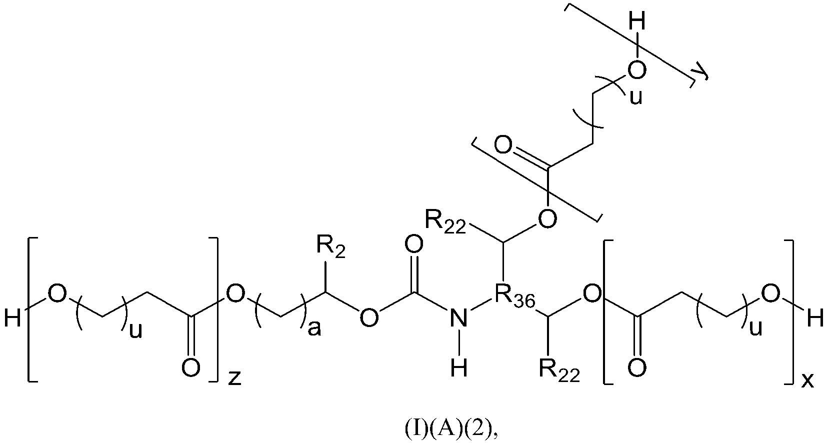 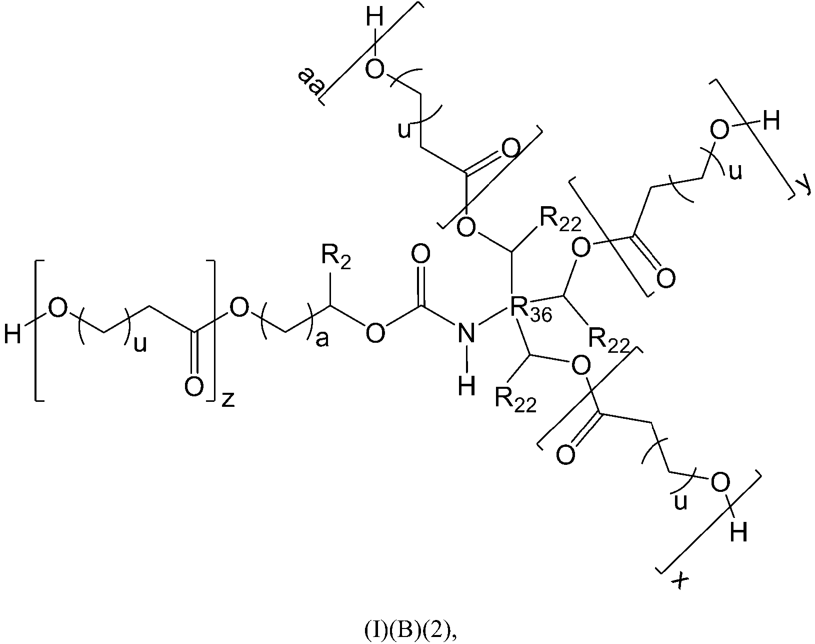 where R₂ can be monovalent and can be a hydrogen, methyl, or 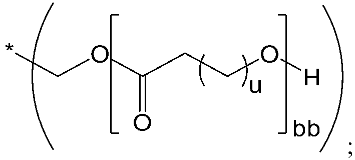 R₂₂ can be monovalent and can be a hydrogen or an alkyl; R₃₆ can be trivalent or tetravalent and can be a linear or branched alkyl, a substituted or unsubstituted cycloalkyl, or a substituted or unsubstituted aryl; a can be 1 or 2; and u can be 1 to 6. For Structure (I)(A)(2), x, y, z, and bb (if present) can each be less than 10 and the sum of x, y, z, and bb (if present) can be less than 10. For Structure (I)(B)(2), x, y, z, aa, and bb (if present) can each be less than 10 and the sum of x, y, z, aa, and bb (if present) can be less than 10. In some non-limiting examples, all of the hydroxyl groups on the polyol-functional urethane compound may not react with the (c) lactone, leaving free hydroxyl groups on the poly-functional urethane compound. All of the hydroxyl groups on the polyol-functional urethane compound may not react with the (c) lactone because of stoichiometry, or the ratio of initial hydroxyl groups relative to the amount of lactone added.

To form the compound of Structure (I), the above polyol-functional urethane compounds may be reacted with the previously described compound comprising an acetoacetate functionality to introduce acetoacetate functionality into the polyol-functional urethane compound backbone. The compound comprising polyacetoacetate functionality and at least one urethane linkage according to Structure (I) may comprise one of the following exemplary structures: 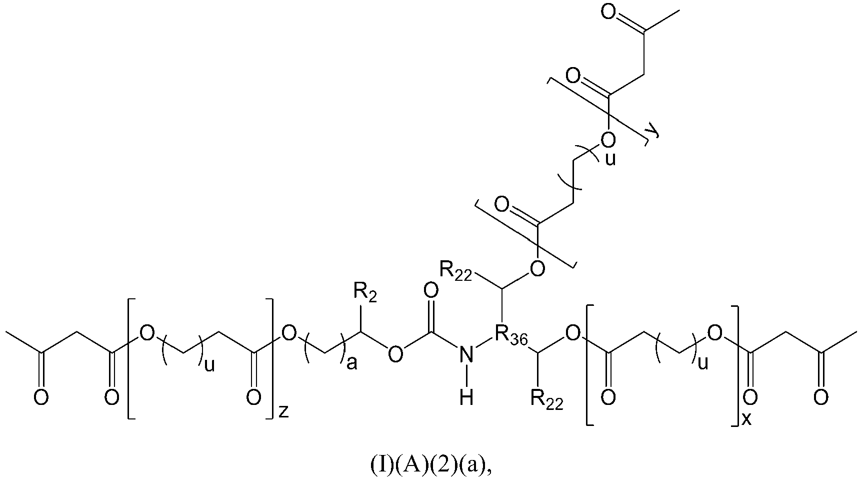 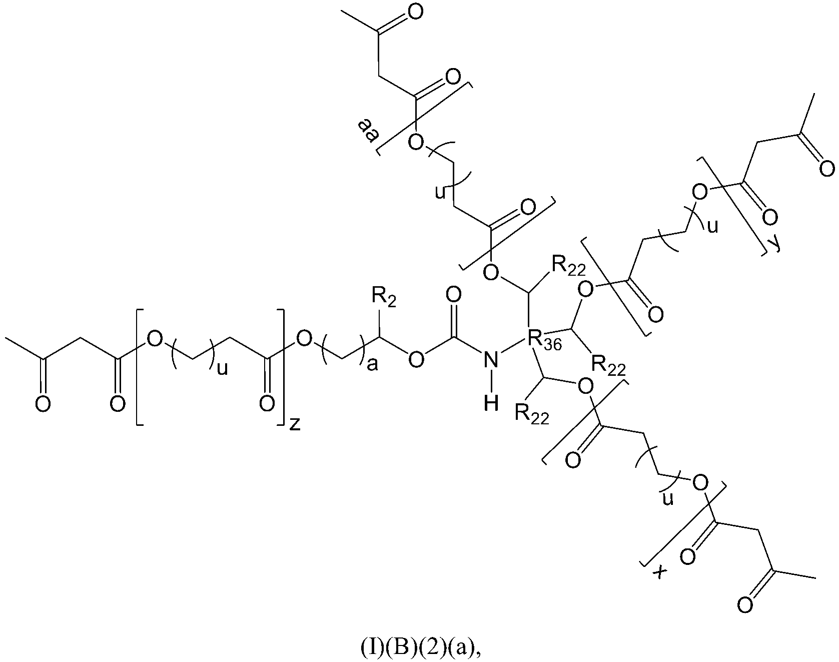 where R₂ can be monovalent and can be a hydrogen, a methyl, or 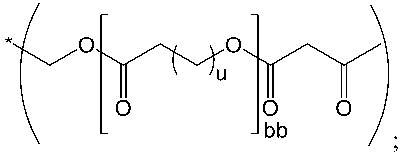 R₂₂ can be monovalent and can be a hydrogen or an alkyl; R₃₆ can be trivalent or tetravalent and can be a linear or branched alkyl, a substituted or unsubstituted cycloalkyl, or a substituted or unsubstituted aryl; a can be 1 or 2; and u can be from 1 to 6. For Structure (I)(A)(2)(a), x, y, z, and bb (if present) can each be less than 10 and the sum of x, y, z, and bb (if present) can be less than 10. For Structure (I)(B)(2)(a), x, y, z, aa, and bb (if present) can each be less than 10 and the sum of x, y, z, aa, and bb (if present) can be less than 10.

In an optional second reaction step, the product of the first reaction may be further reacted with (c) or at least one lactone cyclic di-ester, in the presence of a metal-based catalyst, such as, those described previously. The polyol-functional urethane compound formed from the first and second reaction steps, may comprise one of the following exemplary structures when each polyol functional group reacts with a lactone cyclic di-ester: 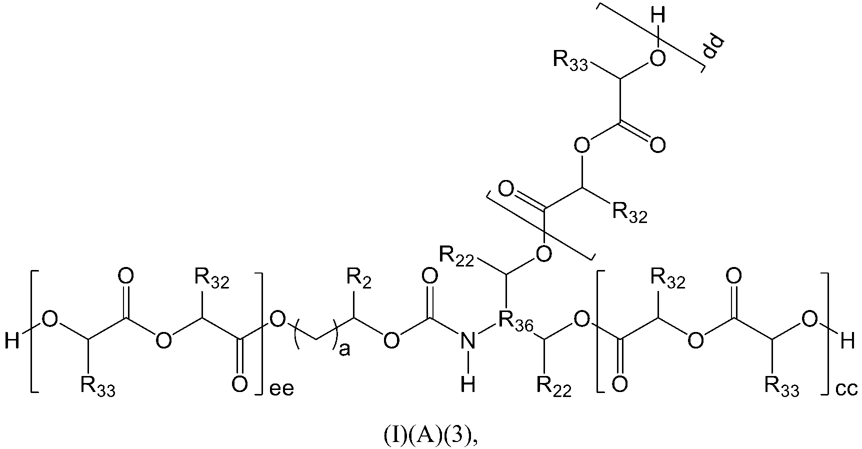 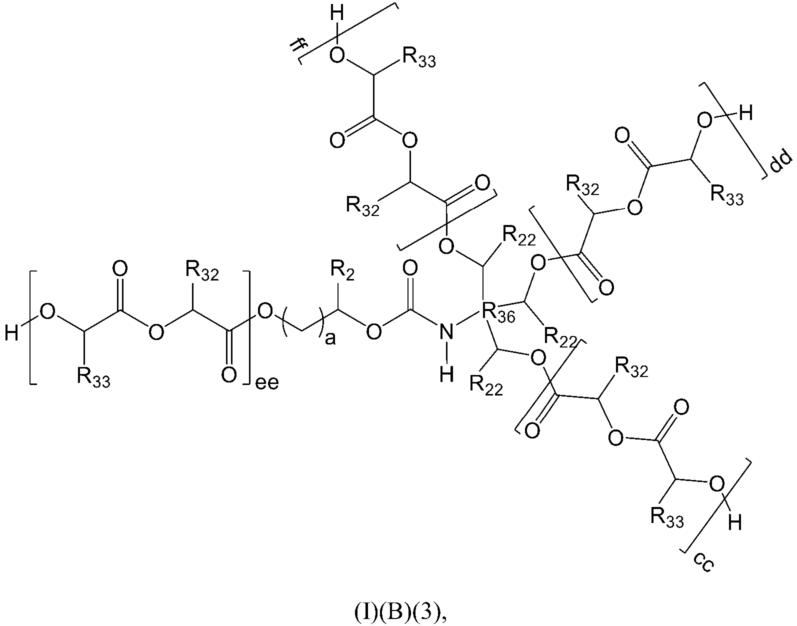 where R₂ can be monovalent and can be a hydrogen, methyl, or 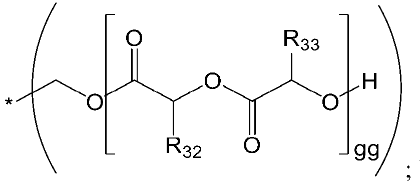 R₂₂ can be monovalent and can be a hydrogen or an alkyl; R₃₂ and R₃₃ can each independently be monovalent and can each independently be a hydrogen or a methyl; R₃₆ can be trivalent or tetravalent and can be a linear or branched alkyl, a substituted or unsubstituted cycloalkyl, or a substituted or unsubstituted aryl; a can be 1 or 2; and u can be 1 to 6. In structure (I)(A)(3), cc, dd, ee, and gg (if present) can each be less than 10 and the sum of cc, dd, ee, and gg (if present) can be less than 10. In structure (I)(B)(3), cc, dd, ee, ff, and gg (if present) can each be less than 10 and the sum of cc, dd, ee, ff, and gg (if present) can be less than 10. In some non-limiting examples, all of the hydroxyl groups on the polyol-functional urethane compound may not react with the (c) lactone cyclic di-ester, leaving free hydroxyl groups on the poly-functional urethane compound. All of the hydroxyl groups on the polyol-functional urethane compound may not react with the (c) lactone cyclic di-ester because of stoichiometry, or the ratio of initial hydroxyl groups relative to the amount of lactone added.

To form the compound of Structure (I), the above polyol-functional urethane compounds may be reacted with the previously described compound comprising an acetoacetate functionality to introduce acetoacetate functionality into the polyol-functional urethane compound backbone. The compound comprising polyacetoacetate functionality and at least one urethane linkage according to Structure (I) may comprise the following exemplary structures: 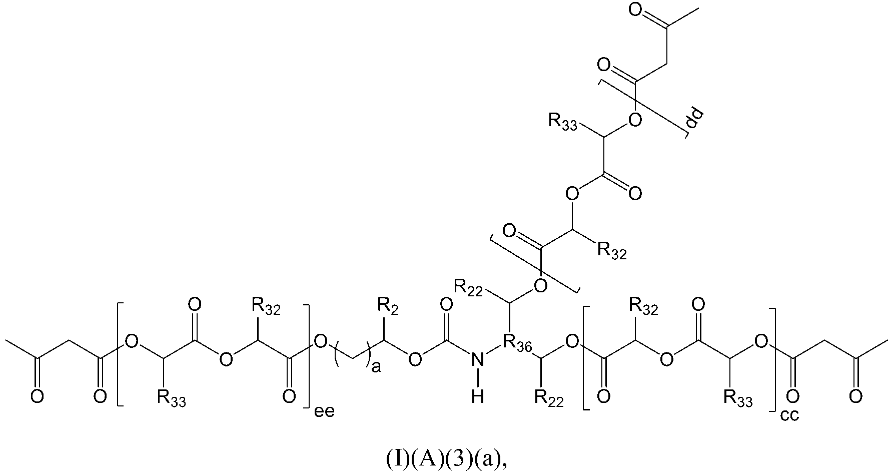 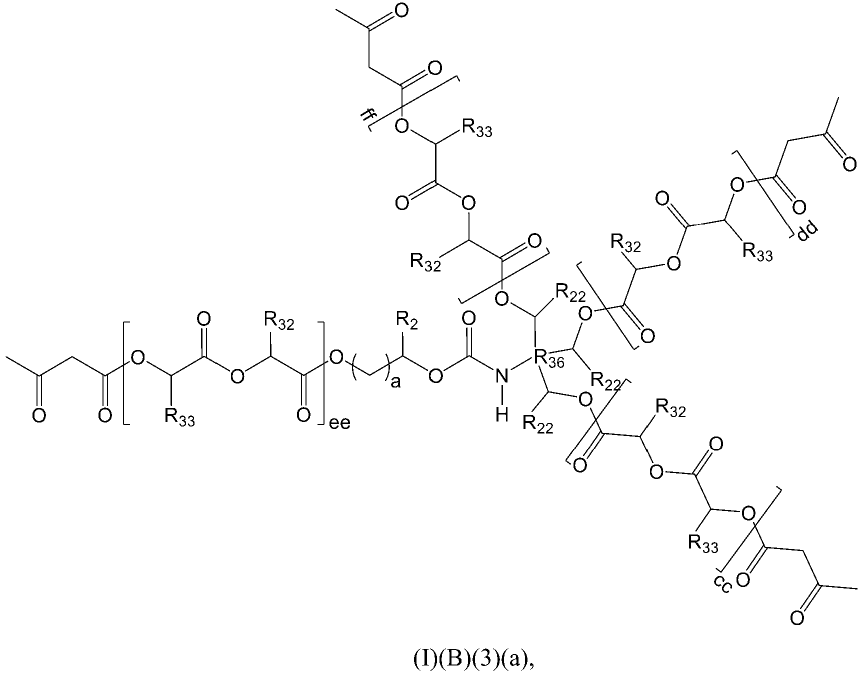 where R₂ can be monovalent and can be a hydrogen, methyl, or 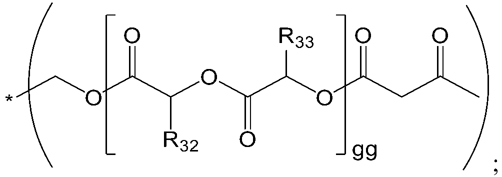 R₂₂ can be monovalent and can be a hydrogen or an alkyl; R₃₂ and R₃₃ can each independently be monovalent and can each independently be a hydrogen or a methyl; R₃₆ can be trivalent or tetravalent and can be a linear or branched alkyl, a substituted or unsubstituted cycloalkyl, or a substituted or unsubstituted aryl; a can be 1 or 2; and u can be 1 to 6. In structure (I)(A)(3)(a), cc, dd, ee, and gg (if present) can each be less than 10 and the sum of cc, dd, ee, and gg (if present) can be less than 10. In structure (I)(B)(3)(a), cc, dd, ee, ff, and gg (if present) can each be less than 10 and the sum of cc, dd, ee, ff, and gg (if present) can be less than 10.

The compound comprising polyacetoacetate functionality and at least one urethane linkage may comprise the following structure (II): 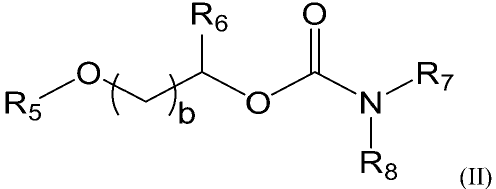 wherein R₅ can be monovalent and comprises an acetoacetate containing group, wherein R₆ can be monovalent and comprises a hydrogen, methyl, or an acetoacetate containing group, wherein R₇ can be monovalent, is bound to the nitrogen of the urethane linkage, and comprises at least one acetoacetate containing group, wherein R₈ can be monovalent, is bound to the nitrogen of the urethane linkage, and comprises at least one acetoacetate containing group, and wherein b may be 1 or 2.

The compound of Structure (II) may have the following exemplary structure: 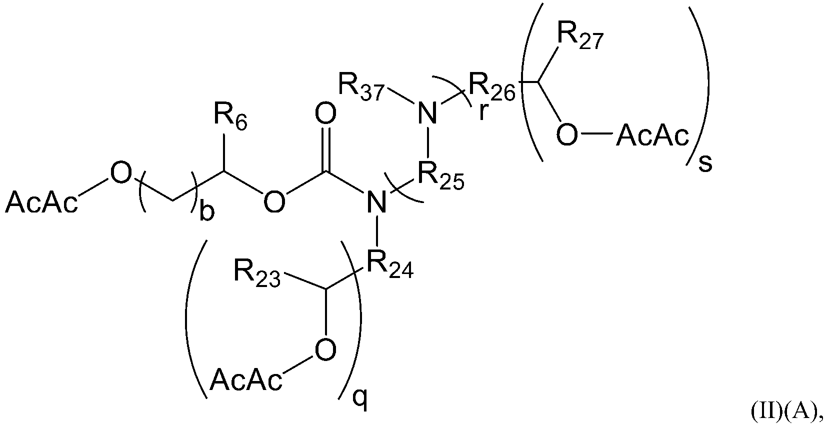 where R₆ can be monovalent and may be a hydrogen, a methyl, or an acetoacetate containing group, such as 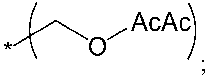 R₂₃ and R₂₇ can independently be monovalent and may independently be a hydrogen or an alkyl, such as C₁ to C₆ alkyl; R₂₄ and R₂₆ can be divalent, trivalent, or tetravalent and can independently be a linear or branched alkyl, such as linear or branched C₁ to C₆ alkyl; R₂₅ can be divalent and can be a linear or branched alkyl, such as linear or branched C₁ to C₆ alkyl; R₃₇ may be monovalent and may be 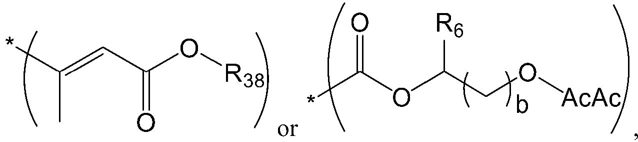 where R₃₈ may be monovalent and may be an alkyl, such as a methyl, ethyl, isobutyl, isopropyl, or *tert*-butyl; b may be 1 or 2; s and q may each independently be 1 to 3; and r may be 0 to 3.

The compound of Structure (II) may have the following exemplary structures: 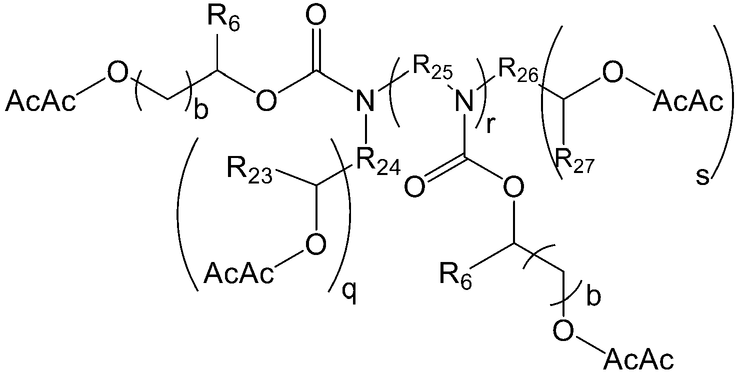 or 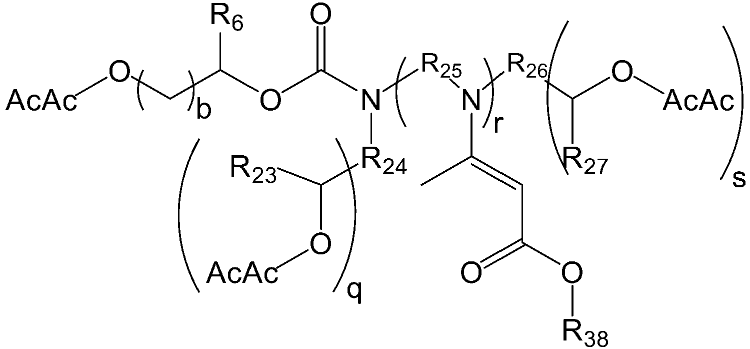 where R₆ may be monovalent and may be a hydrogen, a methyl, or an acetoacetate containing group, such as 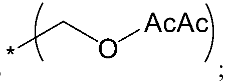 R₂₃ and R₂₇ may each independently be monovalent and may each independently be a hydrogen or an alkyl, such as C₁ to C₆ alkyl; R₂₄ and R₂₆ may each independently be divalent, trivalent, or tetravalent and may each independently be a linear or branched alkyl, such as, linear or branched C₁ to C₆ alkyl; R₂₅ may be divalent and may be a linear or branched alkyl, such as, linear or branched C₁ to C₆ alkyl; R₃₈ may be monovalent and may be an alkyl, such as a methyl, ethyl, isobutyl, isopropyl, or *tert*-butyl; b may be 1 or 2; s and q may each independently be 1 to 3; and r may be 0 to 3. In structure (II)(A), the acetoacetate containing groups may be the same or different and may be: 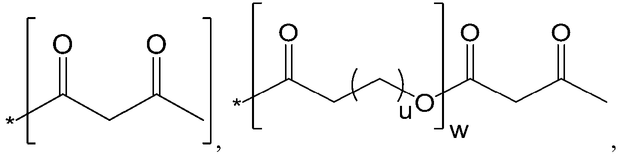 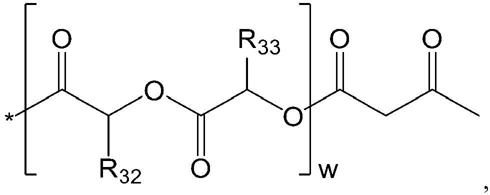 or some combination thereof, where u can be 1 or 2, w in each case can be less than 10, such as less than 5, such as less than 2, and R₃₂ and R₃₃ can each independently be monovalent and can each independently be a hydrogen or a methyl.

The compound of Structure II may be prepared by the reaction of (a) at least one cyclic carbonate compound, (b) at least one polyol having one or more secondary amines, and (c) optionally at least one lactone or at least one lactone cyclic di-ester to form a polyol-functional urethane compound. A compound comprising an acetoacetate functionality may be reacted with one or more hydroxyl groups of the polyol-functional urethane compound to introduce acetoacetate functionality into the polyol-functional urethane compound backbone to form the compound comprising polyacetoacetate functionality and at least one urethane linkage.

The (a) at least one cyclic carbonate compound may be any of the cyclic carbonates described above with respect to Structure (I).

The (b) at least one polyol having one or more secondary amines may comprise the following structure: 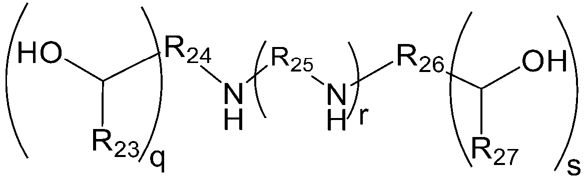 where R₂₃ and R₂₇ may each independently be monovalent and may each independently comprise a hydrogen or an alkyl; R₂₄ and R₂₆ may each independently be divalent, trivalent, or tetravalent and may each independently comprise an alkyl; R₂₅ may be divalent and may comprise an alkyl; q and s may each independently be 1 to 3; and r may be 0 to 3. In some examples, q and s may each independently be 1. Non-limiting examples of the (b) polyol having one or more secondary amines include diethanolamine, 2,2'-iminobis-1,3-propanediol, 1,3-Bis[tris(hydroxymethyl)methylamino]propane, 2-(2-hydroxyethylamino)propane-1,3-diol, 3-(3-hydroxy-propylamino)-propan-1-ol, 2-[(2-hydroxyethylamino)methylamino]ethanol, N'-(2-hydroxyethyl)ethylenediamine, 1,1'-(ethylenediimino)dipropan-2-ol, or some combination thereof. The (b) at least one polyol having one or more secondary amines may also be 1-(1-hydroxyethylamino)ethanol.

The (b) at least one polyol having one or more secondary amines may also be used to form the compound of Structure (I), such as Structures (I)(C) and (I)(D) described above, when r is 0 and s is 2 or 3.

The optional (c) at least one lactone or at least one lactone cyclic di-ester may be any of the lactones or lactone cyclic di-esters described above with respect to Structure (I).

In a first reaction step, (a) at least one cyclic carbonate compound and (b) at least one polyol having one or more secondary amines may be reacted to form a polyol-functional urethane compound. The reaction mixture used to prepare the polyol-functional urethane compound is substantially free of (c) at least one lactone or at least one lactone or at least one lactone cyclic di-ester. The polyol-functional urethane compound may comprise one of the following exemplary structures: 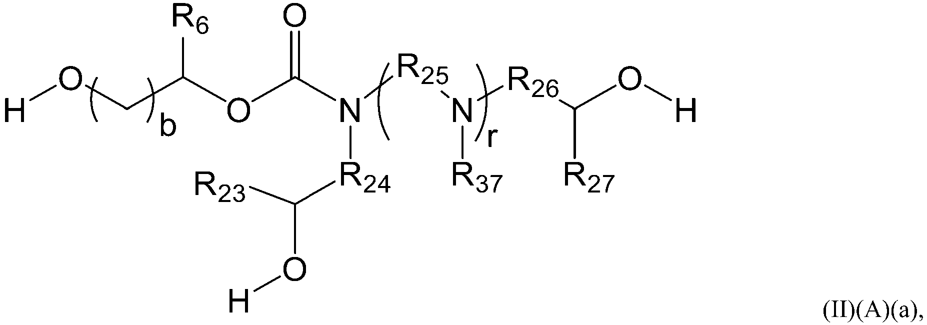 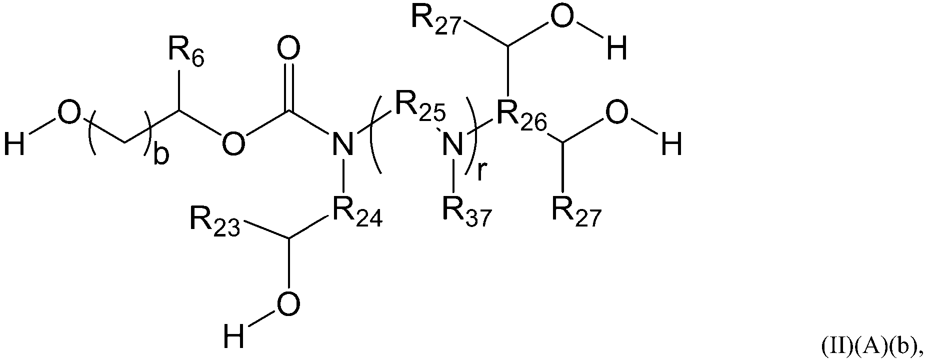 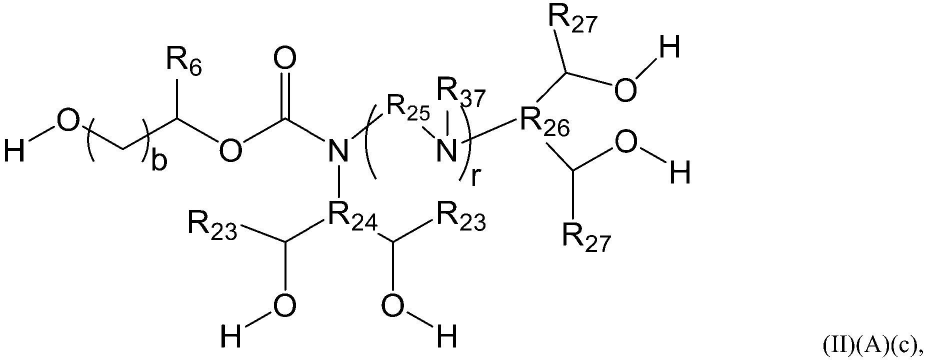 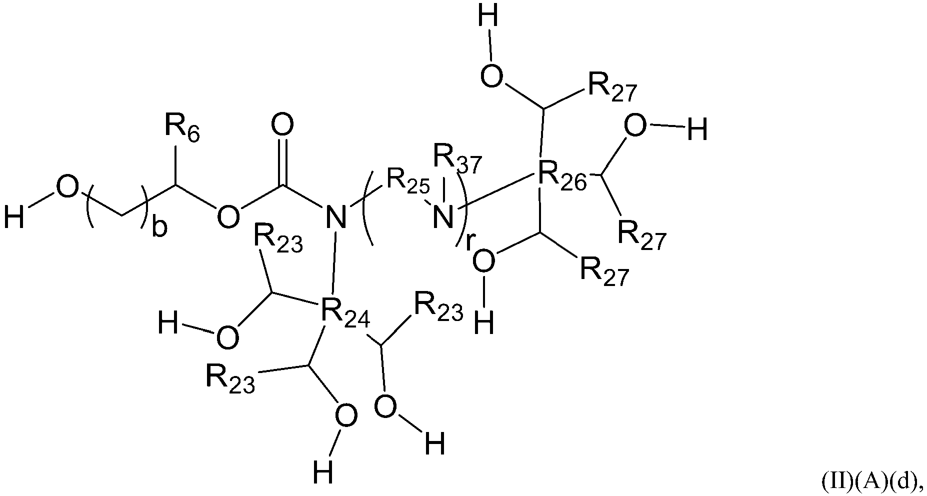 where R₆ may be monovalent and may be a hydrogen, a methyl, or a hydroxymethyl; R₂₃ and R₂₇ may each independently be monovalent and may each independently be a hydrogen or an alkyl; R₂₄ and R₂₆ may each independently be divalent, trivalent, or tetravalent and may each independently be a linear or branched alkyl; R₂₅ may be divalent and may be a linear or branched alkyl; R₃₇ may be 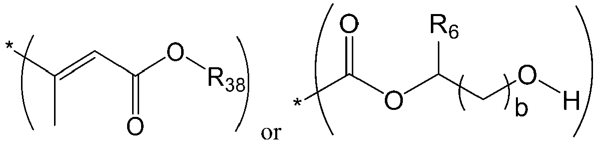 where R₃₈ may be monovalent and may be an alkyl, such as a methyl, ethyl, isobutyl, isopropyl, or *tert*-butyl; b may be 1 or 2; and r is from 0 to 3.

To form the compound of Structure (II), the polyol-functional urethane compound may be reacted with a compound comprising an acetoacetate functionality to introduce acetoacetate functionality into the polyol-functional urethane compound backbone. The compound comprising an acetoacetate functionality may be any of the compounds comprising an acetoacetate functionality described above with respect to Structure (I). The compound comprising polyacetoacetate functionality and at least one urethane linkage according to Structure (II) may comprise one of the following exemplary structure: 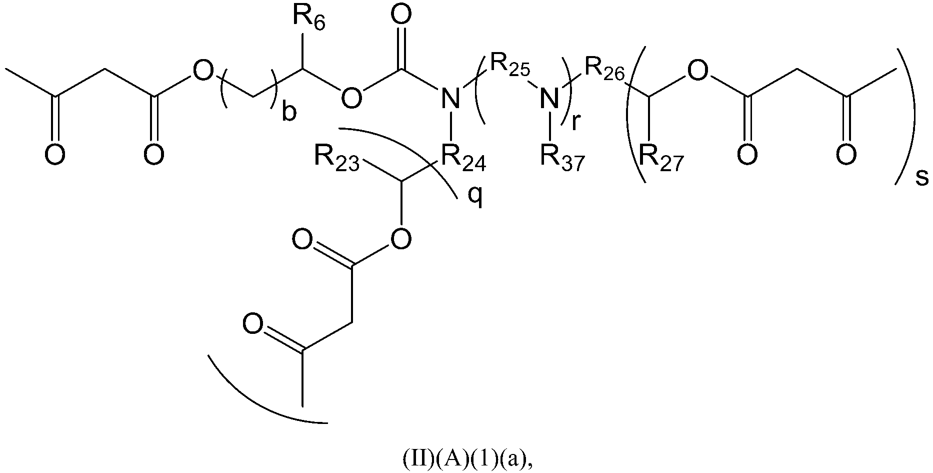 where R₆ may be monovalent and may be a hydrogen, a methyl, or 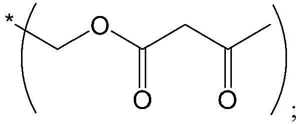 R₂₃ and R₂₇ may each independently be monovalent and may be a hydrogen or an alkyl; R₂₄ and R₂₆ may each independently be divalent, trivalent, or tetravalent and may each independently be a linear or branched alkyl; R₂₅ may be divalent and may be a linear or branched alkyl; R₃₇ may be monovalent and may be 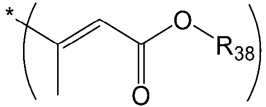 or 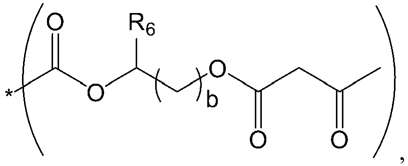 where R₃₈ may be monovalent and may be an alkyl, such as a methyl, ethyl, isobutyl, isopropyl, or *tert*-butyl; b may be 1 or 2; q and s may each independently be 1 to 3; and r may be 0 to 3.

In an optional second reaction step, the reaction product of the first reaction may be further reacted with (c) at least one lactone, such as those described above, to form a polyol-functional urethane compound. The second reaction step may include a metal-based catalyst, such as those described above. The polyol-functional urethane compound formed from the first and second reaction steps, may comprise the following exemplary structure when each polyol functional group reacts with a lactone: 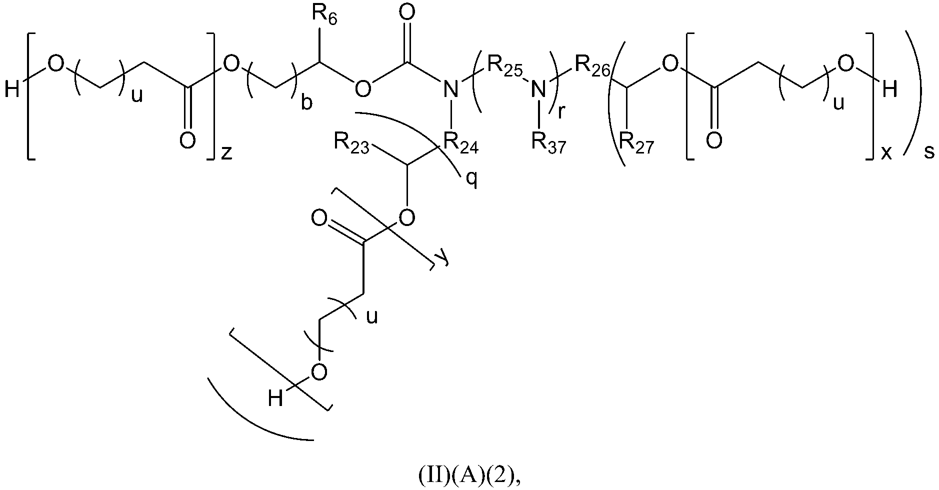 wherein R₆ may be monovalent and may be a hydrogen, a methyl, or 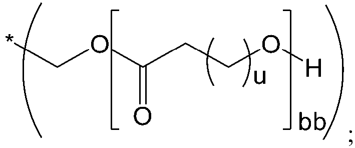 R₂₃ and R₂₇ may each independently be monovalent and may each independently be a hydrogen or an alkyl; R₂₄ and R₂₆ may each independently be divalent, trivalent, or tetravalent and may each independently be a linear or branched alkyl; R₂₅ may be divalent and may be a linear or branched alkyl; R₃₇ may be monovalent and may be 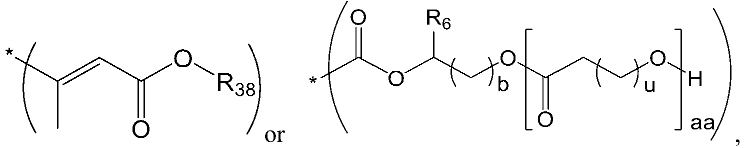 where R₃₈ may be monovalent and may be an alkyl, such as a methyl, ethyl, isobutyl, isopropyl, or *tert*-butyl; b may be 1 or 2; q and s may each independently be 1 to 3; r may be 0 to 3; and u may be 1 or 2. In Structure (II)(A)(2), x, y, z, aa, and bb (if present) can each be less than 10 and the sum of x, y, z, aa (if present), and bb (if present) can be less than 10. In some non-limiting examples, all of the hydroxyl groups on the polyol-functional urethane compound may not react with the (c) lactone, leaving free hydroxyl groups on the poly-functional urethane compound. All of the hydroxyl groups on the polyol-functional urethane compound may not react with the (c) lactone because of stoichiometry, or the ratio of initial hydroxyl groups relative to the amount of lactone added.

To form the compound of Structure (II), the above polyol-functional urethane compounds may be reacted with the previously described compound comprising an acetoacetate functionality to introduce acetoacetate functionality into the polyol-functional urethane compound backbone. The compound comprising polyacetoacetate functionality and at least one urethane linkage according to Structure (II) may comprise the following exemplary structure: 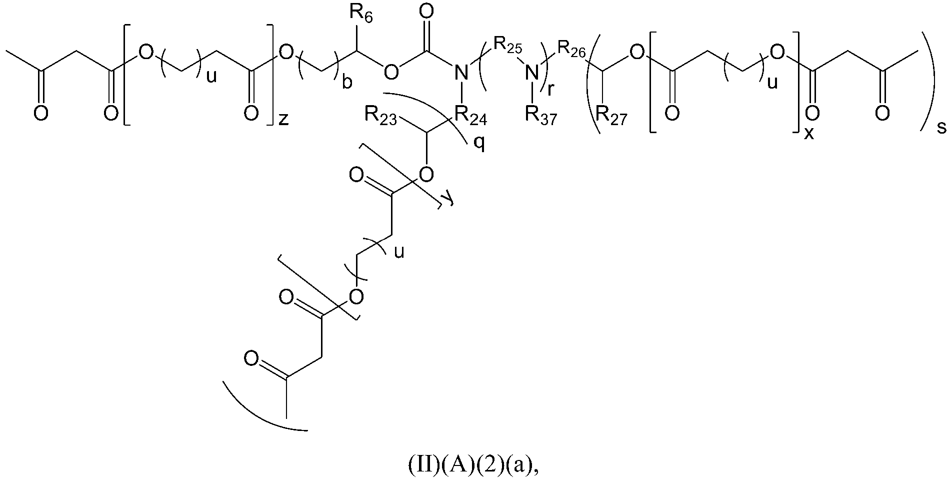 wherein R₆ may be monovalent and may be a hydrogen, a methyl, or 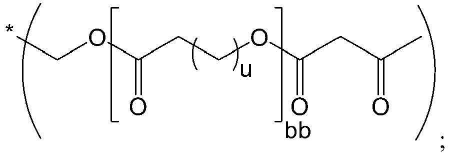 R₂₃ and R₂₇ may each independently be monovalent and may each independently be a hydrogen or an alkyl; R₂₄ and R₂₆ may each independently be divalent, trivalent, or monovalent and may each independently be a linear or branched alkyl; R₂₅ may be divalent and may be a linear or branched alkyl; R₃₇ may be monovalent and may be 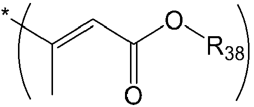 or 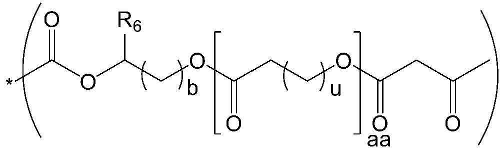 where R₃₈ may be monovalent and may be an alkyl, such as a methyl, ethyl, isobutyl, isopropyl, or *tert*-butyl; b may be 1 or 2; q and s may each independently be 1 to 3; r may be 0 to 3; u may be 1 or 2; x, y, z, aa (if present), and bb (if present) can each be less than 10, and the sum of x, y, z, aa, and bb (if present) can be less than 10.

In an optional second reaction step, the reaction product of the first reaction may be further reacted with (c) or at least one lactone cyclic di-ester, in the presence of a metal-based catalyst, such as those described previously. The polyol-functional urethane compound formed from the first and second reaction steps, may comprise the following exemplary structure when each hydroxyl of the polyol reacts with the at least one (c) lactone cyclic di-ester: 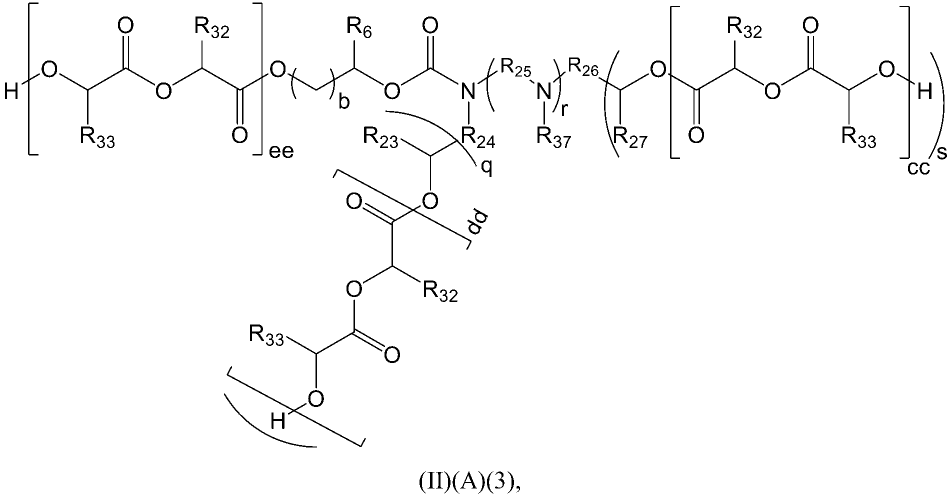 where R₆ can be monovalent and can be a hydrogen, a methyl, or 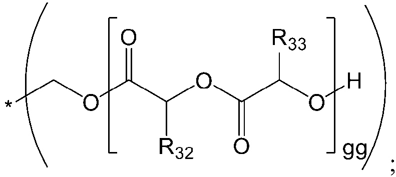 R₂₃ and R₂₇ may each independently be monovalent and may each independently be a hydrogen or an alkyl; R₂₄ and R₂₆ may each independently be divalent, trivalent, or tetravalent and may each independently be a linear or branched alkyl; R₂₅ may be divalent and may be a linear or branched alkyl; R₃₇ may be monovalent and may be 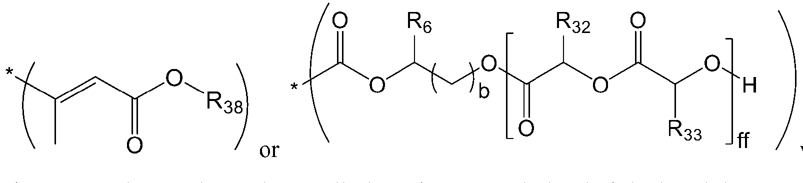 where R₃₈ may be monovalent and may be an alkyl, such as a methyl, ethyl, isobutyl, isopropyl, or *tert-*butyl; R₃₂ and R₃₃ may each independently be monovalent and may each independently be a hydrogen or a methyl; b may be 1 or 2; q and s may each independently be 1 to 3; r may be 0 to 3; cc, dd, ee, ff (if present), and gg (if present) can each be less than 10 and the sum of cc, dd, ee, ff, and gg (if present) can be less than 10. In some non-limiting examples, all the hydroxyls on the polyol-functional urethane compound may not react with the lactone cyclic di-ester, leaving free hydroxyl groups on the poly-functional urethane compound. All of the hydroxyl groups on the polyol-functional urethane compound may not react with the (c) lactone cyclic di-ester because of stoichiometry, or the ratio of initial hydroxyl groups relative to the amount of lactone added.

To form the compound of Structure (II), the above polyol-functional urethane compounds may be reacted with the previously described compound comprising an acetoacetate functionality to introduce acetoacetate functionality into the polyol-functional urethane compound backbone. The compound comprising polyacetoacetate functionality and at least one urethane linkage according to Structure (II) may comprise the following exemplary structure: 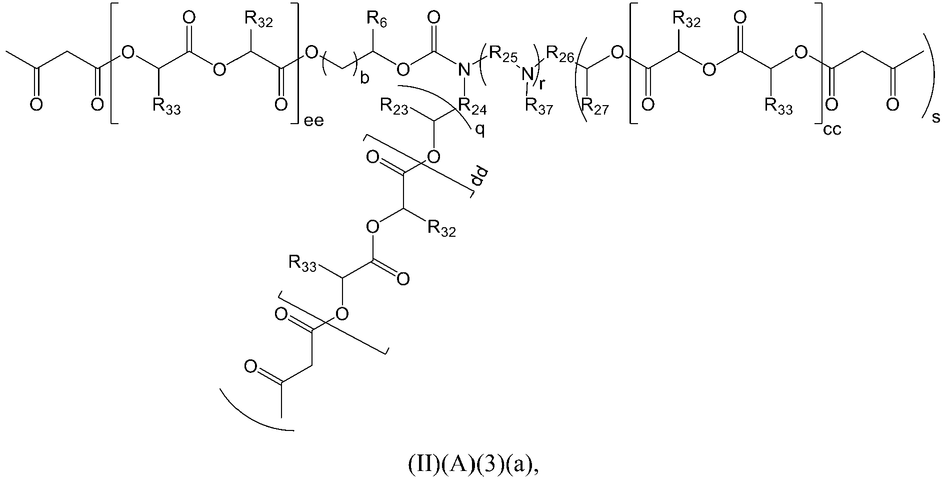 where R₆ can be monovalent and can be a hydrogen, a methyl, or 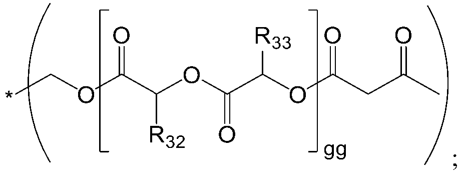 R₂₃ and R₂₇ may each independently be monovalent and may each independently be a hydrogen or an alkyl; R₂₄ and R₂₆ may each independently be divalent, trivalent, or tetravalent and may each independently be a linear or branched alkyl; R₂₅ may be divalent and may be a linear or branched alkyl; R₃₇ may be monovalent and may be 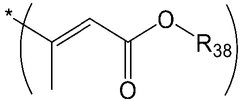 or 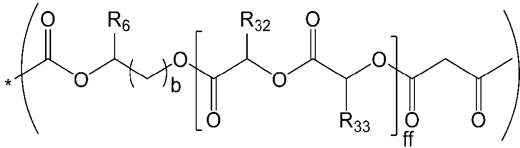 where R₃₈ may be monovalent and may be an alkyl, such as a methyl, ethyl, isobutyl, isopropyl, or *tert*-butyl; R₃₂ and R₃₃ may each independently be monovalent and may each independently be a hydrogen or a methyl; b may be 1 or 2; q and s may each independently be 1 to 3; r may be 0 to 3; cc, dd, ee, ff (if present), and gg (if present) can each be less than 10 and the sum of cc, dd, ee, ff, and gg (if present) can be less than 10.

For example, the compound comprising polyacetoacetate functionality and at least one urethane linkage may comprise the following structure (III): 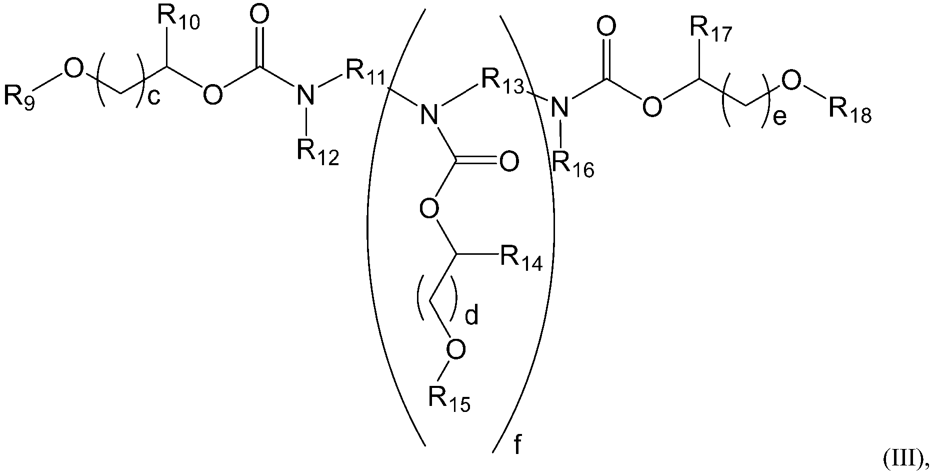 where R₉, R₁₅, and R₁₈ may each independently be monovalent and may each independently comprise an acetoacetate containing group; R₁₀, R₁₄, and R₁₇ may each independently be monovalent and may each independently comprise a hydrogen, methyl, or acetoacetate containing group; R₁₁ may be divalent and is bound to the nitrogen of the urethane linkage and comprises an alkyl, such as a C₁ to C₂₀ alkyl, such as up to a C₁₀ alkyl, such as up to a C₈ alkyl, such as up to a C₄ alkyl, a cycloalkyl, an aryl, 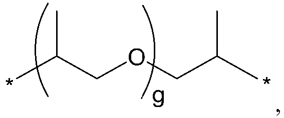 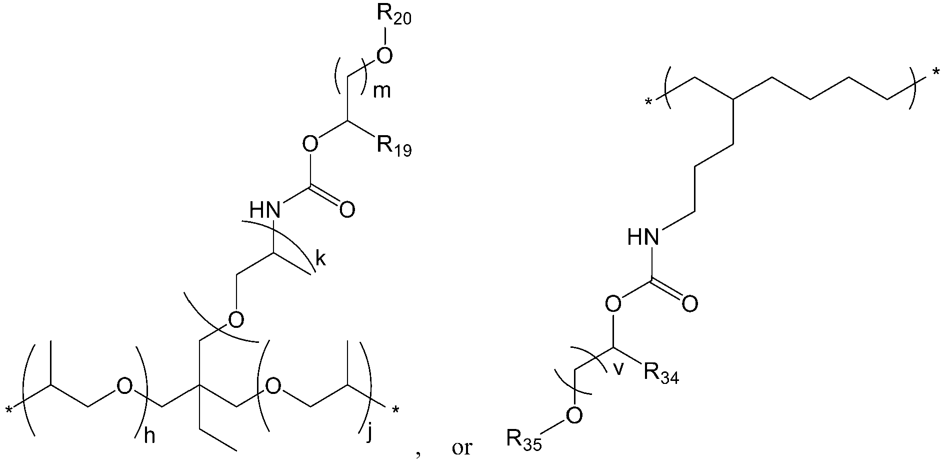 where g may be 2 to 70, the sum of h, j, and k may be 5 to 6, R₁₉ includes a hydrogen, methyl, or an acetoacetate containing group, R₂₀ may be monovalent and includes an acetoacetate containing group, m may be 1 or 2, R₃₄ may be monovalent and comprises a hydrogen, methyl, or an acetoacetate containing group, R₃₅ may be monovalent and comprises an acetoacetate containing group, and v comprises 1 or 2; R₁₂ and R₁₆ may each independently be monovalent and each independently comprise a hydrogen, an alkyl, such as a C₁ to C₂₀ alkyl, such as up to a C₁₀ alkyl, such as up to a C₈ alkyl, such as up to a C₄ alkyl, a cycloalkyl, or an aryl; R₁₃ can be divalent and is bound to the nitrogen of the urethane linkage and comprises an alkyl, such as a C₁ to C₁₂ alkyl; c, d, and e may each independently be 1 or 2; and f may be 0 to 3.

When R₉, R₁₅, and R₁₈ each independently comprise an acetoacetate containing group, R₁₀, R₁₄, and R₁₇ each independently comprise a hydrogen, methyl, or an acetoacetate containing group, R₁₁ comprises an alkyl, R₁₂ and R₁₆ each independently comprise a hydrogen, R₁₃ comprises an alkyl, c, d, and e each independently comprise 1 or 2, and f may be 0 to 3, the compound of Structure (III) may have one of the following exemplary structures: 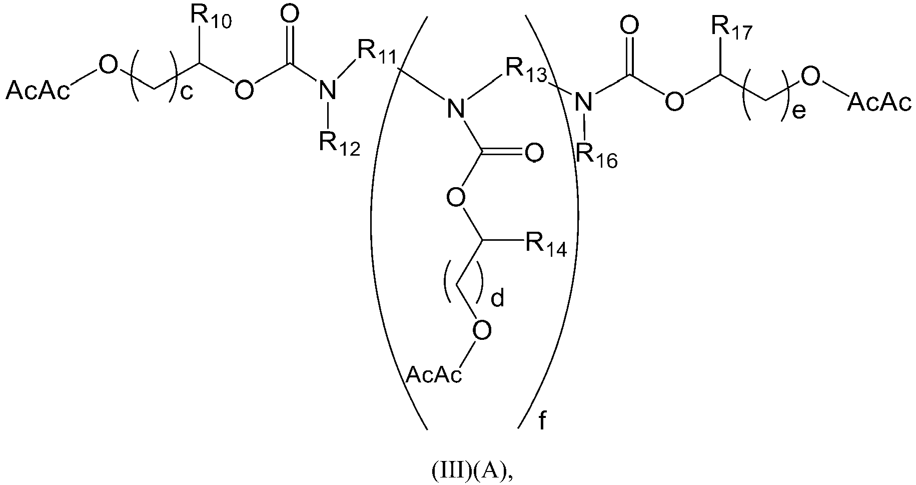 where the acetoacetate containing group of R₁₀, R₁₇, and R₁₄ may comprise the following structure:

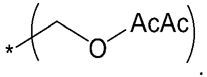

When R₉, R₁₅, and R₁₈ each independently comprise an acetoacetate containing group, R₁₀, R₁₄, and R₁₇ may each independently comprise a hydrogen, methyl, or acetoacetate containing group, f is 0, R₁₁ comprises 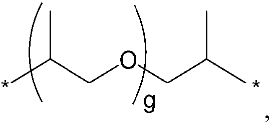 where g may be 2 to 70, R₁₂ and R₁₆ each independently comprise a hydrogen or an alkyl, and c and e each independently comprise 1 or 2, the compound of Structure (III) may have the following exemplary structure: 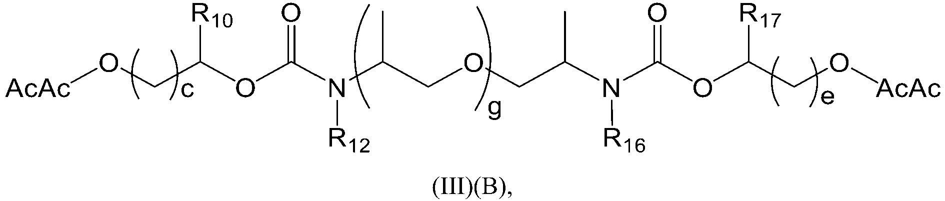 where the acetoacetate containing group of R₁₀ and R₁₇ may comprise the following structure:

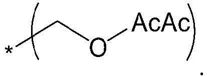

When R₉, R₁₅, and R₁₈ each independently comprise an acetoacetate containing group, R₁₀, R₁₄, and R₁₇ each independently comprise a hydrogen, methyl, or acetoacetate containing group, f is 0, R₁₁ comprises 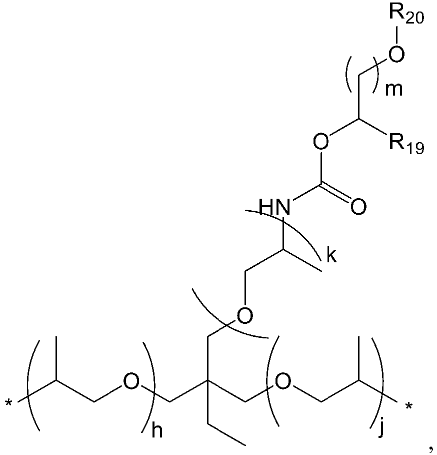 the sum of h, j, and k can be 5 to 6, R₁₉ comprises a hydrogen, methyl, or an acetoacetate containing group, R₂₀ comprises an acetoacetate containing group, m comprises 1 or 2, R₁₂ and R₁₆ each independently comprise a hydrogen or alkyl, and c and e each independently may be 1 or 2, the compound of Structure (III) may have the following exemplary structure: 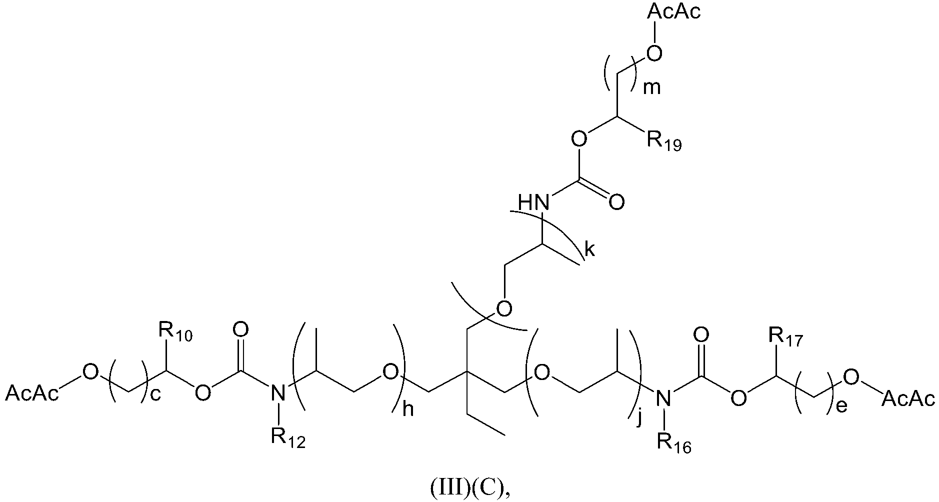 where the acetoacetate containing group of R₁₀, R₁₇, and R₁₉ may comprise the following structure:

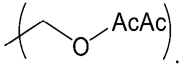

When R₉, R₁₅, and R₁₈ each independently comprise an acetoacetate containing group, R₁₂ and R₁₆ each independently comprise a hydrogen or alkyl, c and e each independently may be 1 or 2, R₁₀, R₁₄, and R₁₇ may each independently comprise a hydrogen, methyl, or acetoacetate containing group, f is 0, R₁₁ comprises 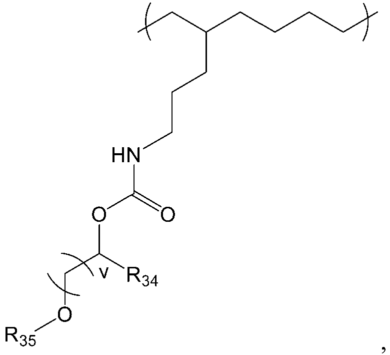 where R₃₄ comprises a hydrogen, methyl, or an acetoacetate containing group, R₃₅ comprises an acetoacetate containing group, and v comprises 1 or 2, the compound of Structure (III) may have the following exemplary structure: 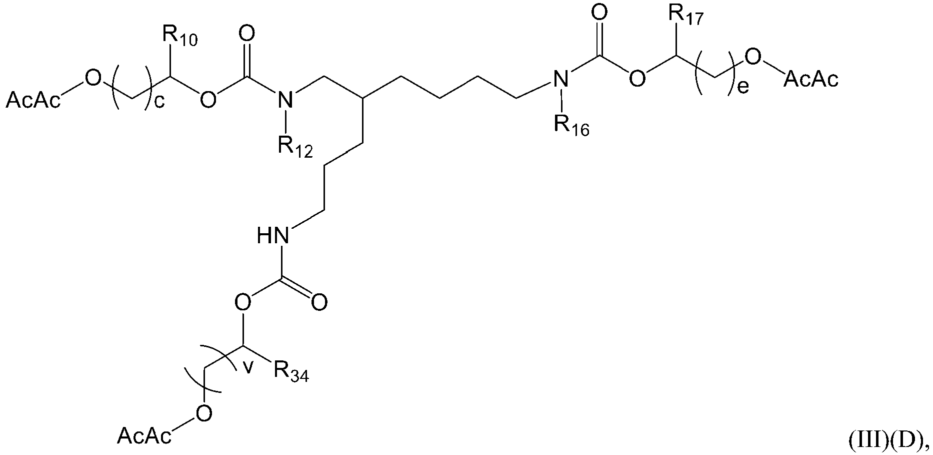 where the acetoacetate containing group of R₁₀, R₁₇, and R₃₄ may comprise the following structure: 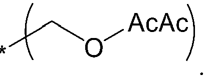 In structures (III)(A)-(D), the acetoacetate containing groups may be the same or different and may be: 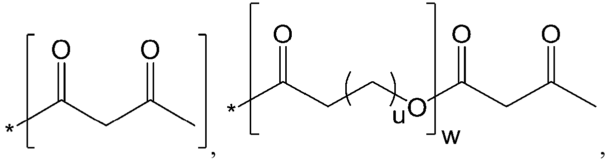 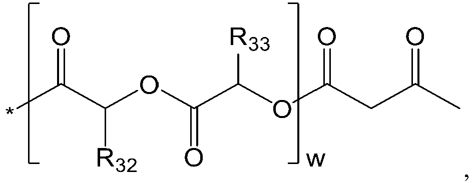 or some combination thereof, where u can be 1 or 2, w in each case can be less than 10, such as less than 5, such as less than 2, and R₃₂ and R₃₃ can each independently be monovalent and can each independently be a hydrogen or a methyl.

The compound of Structure (III) may be prepared by the reaction of (a) at least one cyclic carbonate compound, (b) at least one compound comprising two or more amine groups, and (c) optionally at least one lactone or at least one lactone cyclic di-ester to form a polyol-functional urethane compound. A compound comprising an acetoacetate functionality may be reacted with one or more hydroxyl groups of the polyol-functional urethane compound to introduce acetoacetate functionality into the polyol-functional urethane compound backbone to form the compound comprising polyacetoacetate functionality and at least one urethane linkage.

The (a) at least one cyclic carbonate compound may be any of the cyclic carbonates described above with respect to Structure (I).

The (b) at least one compound comprising two or more amine groups may comprise the following structure: 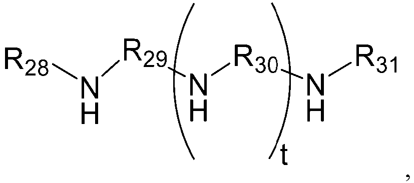 where R₂₈ and R₃₁ may each independently be monovalent and may each independently comprise a hydrogen, an alkyl, a cycloalkyl, or an aryl; R₂₉ may be divalent and may be an alkyl, a cycloalkyl, an aryl, 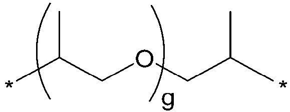 where g is 2 to 70, 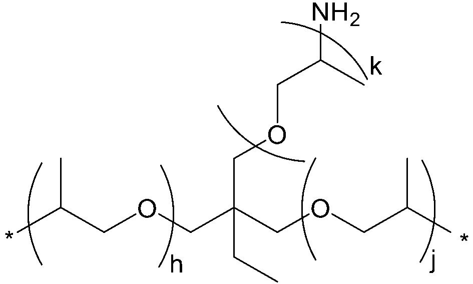 where the sum of h, j, and k may be 5 to 6, or 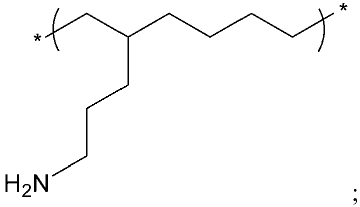 R₃₀ may be divalent and may be an alkyl; and t is 0 to 3.

When t is 0, R₂₉ may be divalent and may be:

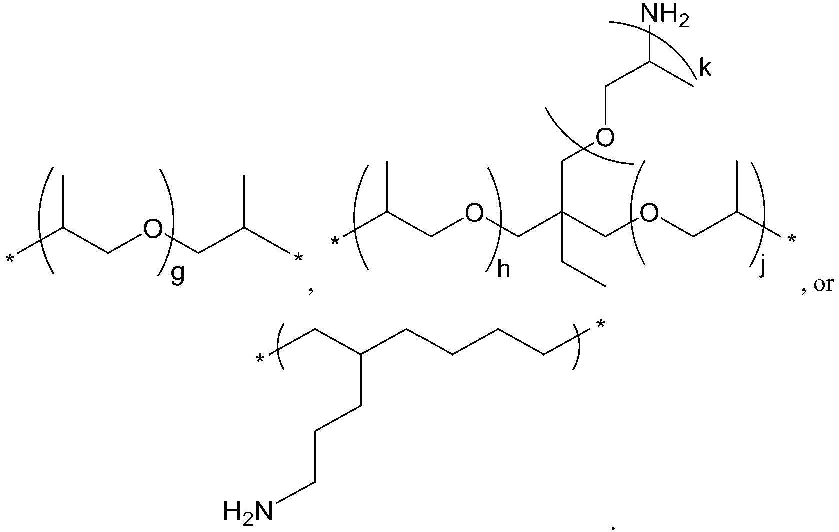

Non-limiting examples of (b) the compound comprising two or more amine groups includes ethylenediamine, trimethylenediamine, 1,4-butanediamine, diethylenetriamine, bis(3-aminopropyl)amine, bis(4-aminobutyl)amine, N-(2-aminoethyl)butane-1,4-diamine, N-(3-aminopropyl)-1,4-butanediamine, N-(aminoethyl)-1,3-propanediamine, triethylenetetramine, N,N'-bis(3-aminopropyl)ethylenediamine, N,N'-bis(4-aminobutyl)-1,2-ethanediamine, N,N'-bis(4-aminobutyl)-1,4-butanediamine, N-{3-[(3-aminopropyl)amino]propyl}butane-1,4-diamine, N'-[3-(4-aminobutylamino)propyl]butane-1,4-diamine, tetrapropylenepentamine, (4-aminobutyl)[3-({4-[(3-aminopropyl)amino]butyl}amino)propyl]amine, (4-aminobutyl)-[3-({4-[(4-aminobutyl)amino]butyl } amino)propyl]amine, N,N'-bis(3-aminopropyl)pentane-1,5-diamine, N'-(3-Aminopropyl)-N-[4-(3-aminopropylamino)butyl]butane-1,4-diamine, N-(3-aminopropyl)-N'-[3-(3-aminopropylamino)propyl]butane-1,4-diamine, N-(4-aminobutyl)-N'-(3-aminopropyl)- 1,4-butanediamine, 1, 5-pentanediamine, 4-(aminomethyl)octane-1,8-diamine, polyoxypropylene amines commercially available under the trademark designation JEFFAMINE^{®} from Huntsman Corporation, such as D-230, D-400, D-2000, and T-403, and mixtures thereof.

The optional (c) at least one lactone or at least one lactone cyclic di-ester may be any of the lactones or lactone cyclic di-esters described above with respect to Structure (I).

In a first reaction step, (a) at least one cyclic carbonate compound and (b) the compound comprising two or more amine groups may be reacted to form a polyol-functional urethane compound. The reaction mixture used to prepare the polyol-functional urethane compound is substantially free of (c) at least one lactone or at least one lactone or at least one lactone cyclic di-ester. The polyol-functional urethane compound may comprise one of the following exemplary structures: 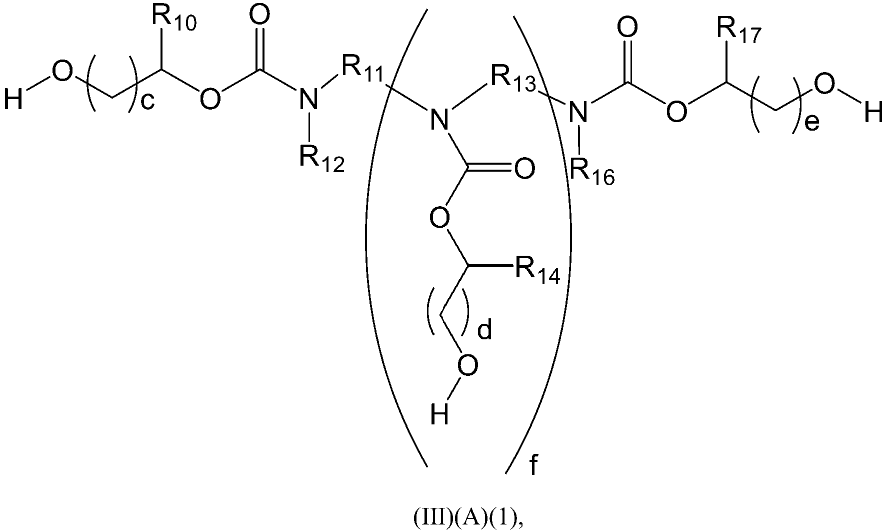 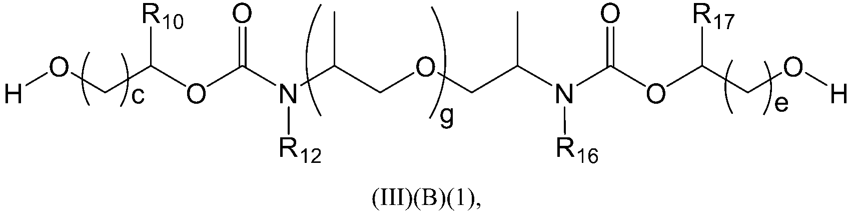 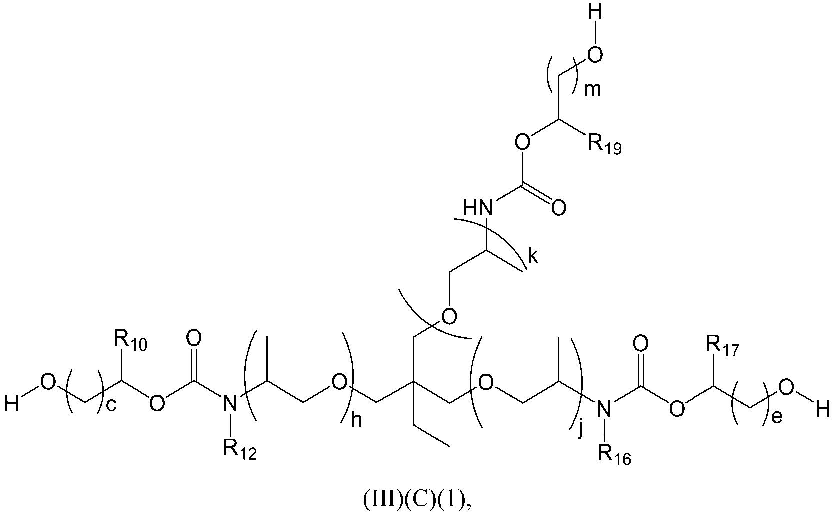 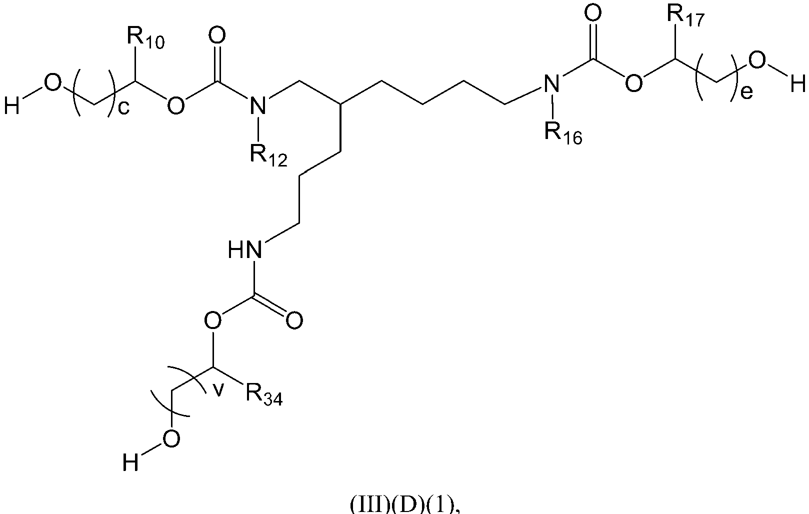 where R₁₀, R₁₄, R₁₇, R₁₉, and R₃₄ may each independently be monovalent and may each independently comprise a hydrogen, methyl, or hydroxymethyl; R₁₁ may be divalent and may be an alkyl, a cycloalkyl, or an aryl when f is from 0 to 3; R₁₂ and R₁₆ may each independently be monovalent and may each independently be a hydrogen, an alkyl, a cycloalkyl, or an aryl; R₁₃ may be divalent and may be an alkyl when f is 1 to 3; c, d, and e may each independently be 1 or 2; g may be 2 to 70; the sum of h, j, and k may be 5 to 6; m may be 1 or 2; and v may be 1 or 2.

To form the compound of Structure (III), the polyol-functional urethane compound may be reacted with a compound comprising an acetoacetate functionality to introduce acetoacetate functionality into the polyol-functional urethane compound backbone. The compound comprising an acetoacetate functionality may be any of the compounds comprising an acetoacetate functionality described above with respect to Structure (I). The compound comprising polyacetoacetate functionality and at least one urethane linkage according to Structure (III) may comprise one of the following exemplary structures: 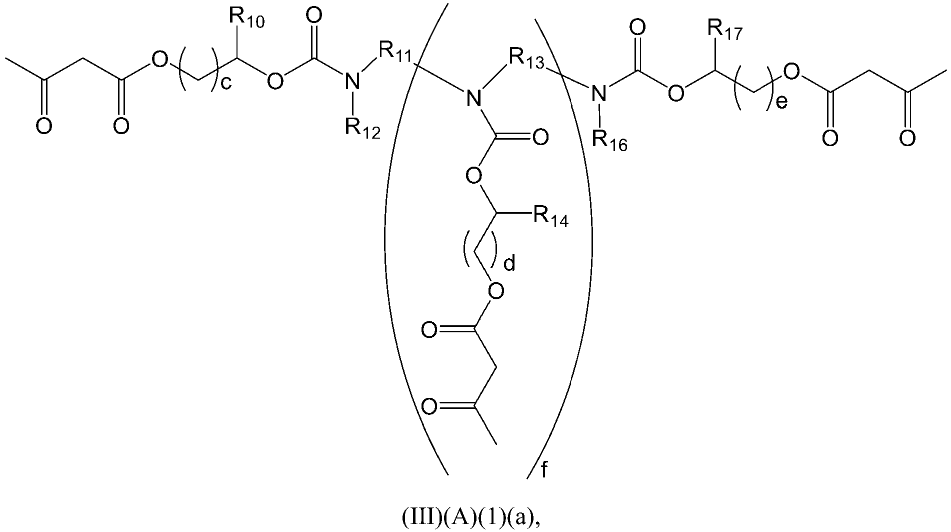 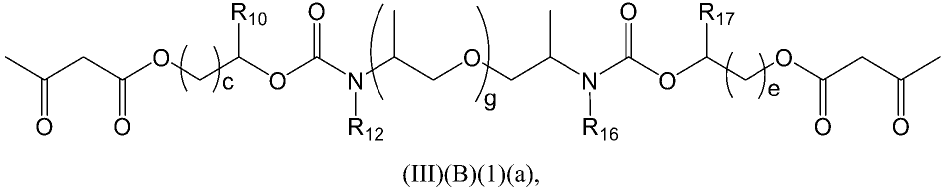 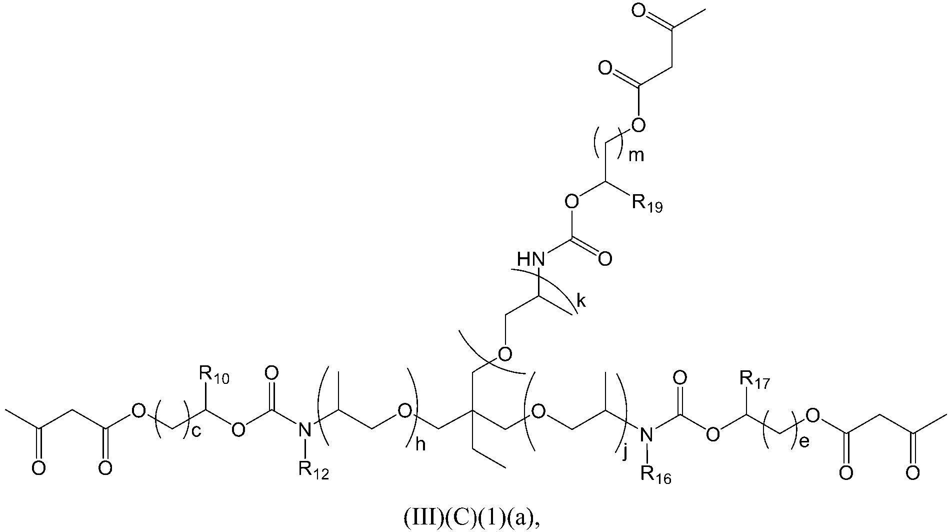 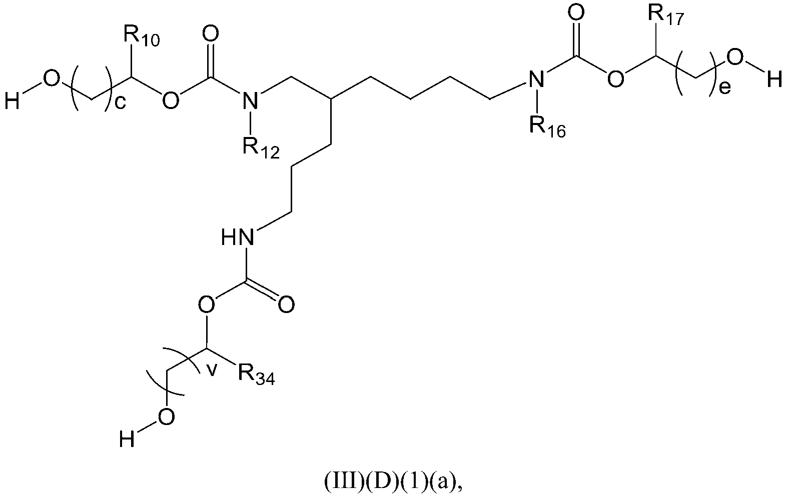 where R₁₀, R₁₄, R₁₇, R₁₉, and R₃₄ may each independently be monovalent and may each independently comprise a hydrogen, methyl, or 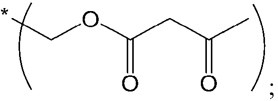 R₁₁ may be divalent and may be an alkyl, a cycloalkyl, or an aryl when f is from 0 to 3; R₁₂ and R₁₆ may be monovalent and may each independently comprise a hydrogen, an alkyl, a cycloalkyl, or an aryl; R₁₃ may be divalent and may be an alkyl when f is 1 to 3; c, d, and e may each independently be 1 or 2; g may be 2 to 70; the sum of h, j, and k may be 5 to 6; m may be 1 or 2; and v may be 1 or 2.

In an optional second reaction step, the reaction product of the first reaction may be further reacted with (c) at least one lactone, such as those described above, to form a polyol-functional urethane compound. The second reaction step may include a metal-based catalyst, such as stannous octoate, as described above. The polyol-functional urethane compound formed from the first and second reaction steps, may comprise one of the following exemplary structures when each hydroxyl of the polyol reacts with a lactone: 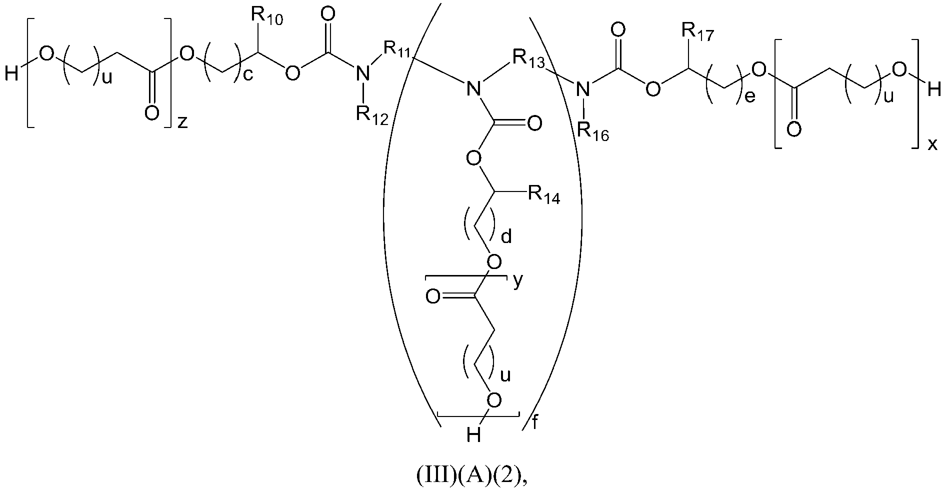 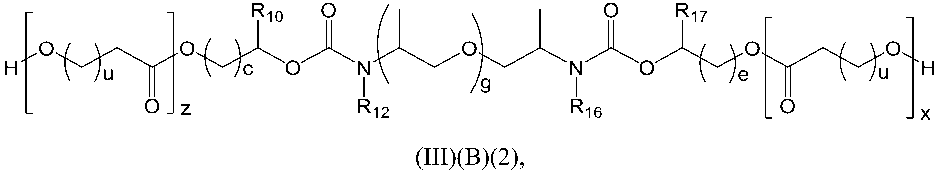 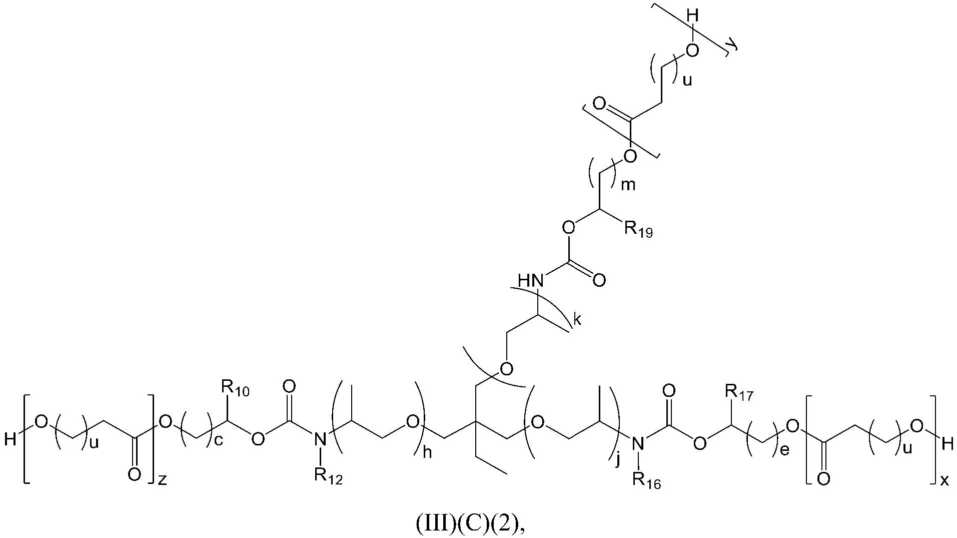 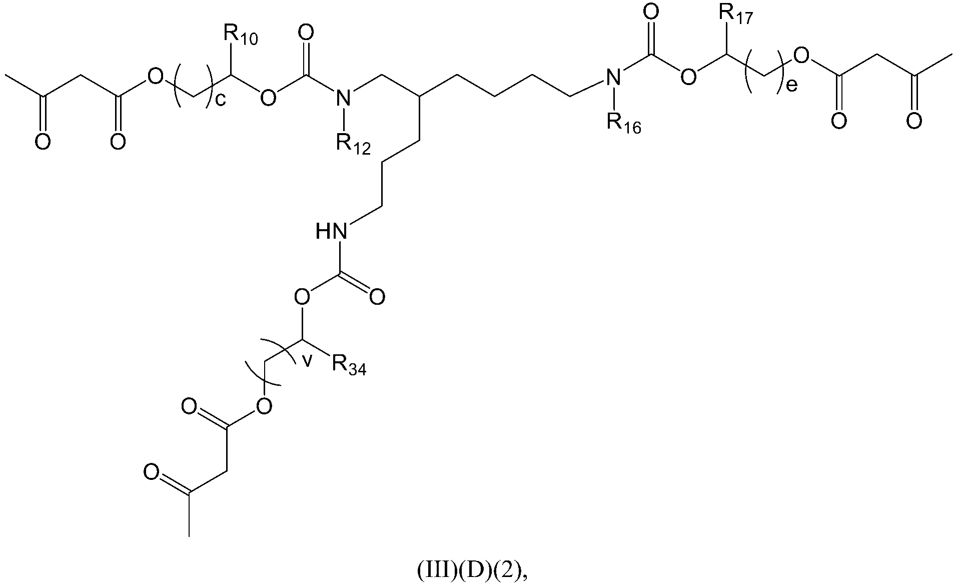 where R₁₀, R₁₄, R₁₇, R₁₉, and R₃₄ may each independently be monovalent and may each independently comprise a hydrogen, methyl, or 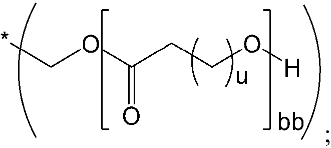 R₁₁ may be divalent and may be an alkyl, a cycloalkyl, or an aryl when f is from 0 to 3; R₁₂ and R₁₆ may each independently be a monovalent and may each independently be a hydrogen, an alkyl, a cycloalkyl, or an aryl; R₁₃ may be divalent and may be an alkyl when f is 1 to 3; c, d, and e may each independently be 1 or 2; g may be 2 to 70; the sum of h, j, and k may be 5 to 6; m may be 1 or 2, u may be 1 or 2; and v may be 1 or 2. In Structures (III)(A)(2), (III)(C)(2), and (III)(D)(2), x, y, z, and bb (if present) can each be less than 10 and the sum of x, y, z, and bb (if present) can be less than 10. In Structure (III)(B)(2), x, z, and bb (if present) can each be less than 10 and the sum of x, z, and bb (if present) can be less than 10. In some non-limiting examples, all the hydroxyls on the polyol-functional urethane compound may not react with the lactone, leaving free hydroxyl groups on the poly-functional urethane compound. All of the hydroxyl groups on the polyol-functional urethane compound may not react with the (c) lactone because of stoichiometry, or the ratio of initial hydroxyl groups relative to the amount of lactone added.

To form the compound of Structure (III), the above polyol-functional urethane compound may be reacted with the previously described compound comprising an acetoacetate functionality to introduce acetoacetate functionality into the polyol-functional urethane compound backbone. The compound comprising polyacetoacetate functionality and at least one urethane linkage according to Structure (III) may comprise one of the following exemplary structures: 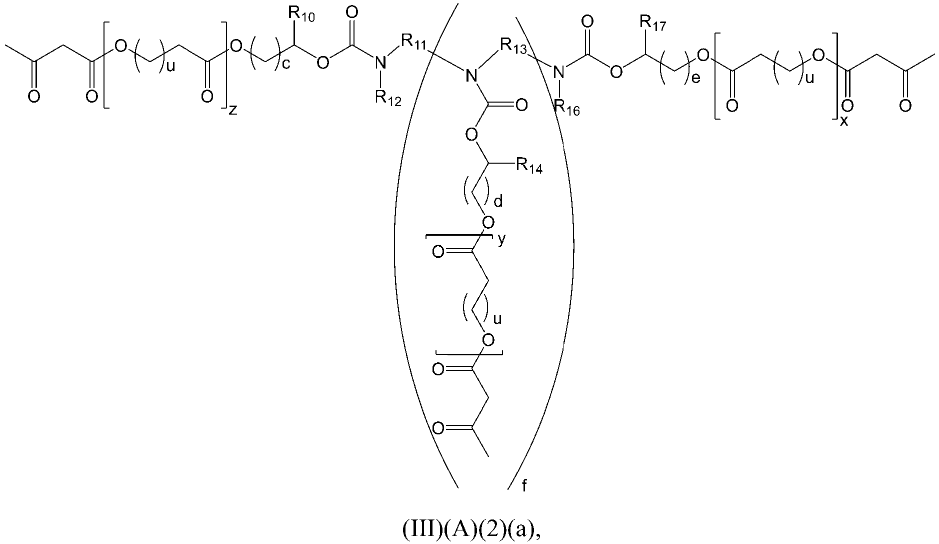 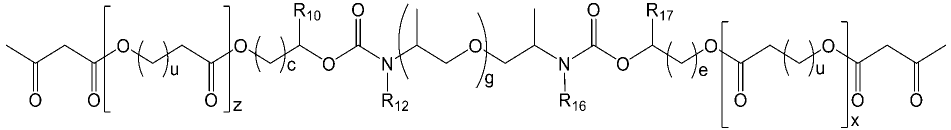 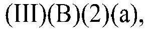 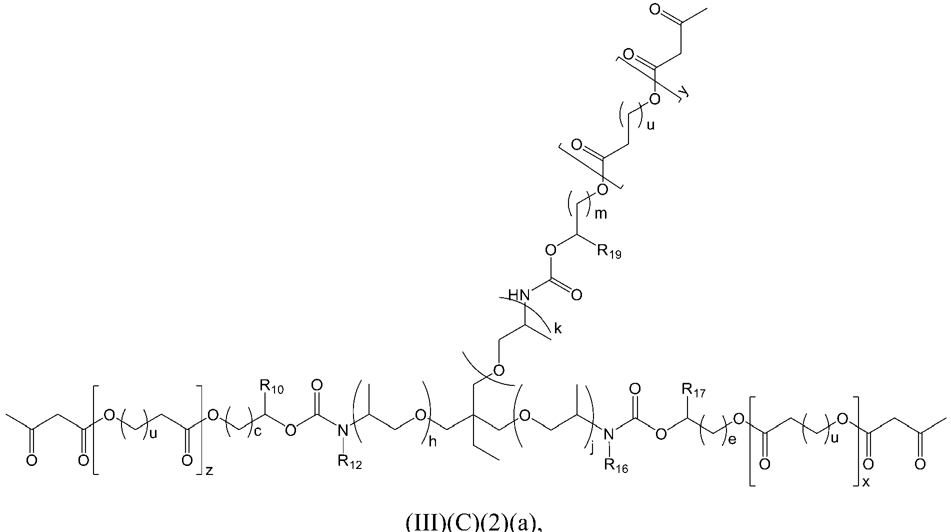 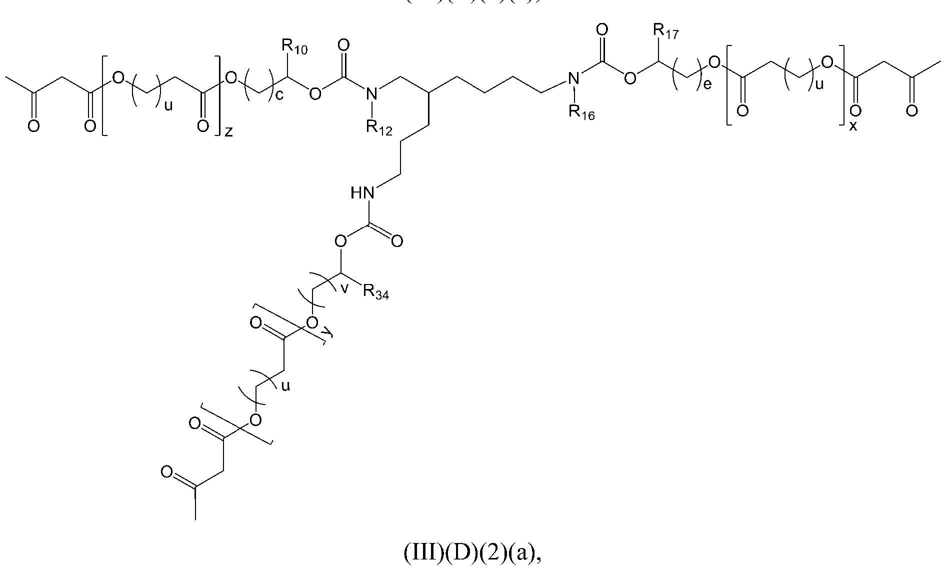 where R₁₀, R₁₄, R₁₇, R₁₉, and R₃₄ may each independently be monovalent and may each independently be a hydrogen, a methyl, or 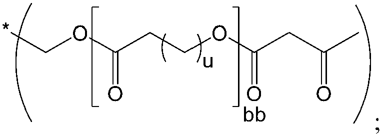 R₁₁ may be divalent and may be an alkyl, a cycloalkyl, or an aryl when f is from 0 to 3; R₁₂ and R₁₆ may each independently be monovalent and may each independently be a hydrogen, an alkyl, a cycloalkyl, or an aryl; R₁₃ may be divalent and may be an alkyl when f is 1 to 3; c, d, and e may each independently be 1 or 2; g may be 2 to 70; the sum of h, j, and k may be 5 to 6; m may be 1 or 2; and u may be 1 or 2. In structures (III)(A)(2)(a), (III)(C)(2)(a), and (III)(D)(2)(a), x, y, z, and bb (if present) can each be less than 10 and the sum of x, y, z, and bb (if present) can be less than 10. In structure (III)(B)(2)(a), x, z, and bb (if present) can each be less than 10 and the sum of x, z, and bb (if present) can be less than 10.

In an optional second reaction step, the reaction product of the first reaction may be further reacted with (c) or at least one lactone cyclic di-ester, in the presence of a metal-based catalyst, such as those described previously. The polyol-functional urethane compound formed from the first and second reaction steps, may comprise one of the following exemplary structures when each hydroxyl of the polyol reacts with a lactone cyclic di-ester: 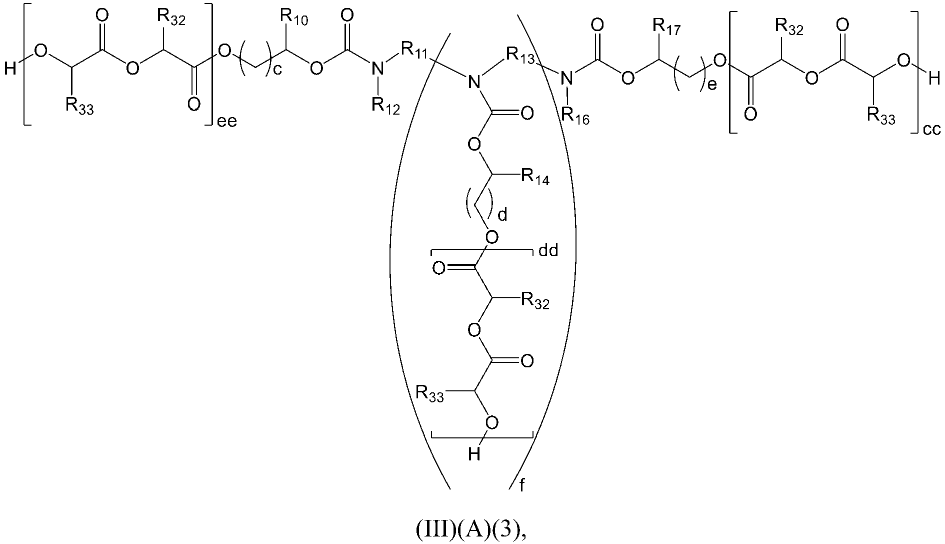 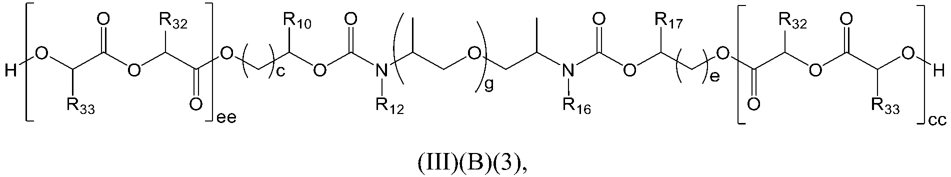 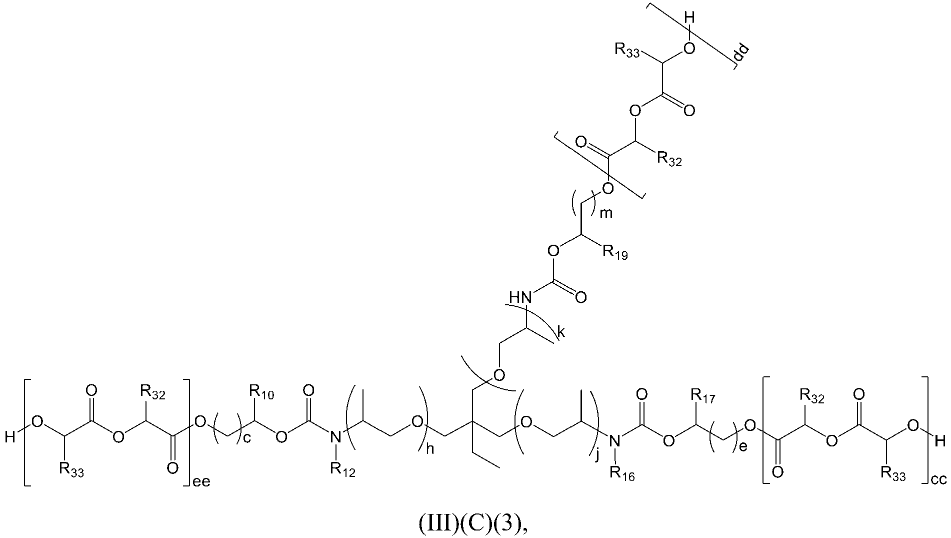 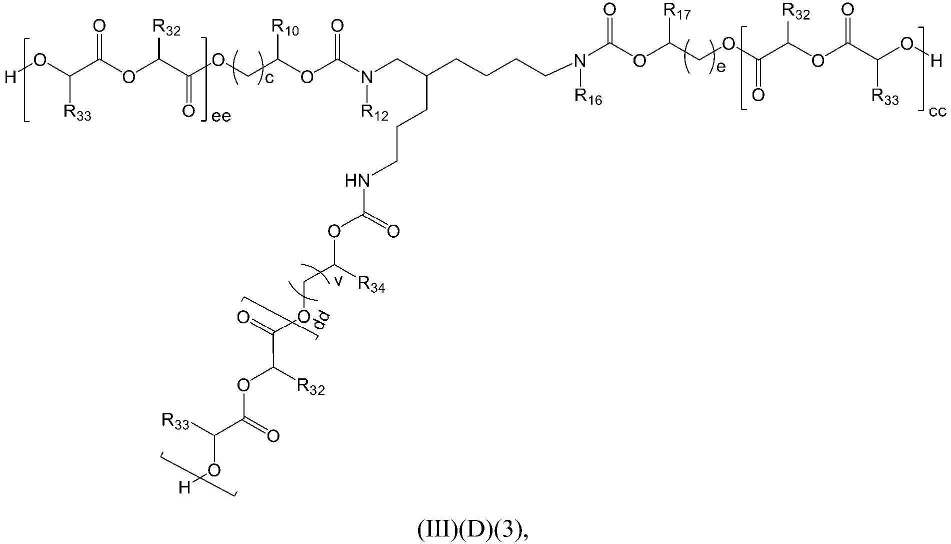 where R₁₀, R₁₄, R₁₇, R₁₉, and R₃₄ may each independently be monovalent and may each independently be a hydrogen, a methyl, or 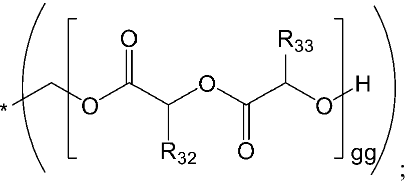 R₁₁ may be divalent and may be an alkyl, a cycloalkyl, or an aryl when f is from 0 to 3; R₁₂ and R₁₆ may each independently be monovalent and may each independently be a hydrogen, an alkyl, a cycloalkyl, or an aryl; R₁₃ may be divalent and may be an alkyl when f is 1 to 3; R₃₂ and R₃₃ may each independently be monovalent and may each independently be a hydrogen or a methyl; c, d, and e may each independently be 1 or 2; g may be 2 to 70; the sum of h, j, and k may be 5 to 6; m may be 1 or 2; and v may be 1 or 2. In Structures (III)(A)(3), (III)(C)(3), and (III)(D)(3), cc, dd, ee, and gg (if present) can each be less than 10 and the sum of cc, dd, ee, and gg (if present) is less than 10. In Structure (III)(B)(3), cc, ee, and gg (if present) can each be less than 10 and the sum of cc, ee, and gg (if present) is less than 10. In some non-limiting examples, all the hydroxyls on the polyol-functional urethane compound may not react with the lactone cyclic di-ester, leaving free hydroxyl groups on the poly-functional urethane compound. All of the hydroxyl groups on the polyol-functional urethane compound may not react with the (c) lactone cyclic di-ester because of stoichiometry, or the ratio of initial hydroxyl groups relative to the amount of lactone added.

To form the compound of Structure (III), the above polyol-functional urethane compounds may be reacted with the previously described compound comprising an acetoacetate functionality to introduce acetoacetate functionality into the polyol-functional urethane compound backbone. The compound comprising polyacetoacetate functionality and at least one urethane linkage according to Structure (III) may comprise one of the following exemplary structures: 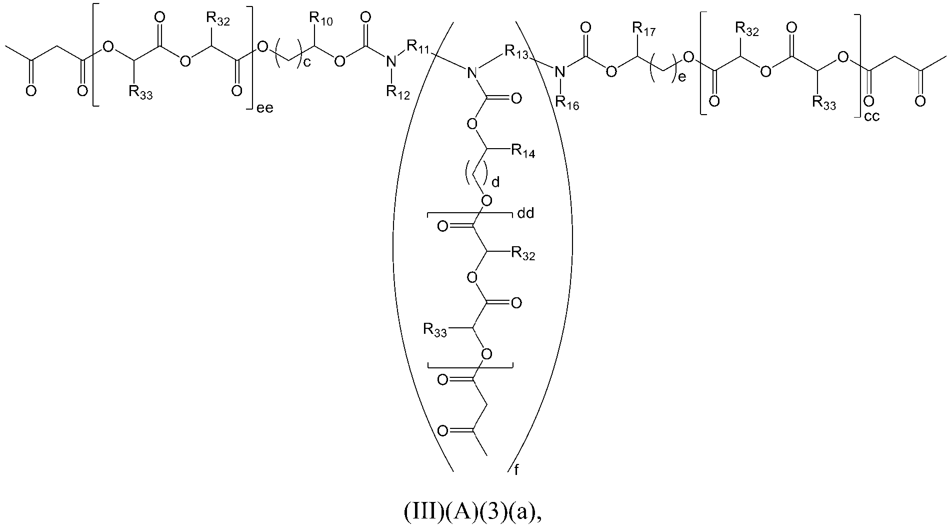 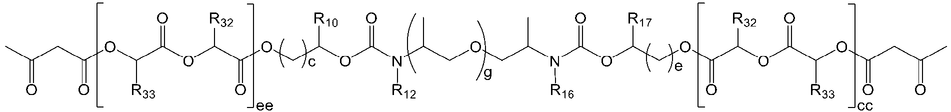 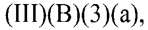 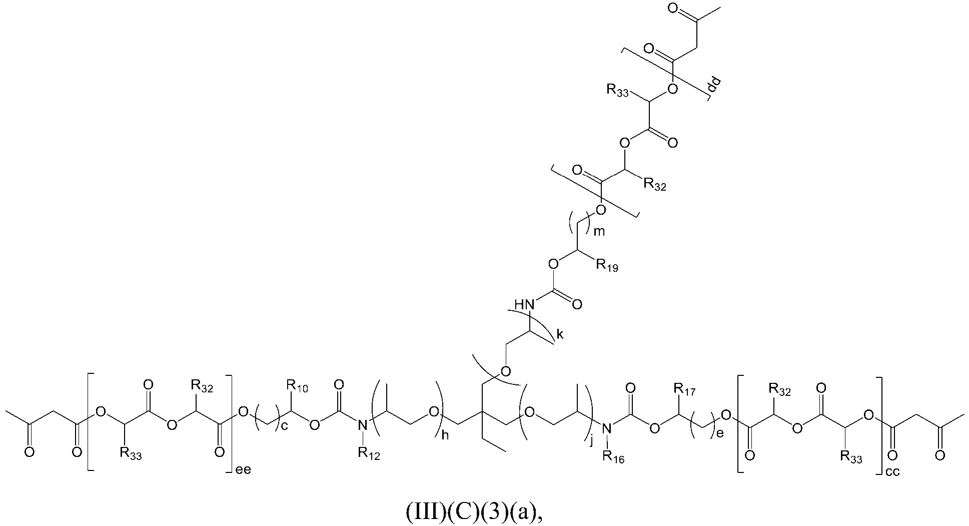 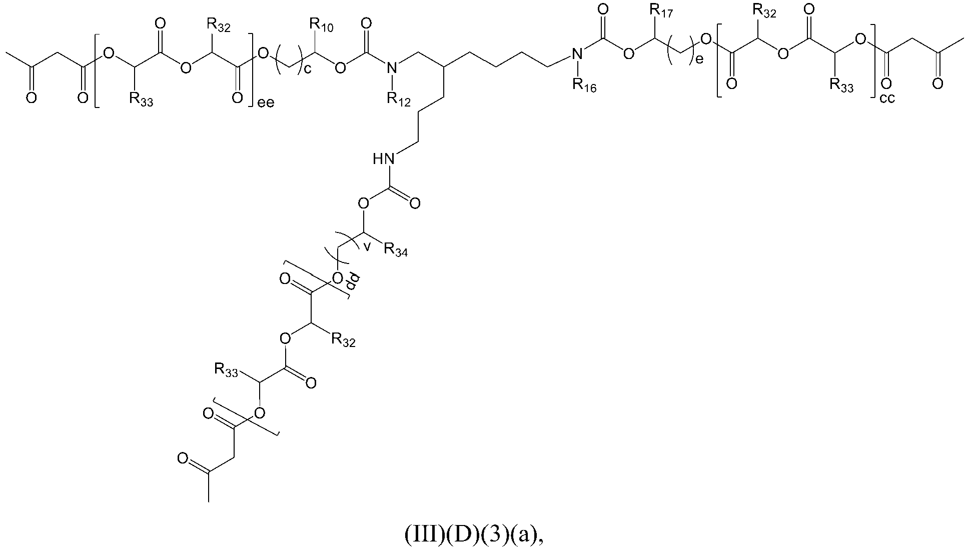 where R₁₀, R₁₄, R₁₇, R₁₉, and R₃₄ may each independently be monovalent and may each independently be a hydrogen, a methyl, or 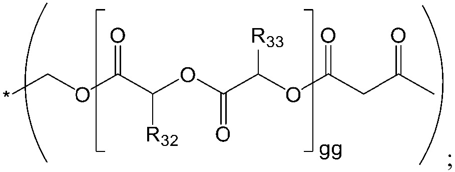 R₁₁ may be monovalent and may be an alkyl, a cycloalkyl, or an aryl when f is from 0 to 3; R₁₂ and R₁₆ may each independently be monovalent and may each independently be a hydrogen, an alkyl, a cycloalkyl, or an aryl; R₁₃ may be divalent and may be an alkyl when f is 1 to 3; R₃₂ and R₃₃ may each independently be monovalent and may each independently be a hydrogen or a methyl; c, d, and e may each independently be 1 or 2; g may be 2 to 70; the sum of h, j, and k may be 5 to 6; m may be 1 or 2; and v may be 1 or 2. In Structures (III)(A)(3)(a), (III)(C)(3)(a), and (III)(D)(3)(a), cc, dd, ee, and gg (if present) can each be less than 10 and the sum of cc, dd, ee, and gg (if present) is less than 10. In Structure (III)(B)(3)(a), cc, ee, and gg (if present) can each be less than 10 and the sum of cc, ee, and gg (if present) is less than 10.

The compound composition may also include versions of Structures (I)-(III) that are not fully converted to acetoacetate groups and at least a portion of polyol-functional urethane compound still comprises hydroxyl groups.

The compound comprising polyacetoacetate functionality and at least one urethane linkage can also comprise an acetoacetate equivalent weight of from 50 grams per equivalent (g/eq) to 700 g/eq, from 75 g/eq to 650 g/eq, from 100 g/eq to 600 g/eq, or from 125 g/eq to 550 g/eq, calculated by dividing the number average molecular weight (Mn) of the compound comprising the polyacetoacetate functionality and at least one urethane linkage by the equivalents of hydroxyls on the polyol-functional urethane compound. The compound comprising polyacetoacetate functionality and at least one urethane linkage can comprise an acetoacetate equivalent weight of at least 50 g/eq, such as at least 75 g/eq, at least 100 g/eq, or at least 125 g/eq. The compound comprising polyacetoacetate functionality and at least one urethane linkage can comprise an acetoacetate equivalent weight of up to 700 g/eq, up to 650 g/eq, up to 600 g/eq, or up to 550 g/eq.

The compound comprising polyacetoacetate functionality and at least one urethane linkage can also comprise a weight average molecular weight (Mw) of up to 2,000 grams per mole. The Mw or Mn, as reported herein, is measured by gel permeation chromatography using a polystyrene standard according to ASTM D6579-11 performed using a Waters 2695 separation module with a Waters 2414 differential refractometer (RI detector); tetrahydrofuran (THF) was used as the eluent at a flow rate of 1 ml/min, and two PLgel Mixed-C (300×7.5 mm) columns were used for separation at the room temperature; Mw and Mn molecular weight of polymeric samples can be measured by gel permeation chromatography relative to linear polystyrene standards of 800 to 900,000 Da.

The compound comprising polyacetoacetate functionality and at least one urethane linkage can also comprise a polydispersity index (PDI) value of at least 1 and up to 3. For example, the PDI may be up to 2.5, such as up to 2, such as up to 1.5 and at least 1.1, such as at least 1. The PDI may be at least 1 and up to 2.5, such as at least 1 and up to 2, such as at least 1.1 and up to 2.5, and at least 1.1 and up to 2. The PDI, as reported herein, calculated by dividing the Mw by the Mn.

The present invention is also directed to a method of preparing a compound comprising polyacetoacetate functionality and at least one urethane linkage, the method comprising: reacting (a) at least one cyclic carbonate compound, (b) at least one polyol having one primary amine and at least two hydroxyl groups, at least one polyol having one or more secondary amines, and/or at least one compound comprising two or more amine groups, and (c) optionally at least one lactone or at least one lactone cyclic di-ester to form a polyol-functional urethane compound; and reacting a compound comprising an acetoacetate functionality with a hydroxyl group of the polyol-functional urethane compound to introduce acetoacetate functionality into the polyol-functional urethane compound backbone to form the compound comprising polyacetoacetate functionality and at least one urethane linkage.

The (a) at least one cyclic carbonate compound and the (b) at least one polyol having one primary amine and at least two hydroxyl groups, at least one polyol having one or more secondary amines, and/or at least one compound comprising two or more amine groups may be reacted in a first reaction step to form the a polyol-functional urethane compound. The first reaction step may be completed at a temperature of from greater than 30°C to not greater than 190°C, such as from 40°C to 180°C, 50°C to 170°C, 60°C to 170°C, 70°C to 160°C, 80°C to 150°C, 90°C to 140°C, or 100°C to 130°C. The polyol-functional urethane compound from the first reaction step may be reacted with (c) at least one lactone or at least one lactone cyclic di-ester in a second reaction step. The second reaction step may be completed in the presence of a metal catalyst at a temperature of from greater than 50°C to not greater than 190°C, such as from 60°C to 180°C, 70°C to 170°C, 80°C to 160°C, 90°C to 150°C, 100°C to 140°C, 110°C to 130°C. The polyol-functional urethane compound from the first reaction step and/or the second reaction step may reacted with a compound comprising an acetoacetate functionality at a temperature from 90°C and 150°C, such as from 95°C to 145°C, 100°C to 140°C, 105°C to 135°C, or 110°C to 130°C, to introduce acetoacetate functionality into the polyol-functional urethane compound backbone.

The compounds comprising polyacetoacetate functionality and at least one urethane linkage as described above can be incorporated into a coating composition. The coating composition may include: (i) a compound comprising polyacetoacetate functionality and at least one urethane linkage as described above; (ii) a blocked polyamine; and (iii) a polyepoxide reactive with the condensation product of (i) and (ii).

The coating composition may comprise from 2 to 80 weight percent (wt.%) of the compound comprising polyacetoacetate functionality and at least one urethane linkage as described above, such as from 5 to 75 wt.%, from 10 to 70 wt.%, from 15 to 60 wt. %, or from 25 to 50 wt.% , based on total resin solids of the coating composition.

Suitable blocked polyamines include aliphatic and aromatic compounds comprising two or more amine groups selected from primary and secondary amine groups, with a blocking group on the primary and/or secondary amines. By "blocked" is meant that the amine group has been reacted with a compound, such as a ketone or aldehyde, so that the resultant blocked amine group is non-reactive. The (ii) polyamine may include blocked ethylenediamine, diethylenetriamine, hexamethylenediamine, 1,2-propanediamine, 2-methyl-1,5-pentamethylenediamine, 2,2,4-trimethyl-1,6-hexanediamine, isophoronediamine, diaminocyclohexane, xylylenediamine, 1,12-diamino-4,9-dioxadodecane, or some combination thereof. For example, the (ii) blocked polyamine may be a blocked polyketimine or a blocked polyaldimine. Additional suitable polyamines include those in U.S. Patent Application Publication No. 2018/0162099 A1 at paragraphs [0066]-[0068].

The (ii) blocked polyamine may contain an average amino functionality of greater than 4. The (ii) blocked polyamine may contain an average amino functionality of less than 4. As used herein "average amino functionality" refers to the average number of amino groups on a molecule. The coating composition may contain one blocked polyamine or may contain two different blocked polyamines. For example, the coating composition may contain a first blocked polyamine having an average amino functionality of greater than 4 and a second blocked polyamine having an average amino functionality of less than 4.

Suitable blocked polyketimines or blocked polyaldimines useful for forming coating compositions of this invention include those obtained by the reaction of a primary or secondary amine with either a ketone or an aldehyde, respectively, and include diketimines and dialdimines such as those described in U.S. Pat. No. 3,668,183 at column 2 line 49 to column 3 line 20. Additional blocked polyketimines that are suitable are described in U.S. Pat. No. 5,288,802 at column 3, line 22 to column 4, line 25. Suitable polyketimines include a dimethylisobutyl ketone ketimine of diethylenetriamine, a dimethylisobutyl ketone ketimine of isophorone diamine, a ketiminopropyltriethoxysilane, a polyketimine derived from Novolac epoxy, or some combination thereof. A suitable polyaldimine is a dialdimine of isophorone diamine.

The coating composition may comprise from 2 to 85 wt.% of the blocked polyamine, such as from 10 to 80 wt.%, from 15 to 75 wt.%, from 20 to 70 wt.% or from 30 to 60 wt.%, based on total resin solids of the coating composition.

The unblocking of the polyamine can be facilitated by atmospheric moisture. To the extent that the polyamine is provided in the form of a ketimine or an aldimine, the reactions are autocatalytic in the sense that the reaction between a polyamine, once unblocked, and an acetoacetate functional group of the compound having polyacetoacetate functionality and at least one urethane linkage, produces water. The production of water is used to cause the further unblocking of polyketimine or polyaldimine, useful for further reactions between the acetoacetate functional group of the compound having polyacetoacetate functionality and at least one urethane linkage and the polyamine to produce the condensation product.

The condensation product of (i) and (ii) comprises an enamine which can react with the (iii) polyepoxide.

The (iii) polyepoxide may be a saturated or unsaturated aromatic and/or an aliphatic polyepoxide polymer. The (iii) polyepoxide may be a liquid or a solid at room temperature (23°C). Non-limiting examples of aromatic polyepoxide compounds include EPON 828 (bisphenol A-epichlorohydrin epoxy resin) and/or blends of this resin with difunctional epoxide reactive diluents such as neopentylglycol diglycidylether, resorcinol diglycidylether and cyclohexanedimethanoldiglycidylether; bisphenol F epoxy resins, such as EPON DPL 862 (bisphenol F-epiclorohydrin epoxy resin) available from Hexion Specialty Chemicals (Columbus, OH); and epoxy phenol novolac resins such as EPALLOY 8250 (epoxy novalac resin) from Hunstman Corporation (Woodlands, TX), ARALDITE EPN 1139 from Huntsman Corporation, and DEN438 from Dow Chemical (Midland, MI). Non-limiting examples of non-aromatic epoxy resins include hydrogenated cyclohexane dimethanol and diglycidyl ethers of hydrogenated Bisphenol A-type epoxide resin, such as EPONEX 1510 (hydrogenated bisphenol A-epichlorohydrin epoxy resin), HELOXY 107 and from Hexion Specialty Chemicals; SANTOLINK LSE 120 from Monsanto (Springfield, MA); EPODIL 757 (cyclohexane dimethanol diglycidylether) from Evonik Industries (Essen, Germany); EPIREZ 505 from Hexion Specialty Chemicals; and ERL4221 from Polysciences (Warrington, PA). Other suitable non-aromatic epoxy resins include DER 732 and DER 736 from Palmer Holland (North Olmsted, OH).

The coating composition may comprise from 5 to 60 wt.% of the polyepoxide, such as from 10 to 50 wt.%, from 15 to 40 wt.%, or from 10 to 30 wt.% , based on total resin solids of the coating composition.

The coating composition may further comprise a multi-functional (meth)acrylate. As used herein, the term "(meth)acrylate" refers to both the methacrylate and the acrylate. As used herein, the term "multi-functional (meth)acrylate" refers to a methacrylate or an acrylate having greater than or equal to two acrylate or methacrylate groups. Examples of suitable mutli-functional (meth)acrylate materials include 1,6-hexanediol diacrylate, trimethylolpropane triacrylate, pentaerythritol tetraacrylate, ethoxylated bisphenol A diacrylate, and tris(2-hydroxyethyl )isocyanurate triacrylate. The mutli-functional (meth)acrylate materials used in the coating composition generally have a Mw from 100 to 50,000 as determined by GPC using a polystyrene standard. The multi-functional (meth)acrylate materials may be low molecular weight materials, which have a formula weight from 100 to 5000, such as from 100 to 500. The multi-functional (meth)acrylate may be added to the coating composition in an amount such as up to 10 wt.%, up to 7.5 wt.%, or up to 5 wt. %, based on total resin solids of the coating composition. The multi-functional (meth)acrylate may be added to the coating composition in an amount such as at least 3 wt.%, at least 3.5 wt.%, or at least 4 wt.%, based on total resin solids of the coating composition. The multi-functional (meth)acrylate may be added to the coating composition in an amount of from 3 wt.% to 10 wt.%, from 3 wt.% to 7.5 wt%, from 3 wt.% to 5 wt.%, from 3.5 wt.% to 10 wt.%, from 3.5 wt.% to 7.5 wt.%, from 3.5 wt.% to 5 wt.%, from 4 wt.% to 10 wt.%, from 4 wt.% to 7.5 wt.%, or from 4 wt.% to 5 wt.%, based on total resin solids of the coating composition.

The coating composition may comprise a multi-functional (meth)acrylate, such as those described previously, in place of the (iii) polyepoxide or in addition to the (iii) polyepoxide.

The coating composition may further comprise a metal compound comprising an alkaline earth metal hydroxide and/or alkaline earth metal oxide to provide the coating with enhanced corrosion resistance. Non-limiting examples of the metal compound may include a magnesium hydroxide, a magnesium oxide, a calcium hydroxide, a calcium oxide, or some combination thereof. The metal compound may be added to the coating composition in amount such as up to 20 wt.%, such as up to 30 wt.%, such as up to 40 wt.%, or such as up 50 wt.%, based on the total resin solids of the coating composition.

The coating composition of the present invention may also include pigments resistant to corrosion. Corrosion resistant pigments include, but, are not limited to, zinc phosphate, calcium ion exchange silica, colloidal silica, amorphous silica synthetic, barium molybdate, strontium molybdate, and mixtures thereof. An example calcium ion exchange silica is commercially available from W.R. Grace & Co. under the SHIELDEX AC3 and SHIELDEX AC5 trademark. An example colloidal silica is commercially available from Nissan Chemical Industries, Ltd. under the trademark SNOWTEX. An example amorphous silica is available from W.R. Grace & Co. under the brand SYLOID.

The coating composition may further comprise an alkoxysilane. The alkoxysilane may have the structure (X)ₙ-Si-(Y), where X can be an alkoxy ester, n may be from 1 to 3, and Y may be an alkyl group having an amino group, a ketimine group, a mercapto group, an epoxide group, or some combination thereof. The alkoxysilane may be reactive with the substrate to which the coating composition is applied and the resin of the coating composition so as to enhance adhesion of the cured coating to the substrate. The alkoxysilane may be added to the coating composition in amount such as up to 1 wt.%, such as up to 5 wt.%, or such as up to 10 wt.%, based on the total resin solids of the coating composition.

The coating composition may further comprise pigments and/or fillers. Suitable pigments may be selected from organic and inorganic color pigments which may include titanium dioxide, carbon black, lampblack, zinc oxide, and extender pigments including ground and crystalline silica, barium sulfate, magnesium silicate, calcium silicate, mica, micaceous iron oxide, calcium carbonate, zinc powder, aluminum and aluminum silicate, gypsum, feldspar and the like.

The coating composition may further comprise a metal phosphate complex. Non-limiting examples of suitable metals that form phosphate complexes include zinc, aluminum, iron, strontium, potassium or some combination thereof. The metal phosphate complex may enhance the corrosion resistance of the cured coating. The metal phosphate complex may be included in coating composition in addition to or in lieu of the previously described metal compound included to enhance corrosion resistance. The metal phosphate complex may be added to the coating composition in amount such as up to 5 wt.%, such as up to 10 wt.%, such as up to 15 wt.%, such as up to 20 wt.%, or such as up to 25 wt.%, based on the total resin solids of the coating composition.

The coating composition may further comprise an acid catalyst, such as a carboxylic acid catalyst or a phosphoric acid catalyst. The coating composition may comprise both a carboxylic acid catalyst and a phosphoric acid catalyst. Suitable carboxylic acid catalysts include those having from four to twenty carbon atoms. Non-limiting examples of suitable carboxylic acid catalysts include isostearic acid, dodecanoic acid, stearic acid, lauric acid, octadecanoic acid, undecylenic acid, vegetable oil, tall oil, coconut oil, benzoic acid, or some combination thereof. Non-limiting examples of suitable phosphoric acids include phosphoric acid, 2-ethyl hexyl acid phosphate, phenyl phosphonic acid, or some combination thereof. The acid catalyst may be added to the coating composition in amount such as up to 1 wt.%, such as up to 2 wt.%, or such as up to 5 wt.%, based on the total resin solids of the coating composition. The acid catalyst may be added to the coating composition to accelerate the deblocking of the polyamine.

Other suitable materials that can be used with the coating composition include, but are not limited to, plasticizers, abrasion resistant particles, anti-oxidants, hindered amine light stabilizers, UV light absorbers and stabilizers, surfactants, flow and surface control agents, thixotropic agents, reaction inhibitors, acrylate functional compounds, and other customary auxiliaries.

A solvent or solvent blend is generally utilized to reduce the coating composition viscosity as desired, such as to facilitate coating application. Non-limiting examples of suitable solvents include acetone, methyl ethyl ketone, methyl isobutyl ketone, methyl amyl ketone, butyl acetate, or some combination thereof.

The coating composition may be applied to a substrate and cured to form a coating thereover. The coating may be a continuous film formed over at least a portion the substrate. The coating composition may be applied as a liquid composition to the substrate.

The substrate over which the coating composition may be applied includes a wide range of substrates. For example, the coating composition of the present invention can be applied to a vehicle substrate, an industrial substrate, an aerospace substrate, and the like.

The vehicle substrate may include a component of a vehicle. In the present disclosure, the term "vehicle" is used in its broadest sense and includes all types of aircraft, spacecraft, watercraft, and ground vehicles. For example, the vehicle can include, but is not limited to an aerospace substrate (a component of an aerospace vehicle), such as an aircraft such as, for example, airplanes (e.g., private airplanes, and small, medium, or large commercial passenger, freight, and military airplanes), helicopters (e.g., private, commercial, and military helicopters), aerospace vehicles (e.g., rockets and other spacecraft), and the like). The vehicle can also include a ground vehicle such as, for example, animal trailers (e.g., horse trailers), all-terrain vehicles (ATVs), cars, trucks, buses, vans, heavy duty equipment, tractors, golf carts, motorcycles, bicycles, snowmobiles, trains, railroad cars, and the like. The vehicle can also include watercraft such as, for example, ships, boats, hovercrafts, and the like. The vehicle substrate may include a component of the body of the vehicle, such as an automotive hood, door, trunk, roof, and the like; such as an aircraft or spacecraft wing, fuselage, and the like; such as a watercraft hull, and the like.

The coating composition may be applied over an industrial substrate which may include tools, heavy duty equipment, furniture such as, office furniture (e.g., office chairs, desks, filing cabinets, and the like), appliances such as refrigerators, ovens and ranges, dishwashers, microwaves, washing machines, dryers, small appliances (e.g., coffee makers, slow cookers, pressure cookers, blenders, etc.), metallic hardware, extruded metal such as extruded aluminum used in window framing, other indoor and outdoor metallic building materials, and the like.

The coating composition may be applied over storage tanks, windmills, nuclear plant components, packaging substrates, wood flooring and furniture, apparel, electronics, including housings and circuit boards, glass and transparencies, sports equipment, stadiums, buildings, bridges, and the like.

For example, the coating composition of the present invention can be applied to metallic substrate or a non-metallic substrate. The substrate can be metallic or non-metallic. Metallic substrates include, but are not limited to, tin, steel (including electrogalvanized steel, cold rolled steel, hot-dipped galvanized steel, iron phosphate steel, among others), iron, aluminum, aluminum alloys, zinc-aluminum alloys, steel coated with a zinc-aluminum alloy, and aluminum plated steel. Non-metallic substrates include polymeric materials, plastic and/or composite material, polyester, polyolefin, polyamide, cellulosic, polystyrene, polyacrylic, poly(ethylene naphthalate), polypropylene, polyethylene, nylon, ethylene vinyl alcohol (EVOH), polylactic acid, other "green" polymeric substrates, poly(ethyleneterephthalate) (PET), polycarbonate, polycarbonate acrylobutadiene styrene (PC/ABS), wood, veneer, wood composite, particle board, medium density fiberboard, cement, stone, glass, paper, cardboard, textiles, leather, both synthetic and natural, and the like. The substrate may comprise a metal, a plastic and/or composite material, and/or a fibrous material. The fibrous material may comprise a nylon and/or a thermoplastic polyolefin material with continuous strands or chopped carbon fiber. Composite substrates may comprise an epoxy-based matrix with fibers or particles included therein. Non-limiting examples of the fibers or particles include glass and/or carbon. The substrate can be one that has already been treated in some manner.

The coating composition of the present invention may be particularly beneficial when applied to a metallic substrate. The coatings of the present invention may be particularly beneficial when applied to metallic substrates that are used to fabricate automotive vehicles, such as cars, trucks, heavy duty agricultural equipment, and heavy duty construction equipment; architectural structures, such as bridges; and aluminum in aerospace applications.

The coating composition may be particularly suitable as a protective marine coating (PMC) for coating marine equipment, such as, a PMC for ship hulls.

The coating composition may be applied to a substrate having multiple components, wherein the coating composition is simultaneously applied to the multiple components and simultaneously cured to form a coating over the multiple components without deforming, distorting, or otherwise degrading any of the components. The components may be parts of a larger whole of the substrate. The components may be separately formed and subsequently arranged together to form the substrate. The components may be integrally formed to form the substrate.

Non-limiting examples of components of a substrate in the vehicle context include a vehicle body (e.g., made of metal) and a vehicle bumper (e.g., made or plastic) which are separately formed and subsequently arranged to form the substrate of the vehicle. Further examples include a plastic automotive component, such as a bumper or fascia in which the bumper or fascia comprises regions or subcomponents which comprise more than one type of substrate. Further examples include aerospace or industrial components comprising more than one substrate type. It will be appreciated that other such other multi-component substrates are contemplated within the context of this disclosure.

The multiple components may include at least a first component and a second component, and the first component and the second component may be formed from different materials. As used herein, "different materials" refers to the materials used to form the first and second component having different chemical make-ups.

The different materials may be from the same or different class of materials. As used herein, a "class of materials" refers to materials that may have a different specific chemical make-up but share the same or similar physical or chemical properties. For example, metals, polymers, ceramics, and composites may be defined as different classes of materials. However, other classes of materials may be defined depending on similarities in physical or chemical properties, such as nanomaterials, biomaterials, semiconductors, and the like. Classes of materials may include crystalline, semi-crystalline, and amorphous materials. Classes of materials, such as for polymers, may include thermosets, thermoplastics, elastomers, and the like. Classes of materials, such as for metals, may include alloys and non-alloys. As will be appreciated from the above exemplary list of classes, other relevant classes of materials may be defined based on a given physical or chemical property of materials.

The first component may be formed from a metal, and the second component may be formed from a plastic or a composite. The first component may be formed from a plastic, and the second component may be formed from a metal or a composite. The first component may be formed from a composite, and the second component may be formed from a plastic or a metal. The first component may be formed from a first metal, and the second component may be formed from a second metal different from the first metal. The first component may be formed from a first plastic, and the second component may be formed from a second plastic different from the first plastic. The first component may be formed from a first composite, and the second component may be formed from a second composite different from the first composite. As will be appreciated from these non-limiting examples, any combination of different materials from the same or different classes may form the first and second components of the substrate.

Examples of combinations of materials include thermoplastic polyolefins (TPO) and metal, TPO and acrylonitrile butadiene styrene (ABS), TPO and acrylonitrile butadiene styrene/polycarbonate blend (ABS/PC), polypropylene and TPO, TPO and a fiber reinforced composite, and other combinations. Further examples include aerospace substrates or industrial substrates comprising various components made of a plurality of materials, such as various metal-plastic, metal-composite, and/or plastic-composite containing components. The metals may include ferrous metals and/or non-ferrous metals. Non-limiting examples of non-ferrous metals include aluminum, copper, magnesium, zinc, and the like, and alloys including at least one of these metals. Non-limiting examples of ferrous metals include iron, steel, and alloys thereof.

When the coating composition is applied to the substrate having multiple components simultaneously, the applied coating composition may be cured at a temperature which does not deform, distort, or otherwise degrade either of the first and second component (the materials thereof). Thus, the curing temperature may be below the temperature at which either of the first component or the second component of the substrate would deform, distort, or otherwise degrade.

The coating composition may be applied to the substrate by any suitable means, such as spraying, electrostatic spraying, dipping, rolling, brushing, and the like.

The coating composition can be applied to a substrate to form a primer coating layer. A "primer coating layer" refers to an undercoating that may be deposited onto a substrate (e.g., directly on or over a pre-treatment layer) in order to prepare the surface for the application of a protective or decorative coating system.

The coating composition can be applied to a substrate as a coating layer of a multi-layer coating system, such that one or more additional coating layers are formed above the coating formed from the coating composition. In the multi-layer coating system, a first coating layer may be applied over at least a portion of the substrate, wherein the first coating layer is formed from a first coating composition. A second coating layer may be applied over at least a portion of the first coating layer, wherein the second coating layer is from a second coating composition that is different from the first coating composition. The second coating composition may comprise a pigment. The second coating composition may be applied after the first coating composition has been cured to form the first coating layer or may be applied in a wet-on-wet process prior to curing the first coating composition, after which the first and second coating compositions are simultaneously cured to form the first and second coating layers. The first coating composition may be the coating composition of the present invention.

The first and second coating compositions may be cured individually or simultaneously to form first and second coating layers. The first and/or second coating compositions may be cured under ambient conditions or may be heated to form the first and second coating layers. For example, the first and second coating composition may be cured at temperatures ranging from 20°C to 120°C, 20°C to 100°C, 20°C to 90°C, 20°C to 80°C, 40°C to 120°C, 40°C to 100°C, 40°C to 90°C, 40°C to 80°C, 60°C to 120°C, 60°C to 100°C, 60°C to 90°C, 60°C to 80°C, 80°C to 120°C, or 80°C to 100°C where neither the first component nor the second component would deform, distort, or otherwise degrade within that range. The coating composition may be cured at temperatures less than or equal to 120°C, less than or equal to 110°C, less than or equal to 100°C, less than or equal to 90°C, less than or equal to 80°C, less than or equal to 70°C, less than or equal to 60°C, less than or equal to 50°C, less than or equal to 40°C, less than or equal to 30°C, or ambient temperature (20°C-25°C) where neither the first component nor the second component would deform, distort, or otherwise degrade within these ranges.

The multi-layer coating system may include a topcoat layer formed from a topcoat composition applied over the substrate. The topcoat composition may be applied over at least a portion of second coating layer. The topcoat may be a clearcoat. As used herein, a "clearcoat" refers to a coating layer that is at least substantially transparent or fully transparent. The term "substantially transparent" refers to a coating, wherein a surface beyond the coating is at least partially visible to the naked eye when viewed through the coating. The term "fully transparent" refers to a coating, wherein a surface beyond the coating is completely visible to the naked eye when viewed through the coating. It is appreciated that the clearcoat can comprise colorants, such as pigments, provided that the colorants do not interfere with the desired transparency of the clearcoat. The clearcoat can be substantially free, essentially free, or completely free of pigments. As used herein, the term "substantially free of pigment" means that the coating composition comprises less than 5% by weight of pigment based on total solids of the coating composition. As used herein, the term "essentially free of pigment" means that the coating composition comprises less than 1% by weight of pigment based on total solids of the coating composition. As used herein, the term "completely free of pigment" means that the coating composition comprises 0% by weight of pigment based on total solids of the coating composition.

The topcoat composition may be applied onto the second coating composition prior to or after curing the first and second coating compositions. The first coating composition, the second coating composition, and the topcoat composition may be simultaneously cured under ambient conditions or may be heated under the conditions described previously.

The coating composition of the present invention can be supplied as a two-component or two-package system. The first component or the first package can include (ii) the blocked polyamine. The second component or the second package (separate from the first package) can include a mixture of (i) the compound comprising the acetoacetate functionality and at least one urethane linkage and (iii) the polyepoxide.

The two-component system described herein may be prepared by preparing the first component as described herein and filling a first container therewith. The two-component system may be prepared by preparing the second component as described herein and filling a second container different from the first container with the second component. The first container and the second container may be arranged such that the first component and the second component do not contact one another until a user is preparing to apply the coating composition to a substrate. To prepare the coating composition, the user may contact the first component from the first container with the second component from the second container to form a coating composition, such as by mixing the first component with the second component. The coating composition may be applied over a substrate and cured to form a cured coating layer thereover. The coating composition (the mixture of the first and second components) may be applied over the substrate within 48 hours of first contacting the first component with the second component, such as within 24 hours, within 12 hours, or within 8 hours.

### EXAMPLES

The following examples are presented to demonstrate the general principles of the invention. The invention should not be considered as limited to the specific examples presented.

### Example 1

### Part A: Preparation of a compound having polyacetoacetate and urethane functionality

A compound was prepared by charging the following components in order into a 1-liter, 4-necked flask under a nitrogen sparge equipped with a motor driven stainless steel stir blade, a water-cooled condenser, and a heating mantle with a thermometer connected through a temperature feedback control device: 72.69 parts by weight of ethylene carbonate (commercially available from Sigma Aldrich (St. Louis, Missouri)) and 100 parts by weight of tris (2-amino-2-(hydroxymethyl)-1,3-propanediol) (commercially available from Sigma Aldrich). The mixture was heated to 100°C and held until infrared (IR) spectroscopy showed the absence of the characteristic carbonate band at 1977 cm⁻¹. Next, 626.84 parts by weight of *tert-butyl* acetoacetate (commercially available from Eastman Chemical Company (Kingsport, Tennessee)) was charged into the reactor and heated to 110°C. The mixture was maintained at reflux for 30 minutes. The *tert-butyl* alcohol by-product was distilled from the reaction mixture, followed by a vacuum distillation to completely remove any non-reacted *tert-butyl* acetoacetate. The resulting compound was cooled to 45°C and poured from the reaction vessel.

### Part B: Characterization of the compound having polyacetoacetate and urethane functionality

The final compound had a theoretical acetoacetate equivalent weight of 136 grams per equivalent (g/eq).

The weight average molecular weight (Mw) of the compound was determined by Gel Permeation Chromatography using a Waters 2695 separation module with a Waters 410 differential refractometer (RI) detector (Waters Corp. (Milford, Massachusetts)). Polystyrene was used as a standard. Tetrahydrofuran (THF) was used as the eluent at a flow rate of 1 mL per minute (mL/min) and two PLgel Mixed C columns from Agilent Technologies (Santa Clara, California) were used for separation. The final compound had a measured Mw of 392 grams per mole (g/mol) and a PDI of 1.1.

The final compound had the following theoretical structure:

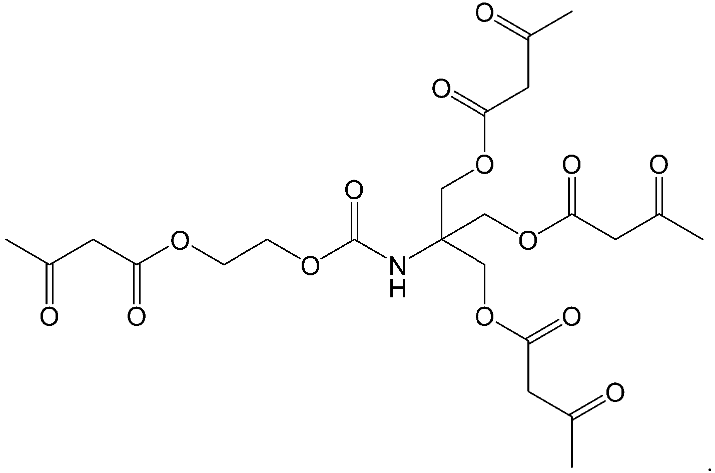

### Example 2

### Preparation of a compound having polyacetoacetate and urethane functionality

A compound was prepared by charging the following components in order into a 1-liter, 4-necked flask under a nitrogen sparge equipped with a motor driven stainless steel stir blade, a water-cooled condenser, and a heating mantle with a thermometer connected through a temperature feedback control device: 58.15 parts by weight of ethylene carbonate (commercially available from Sigma Aldrich) and 100 parts by weight tris (2-amino-2-(hydroxymethyl)-1,3-propanediol) (commercially available from Sigma Aldrich (St. Louis, MO)). The mixture was heated to 100°C and held until IR spectroscopy showed the absence of the characteristic carbonate band of ethylene carbonate at 1799 cm⁻¹. Next, 192.21 parts by weight of ε-caprolactone (commercially available from BASF (Ludwigshafen, Germany)) and 1.32 parts by weight stannous octoate (Tin II) (commercially available from PMC Organometallix, Inc. (Carrolton, Kentucky)) was charged into the reactor. The mixture was heated to 130°C and held until IR spectroscopy showed the absence of the characteristic ε-caprolactone bands at 850 and 860 cm⁻¹. Next, 195.8 parts by weight of *tert*-butyl acetoacetate (commercially available from Eastman Chemical Company (Kingsport, TN)) was charged into the reactor and heated to 110°C. The mixture was maintained at reflux for 30 minutes. The *tert-butyl* alcohol by-product was distilled from the reaction mixture, followed by a vacuum distillation to completely remove any non-reacted *tert-butyl* acetoacetate. The resulting compound was cooled to 45°C and poured from the reaction vessel.

The equivalent weight, Mn, and PDI of the compound were determined using the methods described above. The final compound had the following theoretical structure with a theoretical acetoacetate equivalent weight of 193 g/eq:

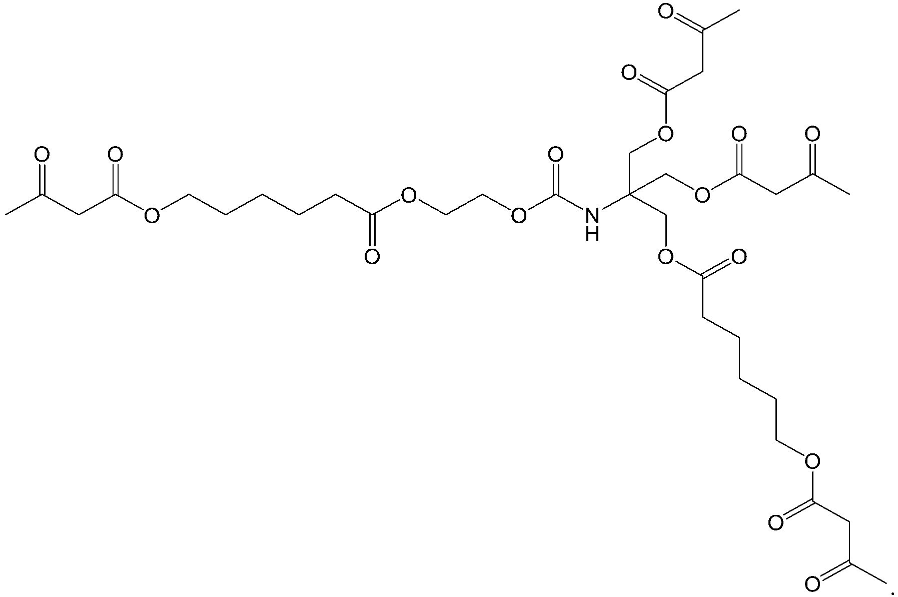

The final compound had a measured Mw of 751 g/mol and a PDI of 1.4.

### Example 3

### Preparation of a compound having polyacetoacetate and urethane functionality

A compound was prepared by charging the following components in order into a 0.5-liter, 4-necked flask under a nitrogen sparge equipped with a motor driven stainless steel stir blade, a water-cooled condenser, and a heating mantle with a thermometer connected through a temperature feedback control device: 89.03 parts by weight of diethanolamine (commercially available from BASF) and 100 parts by weight of Glycerol Carbonate (commercially available from Innospec (Littleton, CO)). The mixture was heated to 75°C and held until IR spectroscopy showed the absence of the characteristic carbonate band of ethylene carbonate at 1799 cm⁻¹. Next, 643.03 parts by weight of *tert*-butyl acetoacetate (commercially available from Eastman Chemical Company) was charged into the reactor and heated to 110°C. The mixture was maintained at reflux for 30 minutes. The *tert-butyl* alcohol by-product was distilled from the reaction mixture, followed by a vacuum distillation to completely remove any non-reacted *tert-butyl* acetoacetate. The resulting compound was cooled to 45°C and poured from the reaction vessel.

The equivalent weight, Mn, and PDI of the compound were determined using the methods described above. The final compound had the following theoretical structure with a theoretical acetoacetate equivalent weight of 140 g/eq:

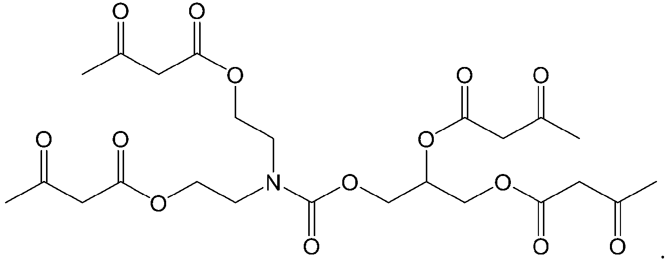

The final compound had a measured Mw of 422 g/mol and a PDI of 1.4.

### Example 4

### Preparation of a compound having polyacetoacetate and urethane functionality

A compound was prepared by charging the following components in order into a 0.5-liter, 4-necked flask under a nitrogen sparge equipped with a motor driven stainless steel stir blade, a water-cooled condenser, and a heating mantle with a thermometer connected through a temperature feedback control device: 49.20 parts by weight of 2-methylpentane-1,5-diamine (DYTEK^{®} A Amine, commercially available from INVISTA (Wichita, KS)) and 100 parts by weight of Glycerol Carbonate (commercially available from Innospec). The mixture was heated to 75°C and held until IR spectroscopy showed the absence of the characteristic carbonate band of ethylene carbonate at 1799 cm⁻¹. Next, 321.52 parts by weight of *tert-butyl* acetoacetate (commercially available from Eastman Chemical Company) was charged into the reactor and heated to 110°C. The mixture was maintained at reflux for 30 minutes. The *tert-*butyl alcohol by-product was distilled from the reaction mixture, followed by a vacuum distillation to completely remove any non-reacted *tert*-butyl acetoacetate. The resulting compound was cooled to 45°C and poured from the reaction vessel.

The equivalent weight, Mn, and PDI of the compound were determined using the methods described above. The final compound had the following theoretical structure with a theoretical acetoacetate equivalent weight of 172 g/eq:

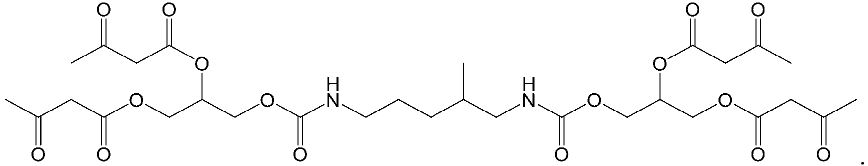

The final compound had a measured Mw of 1226 g/mol and a PDI of 1.5.

### Example 5

### Preparation of a compound having polyacetoacetate and urethane functionality

A compound was prepared by charging the following components in order into a 0.5-liter, 4-necked flask under a nitrogen sparge equipped with a motor driven stainless steel stir blade, a water-cooled condenser, and a heating mantle with a thermometer connected through a temperature feedback control device: 162.00 parts by weight of JEFFAMINE T403 (commercially available from Huntsman Corporation (The Woodlands, TX)) and 39.36 parts by weight of Glycerol Carbonate (commercially available from Innospec) and 60.20 parts by weight of Ethylene Carbonate (commercially available from Sigma Aldrich). The mixture was heated to 150°C and held until infrared (IR) spectroscopy showed the stall of the characteristic carbonate band of ethylene carbonate at 1799 cm⁻¹. Next, 196.17 parts by weight of *tert-butyl* acetoacetate (commercially available from Eastman Chemical Company) was charged into the reactor and heated to 110°C. The mixture was maintained at reflux for 30 minutes. The *tert-*butyl alcohol by-product was distilled from the reaction mixture, followed by a vacuum distillation to completely remove any non-reacted *tert-butyl* acetoacetate. The resulting compound was cooled to 45°C and poured from the reaction vessel.

The equivalent weight, Mn, and PDI of the compound were determined using the methods described above. The final compound had the following theoretical structure with a theoretical acetoacetate equivalent weight of 270 g/eq: 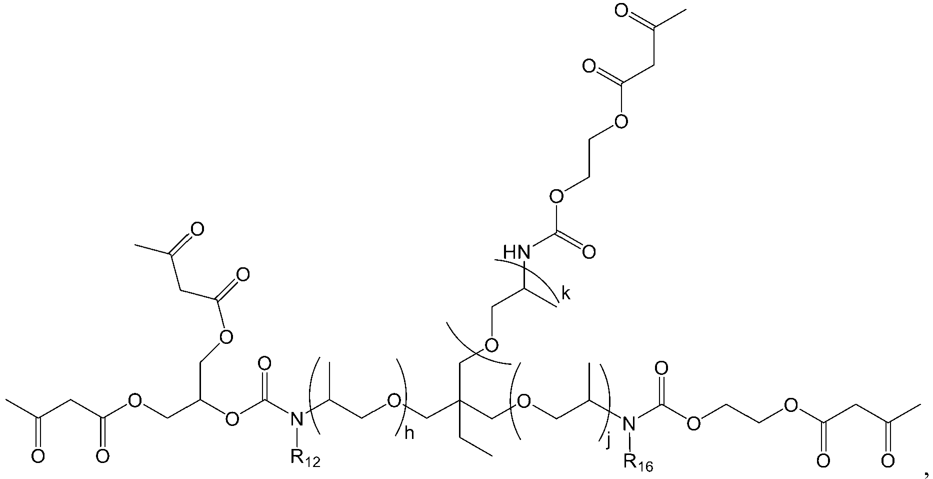 where R₁₂ is hydrogen, R₁₆ is hydrogen, and the sum of h, j, and k is 5 to 6.

The final compound had a measured Mw of 4999 g/mol and a PDI of 2.3.

### Example 6

### Preparation of a compound having polyacetoacetate and urethane functionality

A compound was prepared by charging the following components in order into a 0.5-liter, 4-necked flask under a nitrogen sparge equipped with a motor driven stainless steel stir blade, a water-cooled condenser, and a heating mantle with a thermometer connected through a temperature feedback control device: 162.00 parts by weight of Jeffamine T403 (commercially available from Huntsman Corporation) and 90.30 parts by weight of Ethylene Carbonate (commercially available from commercially available from Sigma Aldrich). The mixture was heated to 150°C and held until IR spectroscopy showed the stall of the characteristic carbonate band of ethylene carbonate at 1799 cm⁻¹. Next, 199.33 parts by weight of *tert-butyl* acetoacetate (commercially available from Eastman Chemical Company) was charged into the reactor and heated to 110°C. The mixture was maintained at reflux for 30 minutes. The *tert-butyl* alcohol by-product was distilled from the reaction mixture, followed by a vacuum distillation to completely remove any non-reacted *tert-butyl* acetoacetate. The resulting compound was cooled to 45°C and poured from the reaction vessel.

The equivalent weight, Mn, and PDI of the compound were determined using the methods described above. The final compound had the following theoretical structure with a theoretical acetoacetate equivalent weight of 322 g/eq: 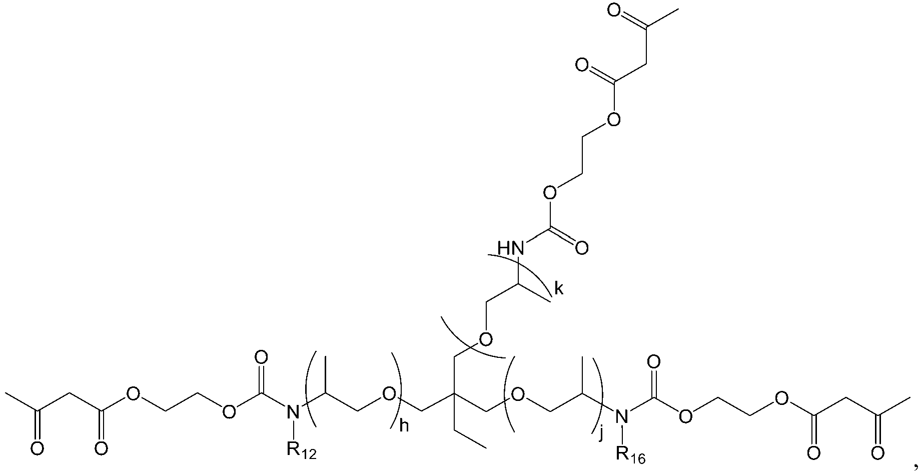 where R₁₂ is hydrogen, R₁₆ is hydrogen, and the sum of h, j, and k is 5 to 6.

The final compound had a measured Mw of 4083 g/mol and a PDI of 2.2.

### Example 7

### Preparation of a Primer Composition using the Compounds of Examples 1 and 2

A primer coating composition was prepared by mixing an A side and a B side. For the A side, the base components of Table 1 were weighed and placed into a metal jar. The premix was charged to an Eiger mill at 3000 revolutions per minute and milled for 25 min with a continuous recycle of the formulation such that the mill output was direct to the mill input to create a continuous loop. The temperature during mixing was maintained between 110°F to 120°F (43°C and 49°C). The final dispersions had a Hegman gauge reading of greater than 7. For the B side, the base components of Table 1 were weighed and placed into a metal can and mixed until homogenous. All material amounts are in terms of weight percent unless otherwise specified.

**TABLE 1**

| **Components** | **Comparative Example 1 (weight percent, wt. %)** | **Example A (wt. %)** | **Example B (wt. %)** |
|---|---|---|---|
| **A Side** | | | |
| ¹5.5 functional epoxy ketimine | 11.03% | 11.76% | 11.14% |
| ²IPDA ketimine | 3.86% | 4.11% | 3.89% |
| ³DISPERBYK-110 | 1.58% | 1.67% | 1.58% |
| ⁴BARTEX OWT | 21.13% | 22.71% | 21.53% |
| ⁵TIONA 595 | 8.35% | 8.90% | 8.44% |
| ⁶TALCRON MP 15-38 | 7.66% | 8.90% | 8.44% |
| ⁷Magnesium oxide | 7.63% | - | - |
| ⁸SHIELDEX AC 5 | - | 4.42% | 4.19% |
| ⁹HEUCOPHOS ZP10 | 7.60% | 8.15% | 7.73% |
| ¹⁰Lamp Black #6 | 0.12% | 0.12% | 0.12% |
| ¹¹LO-VEL 27 | 0.20% | 0.21% | 0.20% |
| ¹²BENTONE SD-2 | 0.20% | - | - |
| ¹³M-P-A 4020BA | 0.61% | - | - |
| ¹⁴SUSPENO 201-NBA | - | 1.05% | 1.00% |
| ¹⁵ Acetone | 4.00% | 3.90% | 3.67% |
| ¹⁶Methyl n-amyl ketone | 4.73% | 4.98% | 4.72% |
| ¹⁷n-Butyl acetate | 2.27% | 2.42% | 2.29% |

| **B Side** | | | |
|---|---|---|---|
| ¹⁸Acetoacetate polyester | 9.90% | - | - |
| ¹⁹Example 1 | - | 8.09% | - |
| ²⁰Example 2 | - | - | 10.29% |
| ²¹EPON 828 | 4.90% | 4.61% | 5.76% |
| ²²Isostearic acid | 0.04% | 0.04% | 0.04% |
| ¹⁵Acetone | 3.13% | 2.95% | 3.69% |
| ²³SILQUEST A-187 (GLYMO) | 1.08% | 1.01% | 1.27% |

| | | | |
|---|---|---|---|
| ¹ ~ (Approximately) 5.5 functional polyketimine prepared according to Example C of United States Patent No. 5,288,802 (as used herein, "5.5 functional polyketimine" refers to a polyketimine compound having an average of 5.5 ketimine groups) ² Diketimine prepared from the reaction of isophoronediamine and methylisobutyl ketone at 98 wt. % in methyl isobutyl ketone (MIBK) ³ Pigment dispersant available from BYK Chemie (Wesel, Germany) ⁴ Barium sulfate commercially available from TOR Minerals (Corpus Christ, Texas) ⁵ Titanium dioxide commercially available from Chemours (Wilmington, Delaware) ⁶ Talc commercially available from Barretts Minerals Inc. (Dillon, Montana) ⁷ Commercially available from Martin Marietta Inc. (Raleigh, North Carolina) ⁸ Calcium oxide modified silica commercially available from W.R. Grace and Company (Columbia, Maryland) ⁹ Zinc phosphate commercially available from Heubach GmbH (Langelsheim, Germany) ¹⁰ Commercially available from General Carbon Corp. (Paterson, New Jersey) ¹¹ Silica matting agent commercially available from PPG Industries, Inc. (Pittsburgh, Pennsylvania) ¹² Bentonite clay commercially available from Elementis (London, United Kingdom) ¹³ Oxidized polyolefin wax dispersion commercially available from Elementis ¹⁴ Wax solution commercially available from POLY-RESYN, INC. (Dundee, Illinois) ¹⁵ Commercially available from Dow Chemical Company (Midland, Michigan) ¹⁶ Commercially available from Eastman Chemical Company ¹⁷ Aromatic solvent blend commercially available from Exxon Mobile Corporation (Irving, Texas) ¹⁸ Acetoacetylated polyester prepared according to Example A of United States Patent No. 5,288,802 ¹⁹ Compound of Example 1 ²⁰ Compound of Example 2 ²¹ Liquid epoxy resin commercially available from Hexion Specialty Chemicals (Columbus, Ohio) ²² Commercially available from Sartomer Company (Exton, Pennsylvania) ²³ Commercially available from Momentive Performance Materials (Waterford, New York) | | | |

Prior to the coating application, the corresponding amounts of the A side formulation and the B side formulation shown in Table 2 were combined and mixed thoroughly. The coating composition was sprayed onto metal substrate panels to a dry film thickness of from 1.5 to 1.8 mils (thousands of an inch) using an air-atomized spray gun. The coated panels were cured under ambient conditions (21°C to 24°C, 20% to 25% relative humidity). The coated panels were post cured for seven days under ambient conditions prior to testing.

**TABLE 2**

| | **A Side (wt.%)** | **B Side (wt.%)** |
|---|---|---|
| Comparative Example 1 | 80.9 | 19.1 |
| Example A | 83.3 | 16.7 |
| Example B | 78.9 | 21.1 |

The coating compositions had the resulting properties as found in Table 3. VOC is volatile organic carbon in grams per Liter (g/L), NVW is non-volatile weight (solids by weight %), and PVC is pigment volume concentration. The VOC, NVW, and PVC values were calculated using the attributes of the raw materials used to make the coating composition.

**TABLE 3**

| | **Comparative Example 1** | **Example A** | **Example B** |
|---|---|---|---|
| VOC, g/L | 275 | 285 | 277 |
| NVW, % | 81.8 | 81.2 | 81.3 |
| PVC, % | 35.2 | 36.3 | 33.14 |

Corrosion is measured by "scribe creep", which is defined as the total distance the corrosion has traveled across the scribe, measured in millimeters. The coated substrates were tested for corrosion resistance using a salt spray resistance test according to ASTM B117 (Table 4). Lower scribe creep values are better. Lower scribe creep values before scraping (CVBS) and creep values after scraping (CVAS) are better.

**TABLE 4**

| | **Scribe Creep, mm** | | | | | | | |
|---|---|---|---|---|---|---|---|---|
| | | | | | **20 cycles** | | | |
| | **500 hours** | **700 hours** | **100 hours** | | **CVBS** | **CVBS Max.** | **CVAS** | **CVAS Max.** |
| **Coated Substrate** | **P99X²⁴** | **P99X** | **B952²⁵** | **Blasted Steel²⁶** | **B1070²⁷** | | | |
| Comparative Example 1 | 4.54 | 5.02 | Delaminated | N/A | 4.1 | 6.21 | 6.48 | 9.21 |
| Example A | 0.56 | 1.8 | 2.08 | 0.23 | 0 | 1.2 | 1.05 | 1.4 |
| Example B | 1.26 | 1.74 | 1.07 | 0.47 | 3.13 | 4.7 | 3.43 | 5.07 |

| | | | | | | | | |
|---|---|---|---|---|---|---|---|---|
| ²⁴P99X - Bonderite 1000P99X, Iron phosphate treated steel with non-chrome sealer ²⁵B952 - Bonderite 952, Zinc phosphate treated steel with non-chrome sealer ²⁶Blasted steel - Act CRS Blast 2 - 2.5 mil profile ²⁷B1070 - Bonderite 1070, cold-rolled steel treated with iron phosphate and a deionized water rinse | | | | | | | | |

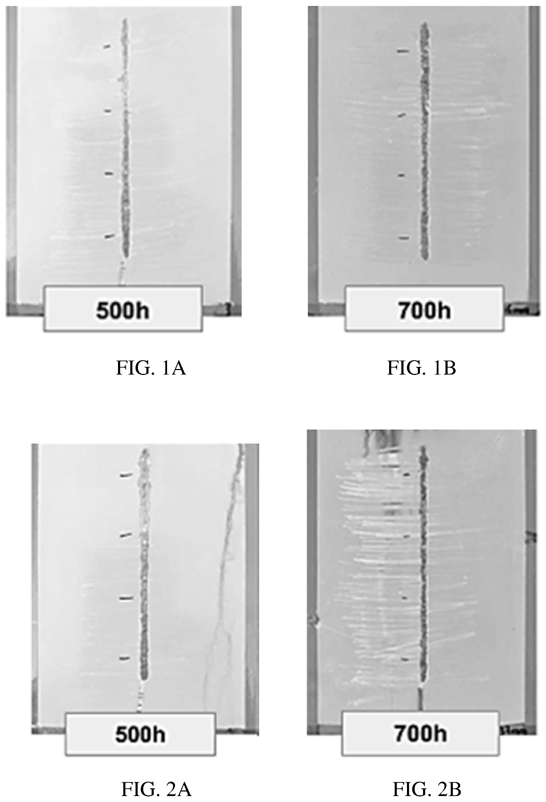

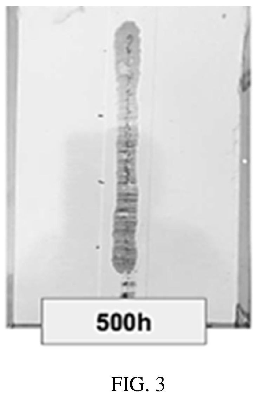

Figs. 1A and 1B show the P99X substrate coated with Example A after the 500 hour (Fig. 1A) and 700 hour (Fig. 2B) salt spray test, respectively. Figs. 2A-B show the P99X substrate coated with Example B after the 500 hour (Fig. 2A) and the 700 hour (Fig. 2B) salt spray test. Figure 3 shows the P99X substrate coated with Comparative Example 1 after the 500 hour salt spray test. As can be seen from this corrosion resistance data, the coating compositions prepared with the compound comprising polyacetoacetate functionality and at least one urethane linkage of the present invention had improved corrosion resistance properties.

The coated P99X substrates were tested for pencil hardness according to ASTM D3363, dry adhesion according to ASTM D3359, mandrel bend according to ASTM D522-93a, and direct and reverse impact according to ASTM D2794. The results are shown in Table 5.

**TABLE 5**

| | **Pencil Hardness** | **Dry Adhesion** | **Mandrel Bend** | **Direct Impact (inch-pound, in-lb)** | **Reverse Impact (in-lb)** |
|---|---|---|---|---|---|
| Comparative Example 1 | 6H | 5B | No cracking | 60 | < 20 |
| Example A | 6H | 5B | No cracking | 120 | 80 |
| Example B | 4H | 5B | No cracking | 144 | 80 |

The coating compositions prepared with the compound comprising polyacetoacetate functionality and at least one urethane linkage of the present invention had maintained similar dry adhesion and mandrel bed properties and had improved direct and reverse impact properties. The coating compositions prepared with the compound comprising polyacetoacetate functionality and at least one urethane linkage of the present invention maintained similar hardness but had improved flexibility, as shown by the direct and reverse impact properties.

### Example 8

### Preparation of an Unpigmented Composition using the Compounds of Examples 1 through 6

A primer coating composition was prepared by mixing an A side and a B side. For the A side, the base components of Table 1 were weighed and placed into a scintillation vial and mixed until homogenous. For the B side, the base components of Table 1 were weighed and placed into a separate scintillation vial and mixed until homogenous. All material amounts are in terms of weight percent unless otherwise specified.

**TABLE 6**

| **Components** | **Example C (wt. %)** | **Example D (wt. %)** | **Example E (wt. %)** | **Example F (wt. %)** | **Example G (wt. %)** |
|---|---|---|---|---|---|
| **A Side** | | | | | |
| ¹5.5 functional epoxy ketimine | 32.62% | 28.78% | 32.42% | 30.81% | 23.77% |
| ²IPDA ketimine | 13.78% | 12.16% | 13.69% | 13.01% | 10.04% |

| **B Side** | | | | | |
|---|---|---|---|---|---|
| ²³SILQUEST A-187 (GLYMO) | 2.51% | 2.22% | 2.50% | 2.37% | 1.83% |
| ²²Isostearic acid | 0.09% | 0.08% | 0.09% | 0.09% | 0.07% |
| ²⁴SARTOMER 349 | 2.62% | 2.32% | 2.61% | 2.48% | 1.91% |
| ¹⁹Example 1 | 20.07% | - | - | - | - |
| ²⁰Example 2 | - | 27.16% | - | - | - |
| ²⁵Example 3 | - | - | 20.46% | - | - |
| ²⁶Example 4 | - | - | - | 23.44% | - |
| ²⁷Example 5 | - | - | - | - | 36.47% |
| Example 6 | - | - | - | - | - |
| ¹²EPON 828 | 12.93% | 11.41% | 12.85% | 12.21% | 9.42% |
| ¹³n-butylacetate | 15.38% | 15.88% | 15.37% | 15.58% | 16.49% |

| | | | | | |
|---|---|---|---|---|---|
| ²⁴3 mole ethoxylated bisphenol-A dimethacrylate commercially available from Sartomer Company (Exton, Pennsylvania) ²⁵ Compound of Example 3 ²⁶ Compound of Example 4 ²⁷ Compound of Example 5 ²⁸ Compound of Example 6 | | | | | |

Prior to the coating application, the corresponding amounts of the A side formulation and the B side formulation shown in Table 7 were combined and mixed thoroughly. The coating composition was applied onto a 4 inch x 12 inch electrocoated steel substrate (ED7100) obtained from ACT Test Panels LLC (item number 60728, Hillsdale, MI) to a dry film thickness of from 1.6 to 1.9 mils using a drawdown square. The coated panels were flashed for 5 minutes at ambient temperature (20°C to 24°C) and at 10% to 80% relative humidity and then cured by oven baking at 60°C for 30 minutes. The coated panels were then post cured for seven days under ambient conditions prior to testing.

**TABLE 7**

| | **A Side (wt.%)** | **B Side (wt.%)** |
|---|---|---|
| Example C | 46.4 | 53.6 |
| Example D | 40.9 | 59.1 |
| Example E | 46.1 | 53.9 |
| Example F | 43.8 | 56.2 |
| Example G | 33.8 | 66.2 |

The coated ED7100 substrates were tested for pendulum hardness according to ASTM D4366-16, solvent resistance to methyl ethyl ketone (MEK) according to ASTM D5402-92, and mandrel bend according to ASTM D522M-17. The results are shown in Table 8.

**TABLE 8**

| | **Pendulum Hardness (seconds, s)** | **Solvent Resistance (double rubs, DR)** | **Mandrel Bend** |
|---|---|---|---|
| Example C | 112 | 100+ | No cracking |
| Example D | 59 | 100+ | No cracking |
| Example E | 56 | 100+ | No cracking |
| Example F | 115 | 88 | No cracking |
| Example G | 48 | 92 | No cracking |

The coating compositions prepared with the compounds comprising polyacetoacetate functionality and at least one urethane linkage of the present invention displayed utility as a coating with the hardness and solvent resistance properties seen above in Table 8. In addition, the coating compositions prepared with the compound comprising polyacetoacetate functionality and at least one urethane linkage of the present invention displayed adequate flexibility, as shown by the mandrel bend results.

Whereas particular embodiments of this invention have been described above for purposes of illustration, it will be evident to those skilled in the art that numerous variations of the details of the present invention may be made without departing from the invention as defined in the appended claims.

## Claims

1. A coating composition comprising:
(i) a compound comprising polyacetoacetate functionality and at least one urethane linkage, comprising at least one of the following Structures (I) to (III): 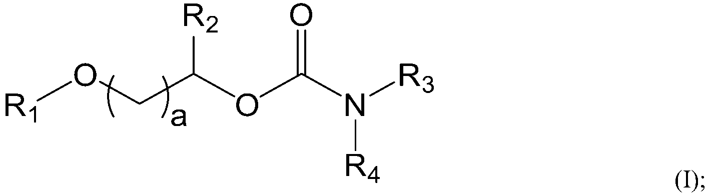 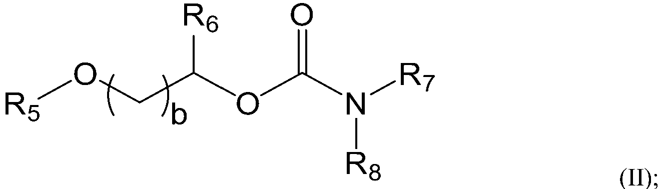 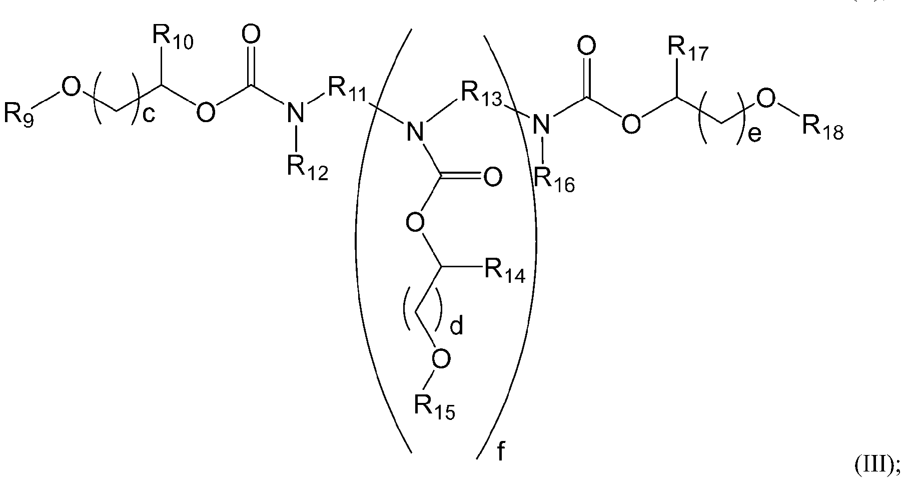
wherein R₁ comprises an acetoacetate containing group; wherein R₂ comprises a hydrogen, methyl, or an acetoacetate containing group; wherein R₃ is bound to the nitrogen of the urethane linkage and comprises at least two acetoacetate containing groups; wherein R₄ is bound to the nitrogen of the urethane linkage and comprises a hydrogen, alkyl, or an acetoacetate containing group; wherein a is 1 or 2;
wherein R₅ comprises an acetoacetate containing group; wherein R₆ comprises a hydrogen, methyl, or an acetoacetate containing group; wherein R₇ is bound to the nitrogen of the urethane linkage and comprises at least one acetoacetate containing group; wherein R₈ is bound to the nitrogen of the urethane linkage and comprises at least one acetoacetate containing group; wherein b is 1 or 2;
wherein R₉, R₁₅, and R₁₈ each independently comprise an acetoacetate containing group; wherein R₁₀, R₁₄, and R₁₇ each independently comprise a hydrogen, methyl, or acetoacetate containing group; wherein R₁₁ is bound to the nitrogen of the urethane linkage and comprises an alkyl, a cycloalkyl, an aryl, 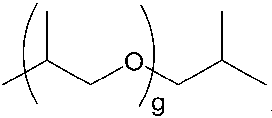 wherein g is 2 to 70, 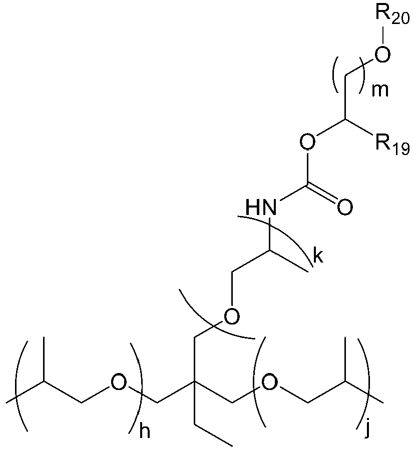 wherein the sum of h, j, and k is 5 to 6, wherein R₁₉ comprises a hydrogen, methyl, or an acetoacetate containing group, wherein R₂₀ comprises an acetoacetate containing group, and wherein m comprises 1 or 2, or 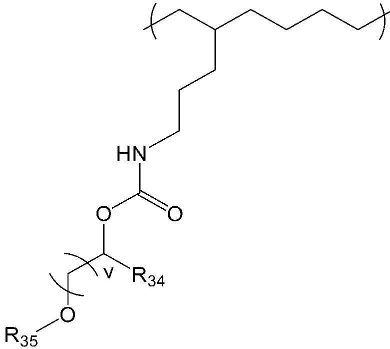
wherein R₃₄ comprises a hydrogen, methyl, or an acetoacetate containing group, wherein R₃₅ comprises an acetoacetate containing group, and wherein v comprises 1 or 2; wherein R₁₂ and R₁₆ each independently comprise a hydrogen, an alkyl, a cycloalkyl, or an aryl;
wherein R₁₃ is bound to the nitrogen of the urethane linkage and comprises an alkyl; wherein c, d, and e are each independently 1 or 2; and
wherein f is 0 to 3;
(ii) a blocked polyamine; and
(iii) a polyepoxide reactive with the condensation product of (i) and (ii).

2. The coating composition of claim 1, wherein Structure (I) is prepared by:
reacting (a) at least one cyclic carbonate compound, (b) at least one polyol having one primary amine and at least two hydroxyl groups , and (c) optionally at least one lactone or at least one lactone cyclic di-ester to form a polyol-functional urethane compound; and
reacting a compound comprising an acetoacetate functionality with a hydroxyl group of the polyol-functional urethane compound to introduce acetoacetate functionality into the polyol-functional urethane compound backbone to form the compound comprising polyacetoacetate functionality and at least one urethane linkage; or
wherein Structure (II) is prepared by:
reacting (a) at least one cyclic carbonate compound, (b) at least one polyol having one or more secondary amines, and (c) optionally at least one lactone or at least one lactone cyclic di-ester to form a polyol-functional urethane compound; and
reacting a compound comprising an acetoacetate functionality with a hydroxyl group of the polyol-functional urethane compound to introduce acetoacetate functionality into the polyol-functional urethane compound to form the compound comprising polyacetoacetate functionality and at least one urethane linkage; or
wherein Structure (III) is prepared by:
reacting (a) at least one cyclic carbonate compound, (b) at least one compound comprising two or more amine groups, and (c) optionally at least one lactone or at least one lactone cyclic di-ester to form a polyol-functional urethane compound; and
reacting a compound comprising an acetoacetate functionality with a hydroxyl group of the polyol-functional urethane compound to introduce acetoacetate functionality into the polyol-functional urethane compound to form the compound comprising polyacetoacetate functionality and at least one urethane linkage.

3. The coating composition of claim 2, wherein the (a) at least one cyclic carbonate compound comprises the following structure: 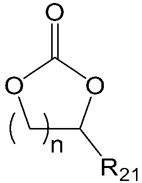
wherein R₂₁ comprises a hydrogen, methyl, or hydroxymethyl, and
wherein n is 1 or 2.

4. The coating composition of claim 2 or 3, wherein the (b) at least one polyol having one primary amine and at least two hydroxyl groups comprises the following structure: 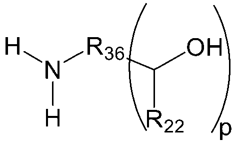
wherein R₂₂ comprises a hydrogen or an alkyl,
wherein R₃₆ comprises a linear or branched alkyl, a substituted or unsubstituted cycloalkyl, or a substituted or unsubstituted aryl, and
wherein p is 2 to 3, or
wherein the (b) at least one polyol having one or more secondary amines comprises the following structure: 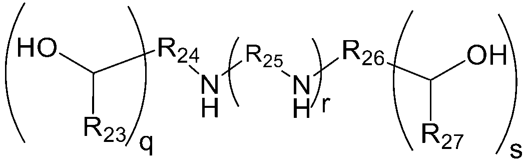
wherein R₂₃ and R₂₇ each independently comprise a hydrogen or an alkyl,
wherein R₂₄, R₂₅, R₂₆ each independently comprise an alkyl,
wherein q and s are each independently 1 to 3, and
wherein r is 0 to 3, or
wherein the (b) at least one compound comprising two or more amine groups comprises the following structure: 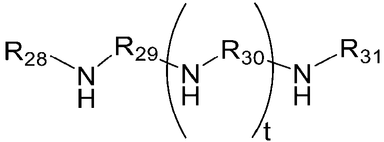
wherein R₂₈ and R₃₁ each independently comprise a hydrogen, an alkyl, a cycloalkyl, or an aryl
wherein R₂₉ comprises an alkyl, a cycloalkyl, an aryl, 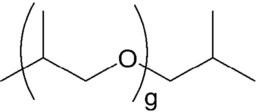 wherein g is 2 to 70, or 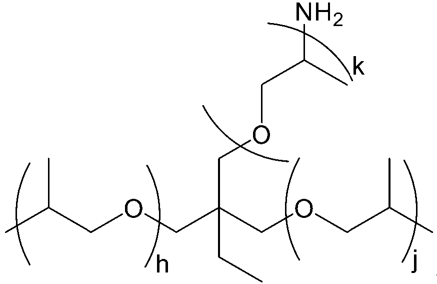 wherein the sum of h, j, and k is 5 to 6, or 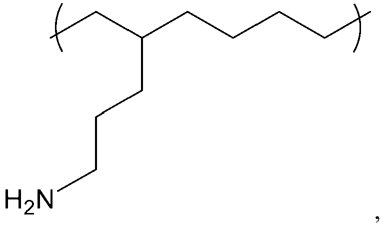
wherein R₃₀ comprises an alkyl, and
wherein t is 0 to 3, where R₂₉ preferably comprises 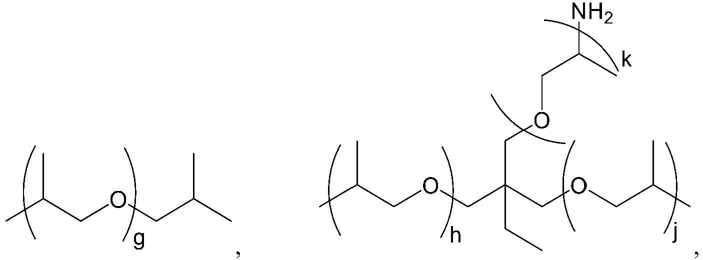 or 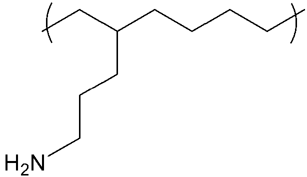 when t is 0.

5. The coating composition of any of claims 2 to 4, wherein the (c) at least one lactone comprises the following structure: 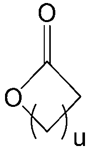
wherein u is 1 to 6; or
wherein the (c) at least one lactone cyclic di-ester comprises D,D-lactide, L,L-lactide, D,L-lactide, glycolide, or some combination thereof; and/or
wherein (a) and (b) are reacted in a first reaction step and further reacted with (c) in second reaction step to form the polyol-functional urethane compound.

6. The coating composition of any claims 2 to 4, wherein (a) and (b) are reacted to form the polyol-functional urethane compound, and
wherein polyol-functional urethane compound is substantially free of (c).

7. The coating composition of any of claims 2-6, wherein the compound comprising the acetoacetate functionality is selected from the group consisting of ethyl acetoacetate, methyl acetoacetate, isobutyl acetoacetate, isopropyl acetoacetate, and *tert-butyl* acetoacetate.

8. The coating composition of any of claims 1-7, wherein the compound comprising polyacetoacetate functionality and at least one urethane linkage has an acetoacetate equivalent weight of from 50 to 700; and/or
wherein the compound comprising polyacetoacetate functionality and at least one urethane linkage has a weight average molecular weight of up to 2,000 grams per mole.

9. The coating composition of any of claims 1-8, wherein the blocked polyamine comprises a polyketimine; and/or
wherein the polyepoxide comprises an aromatic and/or an aliphatic polyepoxide polymer.

10. The coating composition of any of claims 1-9, further comprising:
- a metal compound comprising an alkaline earth metal hydroxide and/or alkaline earth metal oxide, wherein the metal compound preferably comprises a magnesium hydroxide, a magnesium oxide, a calcium hydroxide, a calcium oxide, or some combination thereof; and/or
- an alkoxysilane, wherein the alkoxysilane preferably comprises an amino, ketimine, mercapto, and/or epoxide group; and/or
- a metal phosphate complex; and/or
- a carboxylic acid catalyst; and/or
- a multi-functional (meth)acrylate material.

11. The coating composition of any of claims 1-10, wherein the coating composition comprises a two-component composition comprising (1) a first component comprising the blocked polyamine and (2) a second component comprising a mixture of the compound comprising polyacetoacetate functionality and at least one urethane linkage and the polyepoxide.

12. A substrate at least partially coated with a coating formed from the coating composition of any of claims 1-11.

13. The substrate of claim 12, wherein the substrate comprises a metallic substrate.

14. A multi-layer coated system comprising:
(1) a substrate;
(2) a first coating layer formed over at least a portion of the substrate, the first coating layer obtained from a first coating composition; and
(3) a second coating layer formed over at least a portion of the first coating layer, the second coating layer obtained from a second coating composition that is different from the first coating composition,
wherein the first coating composition comprises the coating composition of any of claims 1-11.
